# FASCICULE DE BREVET EUROPEEN

(11) **EP 1 196 583 B1**
(45) Date de publication et mention de la délivrance du brevet: **12.07.2006**
(21) Numéro de dépôt: 00922717.4
(22) Date de dépôt: 21.04.2000
(51) Int. Cl.: C12N 15/31, C12N 15/54, C12N 15/62, C07K 14/195, C07K 19/00, C12N 9/12, C12P 19/34, C12Q 1/68, C12N 1/21

(54) **ADN POLYMERASE DE TYPE II DE PYROCOCCUS ABYSSI**
TYP II DNS POLYMERASE AUS PYROCOCCUS ABYSSI
TYPE II DNA POLYMERASE FROM PYROCOCCUS ABYSSI

(30) Priorité: 21.04.1999 FR 9905034
(43) Date de publication de la demande: 17.04.2002
(73) Titulaire: CENTRE NATIONAL DE LA RECHERCHE SCIENTIFIQUE (CNRS), 75794 Paris Cedex 16 (FR); IFREMER INSTITUT FRANCAIS DE RECHERCHE POUR L'EXPLOITATION DE LA MER, 92130 Issy-les-Moulineaux (FR)
(72) Inventeur: FORTERRE, Patrick, F-92340 Bourg-la-Reine (FR); THIERRY, Jean-Claude, F-67200 Strasbourg (FR); PRIEUR, Daniel, F-29290 Saint-Renan (FR); DIETRICH, Jacques, F-29470 Plougastel-Daoulas (FR); LECOMPTE, Odile, F-67000 Strasbourg (FR); QUERELLOU, Joël, F-29200 Brest (FR); WEISSENBACH, Jean, F-75015 Paris (FR); SAURIN, William, F-75015 Paris (FR); HEILIG, Roland, F-67490 Dettwiller (FR); FLAMENT, Didier, F-29200 Brest (FR); RAFFIN, Jean-Paul, F-29200 Brest (FR); HENNEKE, Ghislaine, F-76650 Petit-Couronne (FR); GUEGUEN, Yannick, F-29860 Kersaint-Plabennec (FR); ROLLAND, Jean-Luc, F-29810 Plouarzel (FR)
(74) Mandataire: Warcoin, Jacques
(86) Numéro de dépôt international: PCT/FR2000/001065
(87) Numéro de publication internationale: WO 2000/065062

(56) Documents cités:
- EP-A- 0 870 832
- EP-A- 0 997 530
- WO-A-00/08164
- WO-A-98/01567
- PURCAREA C ET AL: "Aspartate transcarbamylase from the deep-sea hyperthermophilic archaeon Pyrococcus abyssi: genetic organization, structure, and expression in Escherichia coli" J. BACTERIOL., vol. 179, no. 13, juillet 1997 (1997-07), pages 4143-4157, XP000867317
- KAWARABAYASI Y ET AL: "Complete sequence and gene organization of the genome of a hyper-thermophilic archaebacterium, Pyrococcus horikoshii OT3" DNA RES, vol. 5, no. 2, 30 avril 1998 (1998-04-30), pages 55-76, XP001002795 -& DATABASE SWALL E.B.I. Hinxton U.K.; Accession Number: O57863, 1 octobre 2000 (2000-10-01) KAWARABAYASI Y ET AL: "DNA POLYMERASE II SMALL SUBUNIT" XP002169459 -& DATABASE SWALL E.B.I. Hinxton U.K.; Accession Number: O57861, 1 octobre 2000 (2000-10-01) KAWARABAYASI Y ET AL: "DNA POLYMERASE II LARGE SUBUNIT" XP002169460
- TOMB J -F ET AL: "THE COMPLETE GENOME SEQUENCE OF THE GASTRIC PATHOGEN HELICOBACTER PYLORI" NATURE,GB,MACMILLAN JOURNALS LTD. LONDON, vol. 388, no. 6642, 7 août 1997 (1997-08-07), pages 539-547,TABEL, XP002062106 ISSN: 0028-0836
- PRIEUR D ET AL: "Complete sequence of Pyrococcus abyssi" PYROCOCCUS ABYSSI HOME PAGE AT GENOSCOPE, [en ligne] 16 octobre 2000 (2000-10-16), XP002155809 Extrait de l'Internet: <URL:http://www.genoscope.cns.fr/> [extrait le 2000-12-18] & DATABASE EMBL EBI, Hinxton, U.K.; Accession Numbers: AJ248283-AJ248288, 9 septembre 1999 (1999-09-09) "Pyrococcus abyssi complete genome"
- HIGUCHI S ET AL: "Comparison of pathways for amino acid biosynthesis in archaebacteria using their genomic DNA sequences" PROCEEDINGS OF THE JAPAN ACADEMY SERIES B-PHYSICAL AND BIOLOGICAL SCIENCES, vol. 75, no. 8, octobre 1999 (1999-10), pages 241-245, XP000874309
- ERAUSO G ET AL: "Caractérisation préliminaire d'une archaebactérie hyperthermophile possèdant un plasmide, isolée d'une source hydrothermale du Bassin Nord Fidjien" COMPTE RENDU DE L'ACADEMIE DES SCIENCES DE PARIS, SERIE III, vol. 314, no. 9, 1992, pages 387-393, XP002128892 cité dans la demande
- ERAUSO G ET AL: "PYROCOCCUS ABYSSI SP. NOC., A NEW HYPERTHERMOPHILIC ARCHAEON ISOLATED FROM A DEEP-SEA HYDROTHERMAL VENT" ARCHIVES OF MICROBIOLOGY,DE,BERLIN, vol. 160, no. 5, 1 janvier 1993 (1993-01-01), pages 338-349, XP000645347 ISSN: 0302-8933

## Description

L'invention a pour objet des séquences nucléotidiques codant pour des polypeptides de l'ADN polymèrase de Type II de Pyrococcus *abyssi,* ainsi que des vecteurs incluant lesdites séquences et cellules transformées par ces vecteurs. L'invention concerne également des procédés mettant en oeuvre ces acides nucléiques ou polypeptides, en particulier des procédés de biosynthèse ou de biodégradation de molécules d'intérêt, ainsi que des kits comprenant ces polypeptides.

C'est au sein des archaebactéries, découvertes en 1977 (Woese et al., Proc. Natl. Acad. Sci. USA, 87 : 1990), que l'on trouve les organismes les plus hyperthermophiles connus à ce jour, capables de résister à des températures maximales de 90°C à 113°C. Parmi ceux-ci, l'ordre des thennococcales, appartenant à l'embranchement des euryarchaeotes, et singulièrement le genre Pyrococcus, présentent un intérêt particulier pour préciser les relations phylogénétiques au sein des archae et aborder des questions relatives à la nature du dernier ancêtre universel commun aux eucaryotes, bactéries et archaebactéries (LUCA: Last Universal Common Ancestor) et à l'origine de la vie.

Les archaebactéries du genre Pyrococcus sont actuellement les modèles privilégiés pour l'étude des organismes hyperthermophiles (températures optimales de croissance comprises entre 80 et 110°C) (Adams Annu. Rev. Microbiol., 47 : 627, 1997). Bien qu'anaérobies, ces organismes sont faciles à cultiver et peuvent être obtenus sous forme de colonies sur boite (en milieu Gelrite). Les protéines des archaebactéries du genre Pyrococcus sont intrinsèquement thermostables, certaines d'entre elles pouvant résister à l'incubation pendant plusieurs dizaines de minutes à des températures supérieures à 110°. Ces protéines peuvent être utilisées dans de nombreux procédés industriels comme par exemple les ADNs polymérases, ADNs ligases, enzymes de modifications de l'ADN, intéines, protéases ou les enzymes du métabolisme des sucres. La stabilité des protéines hyperthermophiles facilitent également souvent leur cristallisation et la qualité des cristaux obtenus, ce qui favorise leur étude structurale.

Parmi les espèces du genre Pyrococcus dont la séquence génomique a déjà fait l'objet d'érude particulière, on peut citer Pyrococcus *furiosus,* espèce la plus étudiée, qui a été isolée à partir d'une source hydrothermale terrestre et dont le génome, partiellement disponible, a été séquencé par l'équipe de Robert Weiss à l'Université de l'Utah. Le génome de Pyrococcus *horikoshii,* isolé à partir d'une source hydrothermale marine profonde (Pacifique Est) a été séquencé par une équipe Japonaise et publié en 1998 (Kawarabayasi Y. et al., DNA Res. 1998,5,55-76). D'après les auteurs, le génome de P. *horikoshii* ne contient que 20 % de gènes identifiables, ce qui est faible par rapport aux génomes d'autres archaebactéries telles que Methanococcus *jannashii,* Archaeoglobus *fulgidus* et Methanobacterium *thermoautotrophicum,* pour lesquels la proportion de gènes identifiables par similarité de séquence tourne autour de 40 %. Les bactéries du genre Pyrococcus sont donc un réservoir de gènes potentiellement d'intérêt industriel dont de nombreux gènes encore inconnus.

Le criblage d'une vingtaine de souches indépendantes de Thermococcales isolées par D. Prieur et coll. à partir d'échantillons de cheminées et de fluides hydrothermaux provenant de deux sites océaniques profonds du bassion Nord-Fitjien a permis d'identifier une souche de Pyrococcus, dénommée souche ORSAY, contenant un plasmide pGT5 de petite taille et multicopies (Erauso et al., 1992, Compte rendu de l'Académie des Sciences de Paris, 314, 381-393). L'espèce correspondant à cette souche, Pyrococcus *abyssi* a été décrite en détail sur les plans physiologique et taxinomique (Erauso et al, Arch Microbiol. 160: 338-349, 1993). Un milieu défini a été établi pour cette souche, ainsi que les conditions de mutagénèse et d'isolement de mutants (Watrin et al., Appl. Environ. Microbiol, 61: 1138 ; 1995 ; Watrin et al., Current Microbiol. 33,377, 1996). Ce plasmide, dénommée pGT5, a été séquencé et identfié comme étant un plasmide du type cercle-roulant (Erauso et al., 1996, J. Bacteriol. 178:3232) et sert actuellement de point de départ pour là mise au point d'un vecteur de navette pour les Pyrococcus (Aagard et al., FEMS Microbiol. Rev. 18, 93, 1996 ; Aravalli et al., Extemophiles 1997, 1:183-91). En raison de la présence du plasmide pGT5, P. *abyssi* a également été choisi comme souche privilégiée pour l'étude de la réplication de l'ADN chez les archaebactéries hyperthermophiles (Marsin et Forterre, Mol. Microbiol., 27, 1183-1192, 1998).

Compte tenu d'une part du nombre important de gènes potentiels d'intérêt industriel encore inconnus ainsi que du développement des études menées sur P. *abyssi,* il existe aujourd'hui un besoin de disposer d'une information plus ample concernant la biologie moléculaire de ces souches, notamment à partir d'une meilleure connaissance de son génome.

D'autre part, le fait de disposer de la séquence génomique complète de trois Pyrococcus d'espèces différentes permettra en particulier l'identification de gènes pouvant être utilisés comme marqueurs génétiques et d'utiliser la puissance de la génomique comparative pour améliorer l'identification des gènes et leur annotation fonctionnelle (ce qui est particulièrement important dans le cas des Pyrococcus compte tenu du grand nombre de gènes orphelins), et apporter des informations importantes quant à la plasticité de leur génome. En effet, du point de vue évolutif, le fait de disposer de trois séquences génomiques est particulièrement intéressant car ceci permettra également de polariser les événements génomiques survenus entre deux d'entre eux (les plus proches, P. *abyssi* et P. *horikoshii*) en utilisant le troisième (P. *furiosus*) comme groupe extérieur. Par ailleurs, l'étude comparative, au niveau le plus fin, du génome de Pyrococcus *abyssi* par rapport au nombre croissant de génomes procaryotes désormais disponibles devrait permettre d'appréhender les mécanismes moléculaires associés à la colonisation des milieux extrêmes de température et de pression.

L'ensemble de ces données pourra permettre notamment la mise en évidence d'événements génomiques liés à la micro-évolution, en particulier les recombinaisons génomiques, les séquences régulatrices conservées, les régions hypervariables, la mobilité des intéines, etc..

La présente description décrit la séquence nucléotidique de séquence SEQ ID N° 1 du génome de Pyrococcus *abyssi* souche ORSAY.

La souche Pyrococcus *abyssi* ORSAY a fait l'objet d'un dépôt le 9 Avril 1999, à la CNCM (Paris, France) sous le numéro I-2180.

Par séquence du génome, ou séquence génomique de Pyrococcus *abyssi,* on entend désigner la séquence du chromosome de Pyrococcus *abyssi.*

On entend par séquence nucléotidique, polynucléotide ou acide nucléique, selon la présente invention, aussi bien un ADN double brin, un ADN simple brin que des produits de transcription desdits ADNs.

Il doit être compris que la présente invention ne concerne pas les séquences nucléotidiques génomiques de Pyrococcus *abyssi* prises dans leur environnement naturel, c'est-à-dire à l'état naturel. Il s'agit de séquences qui ont pu être isolées, purifiées ou partiellement purifiées, à partir de méthodes de séparation telles que par exemple la chromatographie par échange d'ions, par exclusion basée sur la taille moléculaire, ou par affinité, ou encore les techniques de fractionnement basées sur la solubilité dans différents solvants, ou à partir de méthodes du génie génétique telles que l'amplification, le clonage et le sous-clonage, les séquences de l'invention pouvant être portées par des vecteurs.

La séquence nucléotidique SEQ ID N° 1 a été obtenue par séquençage du génome de Pyrococcus *abyssi* souche ORSAY par une méthode comprenant une première étape de séquençage d'extrémités appariées (phase aléatoire), suivie d'un séquençage dirigé assurant la complétion de la séquence et de l'assemblage de ces séquences de fragments nucléotidiques au moyen de logiciels (cf. Exemples). Malgré la grande précision de la séquence SEQ ID N° 1, il est possible que celle-ci ne représente pas de manière parfaite à 100 % la séquence nucléotidique du génome de Pyrococcus *abyssi* souche ORSAY et que quelques rares erreurs de séquençage ou indéterminations subsistent dans la séquence SEQ ID N° 1. Ces quelques rares erreurs ou indéterminations pourraient facilement être mises en évidence et corrigées par l'homme de l'art à partir du chromosome entier et/ou de ses fragments représentatifs selon l'invention et des méthodes standards d'amplification, de clonage et de séquençage, les séquences obtenues pouvant facilement être comparées, en particulier au moyen d'un logiciel informatique et en utilisant des supports d'enregistrement des séquences selon l'invention lisibles par un ordinateur tels que décrits par exemple ci-après. Après correction de ces éventuelles rares erreurs ou indéterminations, la séquence nucléotidique corrigée obtenue comporterait encore au moins 99,9 % d'identité avec la séquence SEQ ID N° 1.

Par séquence nucléotidique homologue à une séquence donnée au sens de la présente invention, on entend une séquence nucléotidique présentant un pourcentage d'identité après alignement optimal avec les bases de la séquence donnée d'au moins 80 %, de préférence 85 %, 90 %, 95 % et 98 %, ce pourcentage étant purement statistique et les différences entre les deux séquences nucléotidiques pouvant être réparties au hasard et sur toute leur longueur.

Par « pourcentage d'identité » entre deux séquences d'acide nucléique ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons de séquences entre deux séquences d'acide nucléique ou d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide nucléique ou d'acides aminés est déterminé en comparant ces deux séquences alignées de manière optimale par fenêtre de comparaison dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 séquences » (Tatusova et al., "Blast 2 sequences - a new tool for comparing protein and nucleotide sequences", FEMS Microbiol. Lett. 174 : 247-250) disponible sur le site http://www.ncbi.nlm.nih.gov/gorf/b12.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

Lesdites séquences homologues présentant un pourcentage d'identité avec les bases de la séquence nucléotidique SEQ ID N° 1 d'au moins 80 %, de préférence 85 %, 90 %, 95 % et 98 %, peuvent comprendre par exemple les séquences correspondant à la séquence génomique ou aux séquences de ses fragments représentatifs de bactérie appartenant aux variants de l'espèce Pyrococcus *abyssi* souche ORSAY. Dans la présente description, les termes famille et genre sont interchangeables entre eux, les termes variant, souche et sous-espèce sont également interchangeables entre eux. Ces séquences homologues peuvent ainsi correspondre à des variations liées à des mutations au sein d'une même espèce ou entre espèces et correspondre notamment à des troncatures, substitutions, délétions et/ou additions d'au moins un nucléotide, Lesdites séquences homologues peuvent également correspondre à des variations liées à la dégénérescence du code génétique ou à un biais dans le code génétique spécifique du genre, de l'espèce ou de variant et qui sont susceptibles d'être présentes chez Pyrococcus *abyssi.*

Par séquence nucléotidique complémentaire d'une séquence de l'invention, on entend tout ADN dont les nucléotides sont complémentaires de ceux de la séquence de l'invention, et dont l'orientation est inversée (séquence antiparallèle ou antisens).

Par fragments représentatifs des séquences selon l'invention, on entendra désigner tout fragment nucléotidique présentant au moins 15 nucléotides, de préférence au moins 30, 75, 150, 300, et 450 nucléotides consécutifs de la séquence dont il est issu.

Parmi ces fragments représentatifs, on peut citer ceux capables de s'hybrider dans des conditions de stringence avec une séquence nucléotidique selon l'invention. Une hybridation dans des conditions de stringence signifie que les conditions de température et de force ionique sont choisies de telle manière qu'elles permettent le maintien de l'hybridation entre deux fragments d'ADN complémentaires.

A titre illustratif, des conditions de forte stringence de l'étape d'hybridation aux fins de définir les fragments nucléocidiques décrits ci-dessus, sont avantageusement les suivantes.

L'hybridation est réalisée à une température préférentielle de 65°C en présence de tampon SSC, 1 x SSC correspondant à 0,15 M NaCl et 0,05 M citrate de Na. Les étapes de lavage peuvent, par exemple, être les suivantes :
- 2 x SSC, 0,1 % SDS à température de la pièce suivi de trois lavages à 1 x SSC, 0,1 % SDS ; 0,5 x SSC, 0,1 % SDS ; 0,1 x SSC, 0,1 % SDS à 68°C pendant 15 minutes.

Les conditions de stringence intermédiaire, en utilisant par exemple une température de 60°C en présence d'un tampon 5 x SSC, ou de faible stringence, par exemple une température de 50°C en présence d'un tampon 5 x SSC, requièrent respectivement pour l'hybridation entre les deux séquences une complémentarité globale moins importante.

Les conditions stringentes d'hybridation décrites ci-avant pour un polynucléotide d'une taille d'environ 300 bases, seront adaptées par l'homme du métier pour des oligonucléotides de taille plus grande ou plus petite, selon l'enseignement de Sambrook et al., 1989.

Parmi les fragments représentatifs on peut citer également ceux utilisables comme amorce ou sonde dans des méthodes permettant d'obtenir les séquences homologues ou leurs fragments selon l'invention, ou de reconstituer un fragment génomique se révélant incomplet dans la séquence SEQ ID N° 1 ou porteur d'erreur ou d'indétermination, ces méthodes telles la réaction en chaîne à la polymérase (PCR), le clonage et le séquençage d'acide nucléique étant bien connues de l'homme de l'art. Ces séquences nucléotidiques homologues correspondant à des mutations ou à des variations inter- ou intra-espèces, ainsi que la séquence génomique complète ou un de ses fragments susceptibles d'être reconstitués, font bien entendu partie de invention.

Parmi lesdits fragments représentatifs, on peut citer enfin ceux correspondant à des séquences nucléotidiques correspondant à des cadres ouverts de lecture, dénommés séquences ORF (ORF pour « open reading frame »), compris en général entre un codon d'initiation et un codon stop, ou entre deux codons stop, et codant pour des polypeptides, de préférence d'au moins 100 acides aminés, telles que par exemple, mais sans s'y limiter, les séquences ORF qui seront par la suite décrites.

La numérotation des séquences nueléotidiques ORF qui sera par la suite utilisée dans la présente description correspond à la numérotation des séquences d'acides aminés des protéines codées par lesdits ORF.

Ainsi, les séquences nucléotidiques ORF 2, ORF 3,..., ORF 811 et ORF 812 codent respectivement pour les protéines de séquences d'acides aminés SEQ ID N° 2, SEQ ID N° 3, ..., SEQ ID N° 811 et SEQ ID N° 812 figurant dans la liste des séquences ci-après.

Les séquences nucléotidiques détaillées des séquences ORF 2, ORF 3, ..., ORF 790 et ORF 791 sont déterminées par leur position respective sur la séquence génomique SEQ ID N° 1 figurant de la liste des séquences ainsi que par la caractéristique « complement » indiquant, si elle est mentionnée, que ces séquences sont situées sur le brin complémentaire de la séquence SEQ ID N° 1. Dans la présente description, lesdites positions de ces séquences ORF ainsi que la caractéristique "complement ", si cette dernière doit être indiquée, sont mentionnées dans les caractéristiques figurant dans la liste des séquences pour la séquence SEQ ID N° 1 à l'identificateur numérique ou rubrique <222> associé à la rubrique <223> suivante mentionnant « SEQ ID : X » avec X correspondant au numéro de l'ORF ainsi déterminée.

Par exemple :
la séquence nucléotidique détaillée de l'ORF 374, identifiée par ses positions nt 1765176 - nt 1764523 et la cactéristique < complement > mentionnées à la rubrique < 222 > associée à la rubrique « <223> SEQ ID : 374 », est la séquence complémentaire de la séquence comprise entre les positions nt 1765176 - nt 1764523, extrémités comprises, de la séquence SEQ ID N° 1 ;
la séquence nucléotidique détaillée de l'ORF 2, identifiée par ses positions nt 25162 - nt 26181 mentionnées à la rubrique <222> associée à la rubrique « <223> SEQ ID : 2 », est la séquence comprise entre les positions nt 25162 - nt 26181, extrémités comprises, de la séquence SEQ ID N°1.

Du fait de la circularité du génome de Pyrococcus *abyssi,* certains éléments de séquences peuvent chevaucher l'origine de la numérotation. Dans cette hypothèse, la coordonnée d'une de ces extrémités peut avoir une valeur supérieure à la longueur du génome. Il faut alors interpréter cette position en y soustrayant la taille du génome. En l'occurence ceci ne concerne que l'ORF 374 dont les coordonnées 1765176 et 1764523 mentionnées à la rubrique <222> associée à la rubrique « <223> SEQ ID : 374 > » doivent être interprétées comme 58 et 1764523 respectivement.

Les fragments représentatifs peuvent être obtenus par exemple par amplification spécifique, telle que la PCR, ou après digestion par des enzymes de restriction appropriées de séquences nucléotidiques selon l'invention, ces méthodes sont en particulier décrites dans l'ouvrage de Sambrook et al., 1989. Cesdits fragments représentatifs peuvent également être obtenus par synthèse chimique lorsque leur taille n'est pas trop importante et selon des méthodes bien connues de l'homme de l'art.

Les fragments représentatifs pourront être utilisés, par exemple en tant qu'amorce, pour reconstituer certains desdits fragments représentatifs, notamment ceux dont une partie de la séquence serait susceptible d'être manquante ou imparfaite, par des méthodes bien connues de l'homme de l'art telles que les techniques d'amplification, de clonage ou de séquençage.

Par séquence nucléotidique modifiée, on entendra toute séquence nucléotidique obtenue par mutagenèse selon des techniques bien connues de l'homme de l'art, et comportant des modifications par rapport aux séquences normales, par exemple des mutations dans les séquences régulatrices et/ou promotrices de l'expression de polypeptide, notamment conduisant à une modification du taux d'expression ou de l'activité dudit polypeptide.

Par séquence nucléotidique modifiée, on entendra également toute séquence nucléotidique codant pour un polypeptide modifié tel que défini ci-après.

La présente description décrit les séquences nucléotidiques de Pyrococcus *abyssi* ORF 2 à ORF 812. La numérotation des séquences ORF correspond à l'ordre d'apparition de ces séquences sur le chromosome, à l'exception des ORF 792 à ORF 812.

Les séquences nucléotidiques détaillées des séquences ORF 2 à ORF 791 sont définies comme indiqué ci-avant.

Les séquences nucléotidiques nucléotidiques détaillées des séquences ORF 792 à ORF 800 sont définies par la position du premier nucléotide et du dernier nucléotide sur la séquence génomique SEQ ID N° 1 mentionée à l'identificateur numérique <223> de la séquence peptidique SEQ ID correspondante.

Les séquences nucléotidiques détaillées des séquences ORF 792 à ORF 800 sont définies par la position du premier nucléotide et du dernier nucléotide sur la séquence génomique SEQ ID N° 1 mentionée à l'identificateur numérique <223> de la séquence peptidique SEQ ID correspondante.

Les séquences nucléotidiques détaillées des séquences ORF 801, ORF 805, ORF 806, ORF 807, ORF 808, ORF 811 et ORF 812 sont définies dans les exemples 3 à 7 ci- après par la position du premier nucléotide et du dernier nucléotide sur la séquence génomique SEQ ID N° 1.

Les séquences nucléotidiques détaillées des séquences ORF 802, ORF 803, ORF 804, ORF 809 et ORF 810 sont identifiables dans les exemples 3 et 7 par déduction, à partir des ORF 801 et ORF 808 dont elles sont issues.

L'invention concerne les séquences nucléotidiques isolées ou purifiées caractérisées en ce qu'elles comprennent une séquence nucléotidique choisie parmi :
a) une séquence nucléotidique codant pour une protéine de séquence d'acides aminés SEQ ID N° 807 et SEQ ID N° 809; et
c) une séquence nucléotidique complémentaire a une séquence telle que définie en a).

L'invention comprend également les séquences nucléotidiques selon l'invention, caractérisées en ce qu'il s'agit d'une séquence ADN ou ARN.

L'invention comprend les polypeptides codés par une séquence nucléotidique selon l'invention.

L'invention comprend également les polypeptides caractérisés en ce qu'ils comprennent un polypeptide choisi parmi :
a) un polypeptide selon l'invention de séquence SEQ ID N° 807 et un polypeptide selon l'invention de séquence SEQ ID N° 809;
b) un fragment d'au moins 100 acides aminés d'un polypeptide de séquence SEQ ID N° 807 et accroissant l'activité de formation de liaisons phosphodiesters de la grande sous-unité de l'ADN polymérase thermostable de type II, ou;
c) un fragment biologiquement actif d'un polypeptide de séquence SEQ ID N° 809 à activité ADN polymérase d'au moins 100 acides aminés et comprenant les acides aminés 954 et suivants de la sequence SEQ ID N°809.

Dans la présente description, les termes polypeptide, peptide et protéine sont interchangeables.

Il doit être compris que l'invention ne concerne pas les polypeptides sous forme naturelle, c'est-à-dire qu'ils ne sont pas pris dans leur environnement naturel mais qu'ils ont pu être isolés ou obtenus par purification à partir de sources naturelles, ou bien obtenus par recombinaison génétique, ou encore par synthèse chimique et qu'ils peuvent alors comporter des acides aminés non naturels, comme cela sera décrit ci-après.

Par polypeptide homologue, on entendra désigner les polypeptides présentant, par rapport au polypeptide naturel, certaines modifications comme en particulier une délétion, addition ou substitution d'au moins un acide aminé, une troncation, un allongement, une fusion chimérique, et/ou une mutation, ou des polypeptides présentant des modifications post-traductionnelles. Parmi les polypeptides homologues, on préfère ceux dont la séquence d'acides aminés présente au moins 80 %, de préférence 90 %, 95 % et 98 %, d'homologie avec les séquences d'acides aminés des polypeptides selon l'invention. Dans le cas d'une substitution, un ou plusieurs acides aminés consécutifs ou non consécutifs, sont remplacés par des acides aminés « équivalents ». L'expression acide aminé « équivalent » vise ici à désigner tout acide aminé susceptible d'être substitué à l'un des acides aminés de la structure de base sans cependant modifier essentiellement les activités biologiques des peptides correspondants et telles qu'elles seront définies par la suite.

Ces acides aminés équivalents peuvent être déterminés soit en s'appuyant sur leur homologie de structure avec les acides aminés auxquels ils se substituent, soit sur des résultats d'essais comparatifs d'activité biologique entre les différents polypeptides susceptibles d'être effectués.

A titre d'exemple, on mentionnera les possibilités de substitutions susceptibles d'être effectuées sans qu'il en résulte une modification approfondie de l'activité biologique des polypeptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Les polypeptides homologues correspondent également aux polypeptides codés par les séquences nucléotidiques homologues telles que définies précédemment et comprennent ainsi dans la présente définition les polypeptides mutés ou correspondant à des variations inter- ou intra-espèces, pouvant exister chez Pyrococcus, et qui correspondent notamment à des troncatures, substitutions, délétions et/ou additions d'au moins un résidu d'acide aminé.

Par fragment biologiquement actif d'un polypeptide selon l'invention, on entendra désigner en particulier un fragment de polypeptide, tel que défini ci-après, présentant au moins une des caractéristiques des polypeptides selon l'invention, notamment en ce qu'il est capable d'exercer de manière générale une activité, même partielle, telle que:
- une activité enzymatique impliquée dans le processus d'amplification de l'ADN.

Par fragment de polypeptide selon l'invention, on entend désigner un polypeptide comportant au minimum 100 acides aminés.

Les fragments de polypeptide selon l'invention peuvent correspondre à des fragments isolés ou purifiés naturellement présents dans Pyrococcus *abyssi* ou sécrétés par Pyrococcus *abyssi,* ou correspondre à des fragments pouvant être obtenus par clivage dudit polypeptide par une enzyme protéolytique, telle que la trypsine ou la chymotrypsine ou la collagénase, ou par un réactif chimique, tel que le bromure de cyanogène (CNBr) ou encore en plaçant ledit polypeptide dans un environnement très acide, par exemple à pH 2,5. De tels fragments polypeptidiques peuvent être également préparés indifféremment par synthèse chimique, à partir d'hôtes transformés par un vecteur d'expression selon l'invention contenant un acide nucléique permettant l'expression desdits fragments, placé sous le contrôle des éléments de régulation et/ou d'expression appropriés.

Par « polypeptide modifié» d'un polypeptide selon l'invention, on entend désigner un polypeptide obtenu par recombinaison génétique ou par synthèse chimique comme cela sera décrit ci-après, présentant au moins une modification par rapport à la séquence normale. Ces modifications pourront notamment porter sur des acides aminés à l'origine d'une spécificité ou de l'efficacité de l'activité, ou à l'origine de la conformation structurale, de la charge, ou de l'hydrophobicité du polypeptide selon l'invention. On pourra ainsi créer des polypeptides d'activité équivalente, augmentée ou diminuée, et de spécificité équivalente, plus étroite, ou plus large. Parmi les polypeptides modifiés, il faut citer les polypeptides dans lesquels jusqu'à 5 acides aminés peuvent être modifiés, tronqués à l'extrémité N- ou C-terminale, ou bien délétés, ou bien ajoutés.

Comme cela est indiqué, les modifications du polypeptide auront pour objectif notamment :
- de permettre sa mise en oeuvre dans des procédés de biosynthèse ou de biodégradation de composés organiques ou inorganiques,
- de permettre sa mise en oeuvre dans des procédés de réplication, d'amplification, de réparation, de transcription, de traduction ou de maturation, notamment de l'ADN et de l'ARN,
- de modifier sa solubilité, l'efficacité ou la spécificité de son activité, ou encore de facilité sa purification.

La synthèse chimique présente également l'avantage de pouvoir utiliser des acides aminés non naturels, ou des liaisons non peptidiques. Ainsi, afin d'améliorer la durée de vie des polypeptides selon l'invention, il pourra être intéressant d'utiliser des acides aminés non naturels, par exemple sous forme D, ou bien des analogues d'acides aminés, notamment des formes soufrées par exemple.

Font également partie de l'invention les séquences nucléotidiques codant pour un polypeptide selon l'invention.

L'invention concerne également des séquences nucléotidiques utilisables comme amorce ou sonde, caractérisées en ce que lesdites séquences sont choisies parmi les séquences nucléotidiques selon l'invention.

L'invention concerne en outre l'utilisation d'une séquence nucléotidique selon l'invention, comme amorce ou sonde, pour l'amplification de séquences d'acide nucléique.

Les séquences nucléotidiques selon l'invention peuvent ainsi être utilisées pour amplifier des séquences nucléotidiques, notamment par la technique PCR (réaction en chaîne à la polymérase) (Erlich, 1989 ; Innis et al., 1990 ; Rolfs et al., 1991, et White et al., 1997).

Ces amorces oligodésoxyribonucléotidiques ou oligo-ribonucléotidiques ont avantageusement une longueur d'au moins 10 nucléotides, de préférence d'au moins 15 nucléotides, et encore plus préférentiellement au moins 20 nucléotides.

D'autres techniques d'amplification de l'acide nucléique cible peuvent être avantageusement employées comme alternatives à la PCR.

Les séquences nucléotidiques de l'invention, en particulier les amorces selon l'invention, peuvent également être mises en oeuvre dans d'autres procédés d'amplification d'un acide nucléique cible, tels que :
- la technique TAS (Transcription-based Amplification System), décrite par Kwoh et al. en 1989 ;
- la technique 3SR (Self-Sustained Sequence Replication), décrite par Guatelli et al. en 1990 ;
- la technique NASBA (Nucleic Acid Sequence Based Amplification), décrite par Kievitis et al. en 1991 ;
- la technique SDA (Strand Displacement Amplification) ou technique d'amplification à déplacement de brin (Walker et al., 1992) ;
- la technique TMA (Transcription Mediated Amplification).

Les polynucléotides de l'invention peuvent aussi être employés dans des techniques d'amplification ou de modification de l'acide nucléique servant de sonde, telles que :
- la technique LCR (Ligase Chain Reaction), décrite par Landegren et al. en 1988 et perfectionnée par Barany et al. en 1991, qui emploie une ligase thermostable ;
- la technique de RCR (Repair Chain Reaction), décrite par Segev en 1992 ;
- la technique CPR (Cycling Probe Reaction), décrite par Duck et al. en 1990 ;
- la technique d'amplification à la Q-beta-réplicase, décrite par Miele et al. en 1983 et perfectionnée notamment par Chu et al. en 1986, Lizardi et al. en 1988, puis par Burg et al., ainsi que par Stone et al. en 1996.

Dans le cas où éventuellement le polynucléotide cible est un ARN, par exemple un ARNm, on pourra utiliser, préalablement à la mise en oeuvre d'une réaction d'amplification à l'aide d'au moins une amorce selon l'invention ou à la mise en oeuvre d'un procédé de détection à l'aide d'au moins une sonde de l'invention, une enzyme de type transcriptase inverse afin d'obtenir un ADNc à partir de l'ARN contenu dans l'échantillon. L'ADNc obtenu servira alors de cible pour la/les amorce(s) ou la/les sonde(s) mise(s) en oeuvre dans le procédé d'amplification selon l'invention.

L'invention comprend également les séquences nucléotidiques selon l'invention, caractérisées en ce qu'eUes sont immobilisées sur un support, de manière covalente ou non covalente.

Un autre objet de la présente invention est un vecteur pour le clonage et/ou l'expression d'une séquence, caractérisé en ce qu'il contient une séquence nucléotidique selon l'invention.

Parmi lesdits vecteurs selon l'invention, on préfère les vecteurs contenant une séquence nucléotidique codant pour un polypeptide de Pyrococcus *abyssi* ou un de ses fragments.

Lesdits vecteurs selon l'invention contiennent une séquence nucléotidique codant pour un polypeptide de Pyrococcus *abyssi,* ou un de ses fragments.

Les vecteurs selon l'invention, caractérisés en ce qu'ils comportent les éléments permettant l'expression et/ou la sécrétion desdites séquences nucléotidiques dans une cellule hôte déterminée, font également partie de l'invention.

Le vecteur doit alors comporter un promoteur, des signaux d'initiation et de terminaison de la traduction, ainsi que des régions appropriées de régulation de la transcription. Il doit pouvoir être maintenu de façon stable dans la cellule hôte et peut éventuellement posséder des signaux particuliers spécifiant la sécrétion de la protéine traduite. Ces différents éléments sont choisis en fonction de l'hôte cellulaire utilisé. A cet effet, les séquences nucléotidiques selon l'invention peuvent être insérées dans des vecteurs à réplication autonome au sein de l'hôte choisi, ou des vecteurs intégratifs de l'hôte choisi.

De tels vecteurs seront préparés selon les méthodes couramment utilisées par l'homme du métier, et les clones en résultant pourront être introduits dans un hôte approprié par des méthodes standard, telles que par exemple la lipofection, l'électroporation, le choc thermique.

Les vecteurs selon l'invention sont par exemple des vecteurs d'origine plasmidique ou virale.

Ces vecteurs sont utiles pour transformer des cellules hôtes afin de cloner ou d'exprimer les séquences nucléotidiques de l'invention.

L'invention comprend également les cellules hôtes transformées par un vecteur selon l'invention.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

L'hôte cellulaire peut être choisi parmi des systèmes procaryotes ou eucaryotes; comme par exemple les cellules bactériennes (Olins et Lee, 1993), mais également les cellules de levure (Buckholz, 1993), de même que les cellules animales, en particulier les cultures de cellules de mammifères (Edwards et Aruffo, 1993), et notamment les cellules d'ovaire de hamster chinois (CHO), mais également les cellules d'insectes dans lesquelles on peut utiliser des procédés mettant en oeuvre des baculovirus par exemple (Luckow, 1993).

Une cellule hôte préférée pour l'expression des protéines de l'invention est constituée par les cellules procaryotes, telles que les bactéries Gram.

L'invention concerne également les animaux, excepté l'Homme, comprenant une desdites cellules transformées selon l'invention.

L'obtention d'animaux transgéniques selon l'invention surexprimant un ou plusieurs des gènes de Pyrococcus *abyssi* sera menée de façon préférée sur des rats, des souris ou des lapins selon des méthodes bien connues de l'homme de l'art telles que par transfections, virales ou non virales. Les animaux transgéniques surexprimant l'un ou plusieurs desdits gènes pourront être obtenus par transfection de copies multiples desdits gènes sous contrôle d'un promoteur puissant de nature ubiquitaire, ou sélectif d'un type de tissu. Les animaux transgéniques pourront être également obtenus par recombinaison homologue sur cellules souches embryonnaires, transfert de ces cellules souches à des embryons, sélection des chimères affectées au niveau des lignées reproductrices, et croissance desdites chimères.

Les cellules transformées ainsi que les animaux transgéniques selon l'invention sont utilisables dans des procédés de préparation de polypeptide recombinant.

Il est aujourd'hui possible de produire des polypeptides recombinants en quantité relativement importante par génie génétique en utilisant les cellules transformées par des vecteurs d'expression selon l'invention ou en utilisant des animaux transgéniques selon l'invention.

Les procédés de préparation d'un polypeptide de l'invention sous forme recombinant, caractérisés en ce qu'ils mettent en oeuvre un vecteur et/ou une cellule transformée par un vecteur selon l'invention et/ou un animal transgénique comprenant une desdites cellules transformées selon l'invention, sont eux-mêmes compris dans la présente invention.

Parmi lesdits procédés de préparation d'un polypeptide de l'invention sous forme recombinante, on préfère les procédés de préparation mettant en oeuvre un vecteur, et/ou une cellule transformée par ledit vecteur et/ou ou un animal transgénique comprenant une desdites cellules transformées, contenant une séquence nucléotidique codant pour un polypeptide de Pyrococcus *abyssi,* ou un de ses fragments selon l'invention.

Parmi lesdits procédés de préparation d'un polypeptide de l'invention sous forme recombinante, on préfère également les procédés de préparation mettant en oeuvre un vecteur, et/ou une cellule transformée par ledit vecteur et/ou un animal transgénique comprenant une desdites cellules transformées, contenant une séquence nucléotidique codant pour un polypeptide de Pyrococcus *abyssi,* ou un de ses fragments, de Pyrococcus *abyssi,* ou un de ses fragments, à activité ADN polymérase.

Les polypeptides recombinants obtenus comme indiqué ci-dessus, peuvent aussi bien se présenter sous forme glycosylée que non glycosylée et peuvent présenter ou non la structure tertiaire naturelle.

Une variante préférée consiste à produire un polypeptide recombinant fusionné à une protéine «porteuse» (protéine chimère). L'avantage de ce système est qu'il permet une stabilisation et une diminution de la protéolyse du produit recombinant, une augmentation de la solubilité au cours de la renaturation in vitro et/ou une simplification de la purification lorsque le partenaire de fusion possède une affinité pour un ligand spécifique.

L'invention décrit un procédé de préparation d'un polypeptide de l'invention comprenant les étapes suivantes :
a) culture des cellules transformées dans des conditions permettant l'expression d'un polypeptide recombinant de séquence d'acide nucléique selon l'invention ;
b) le cas échéant, récupération dudit polypeptide recombinant.

Lorsque le procédé de préparation d'un polypeptide de l'invention met en oeuvre un animal transgénique selon l'invention, le polypeptide recombinant est ensuite extrait dudit animal.

L'invention a aussi pour objet un polypeptide recombinant susceptible d'être obtenu par un procédé de l'invention tel que décrit précédemment.

L'invention comprend aussi un procédé de préparation d'un polypeptide synthétique, caractérisé en ce qu'il utilise une séquence d'acides aminés de polypeptides selon l'invention:

L'invention concerne également un polypeptide synthétique obtenu par un procédé selon l'invention.

Les polypeptides selon l'invention peuvent également être préparés par les techniques classiques, dans le domaine de la synthèse des peptides. Cette synthèse peut être réalisée en solution homogène ou en phase solide.

Par exemple, on aura recours à la technique de synthèse en solution homogène décrite par Houbenweyl en 1974.

Selon une autre technique préférée de l'invention, on a recours à celle décrite par Merrifield.

L'invention est en outre relative à des polypeptides hybrides présentant au moins un polypeptide ou un de ses fragments selon l'invention.

Les procédés de synthèse des molécules hybrides englobent les méthodes utilisées en génie génétique pour construire des séquences nucléotidiques hybrides codant pour les séquences polypeptidiques recherchées. On pourra, par exemple, se référer avantageusement à la technique d'obtention de gènes codant pour des protéines de fusion décrite par Minton en 1984.

Lesdites séquences nucléotidiques hybrides codant pour un polypeptide hybride ainsi que les polypeptides hybrides selon l'invention caractérisés en ce qu'il s'agit de polypeptides recombinants obtenus par l'expression desdites séquences nucléotidiques hybrides, font également partie de l'invention.

L'invention comprend également les vecteurs caractérisés en ce qu'ils contiennent une desdites séquences nucléotidiques hybrides. Les cellules hôtes transformées par lesdits vecteurs, les animaux transgéniques comprenant une desdites cellules transformées ainsi que les procédés de préparation de polypeptides recombinants utilisant lesdits vecteurs, font bien entendu également partie de l'invention.

Comme il l'a été précédemment mentionné, les séquences nucléotidiques décrites ont été identifiées par homologie avec des séquences connues comme codant par exemple pour des polypeptides ou des fragments de polypeptides enzymatiques impliqués dans la biosynthèse ou la biodégradation de molécules organiques ou inorganiques.

Il est ainsi possible d'utiliser lesdits polypeptides de l'invention de manière similaire pour la biosynthèse ou la biodégradation de composés organiques ou inorganiques présentant un intérêt industriel ou thérapeutique.

Compte tenu du caractère hyperthermophile de Pyrococcus *abyssi,* l'activité et la stabilité de ces enzymes à de telles températures (entre 80°C et plus de 110°C) pourront être avantageusement exploitées dans ces procédés.

Les procédés de biosynthèse ou de biodégradation de composés organiques ou inorganiques, caractérisés en ce qu'ils mettent en oeuvre un polypeptide ou un de ses fragments selon l'invention, font aussi partie de l'invention.

Les polypeptides à activité ADN polymérase selon l'invention, tels que les polypeptides à activité ADN polymérase de type II décrits dans les exemples ci- après, peuvent avantageusement être mis en oeuvre dans des procédés nécessitant l'amplification d'un acide nucléique, ou dans les utilisations, méthodes ou applications mentionées dans les exemples.

Ces polypeptides à activité ADN polymérase selon l'invention peuvent être mis en oeuvre dans un procédé pour la polymérisation et/ou l'amplification d'acide nucléique cible dans un échantillon biologique (tissu, cellule ou fluide biologique), caractérisé en ce qu'il comprend l'étape suivante :
a) mise en contact de cet échantillon biologique avec un polypeptide, ou un de ses fragments, à activité ADN polymérase selon l'invention dans des conditions permettant une réaction de polymérisation et/ou d'amplification entre ledit polypeptide et les acides nucléiques présents dans l'échantillon biologique.

L'invention concerne également un réactif ou un kit (ou nécessaire) comprenant un polypeptide, ou un de ses fragments, à activité ADN polymérase selon l'invention pour la polymérisation et/ou l'amplification d'acide nucléique cible dans un échantillon biologique.

De tels réactifs ou kits pourront en particulier être utililisés pour la détection et/ou l'identification d'acide nucléique cible, notamment pour la détection et/ou l'identification d'acide nucléique spécifique de micro-organisme pathogène, telle que des bactéries, des levures, des parasites ou des virus, ou encore pour la détection et/ou l'identification de gènes d'intérêt, ou de mutation parmi ces gènes d'intérêt, ainsi que pour les utilisations, méthodes ou applications mentionées dans les exemples ci- après relatifs à l'ADN polymérase de type II.

D'autres caractéristiques et avantages de l'invention apparaissent dans la suite de la description avec les exemples et les figures dont les légendes sont représentées ci-après.

### Légendes des Figures

Figure 1 : Caractérisation des deux sous-unités du facteur C de réplication recombinant obtenu par co-expression dans *E.coli* MC1061 (DE3)
   Gel SDS/PAGE à 10 %. Lignes 1+2+3+4 : 2 min-95°C ; lignes 1+2 : 15 min-60°C ; lignes 3+4 : 15 min-80°C ; lignes 1+3 : clone induit; lignes 2+4 : témoin cellules.

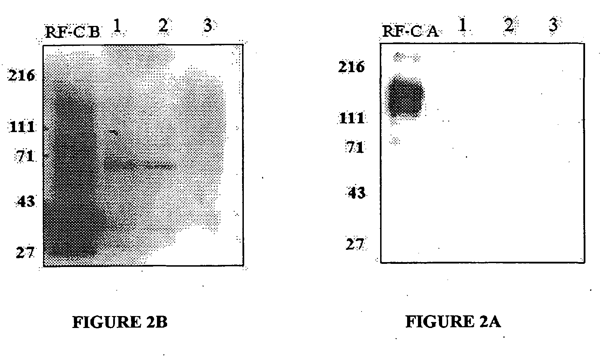

Figures 2A et 2B : Caractérisation de la grande sous-unité du facteur C de réplication recombinant par autoradiographie de l'immunoblot (Western Blot) avec les anticorps A (figure 2A) et B (figure 2B)
   RF-C A et RF-C B : réaction avec la grande sous-unité du RF-C de thymus de veau (témoin).
   ligne 1 : fraction exprimée dans la partie insoluble (culot) ;
   ligne 2: fraction exprimée dans la fraction soluble (surnageant) ;
   ligne 3: fraction soluble d'un clone non induit.

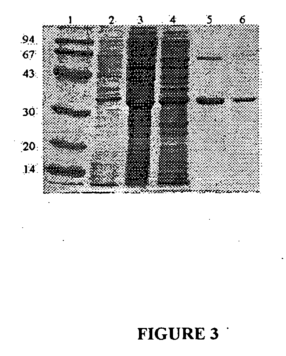

Figure 3 : Caractérisation du polypeptide PCNA recombinant
   Gel d'électrophorèse : ligne 1 : LMW : ligne 2 : non induit ; ligne 3 : induit ; ligne 4 : 75°C, 15 min ; ligne 5 : ressource Q ; ligne 6 : superdex 200.
Figure 4: Effets du polypeptide PCNA sur l'ADN polymérase I de Pyrococcus abyssi (pol I).

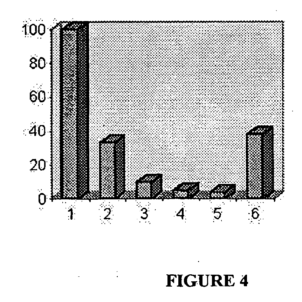

   La figure 4 représente l'incorporation en 3H-TTP utilisant la matrice OligoF/lambda en fonction de la quantité de polI avec ou sans PCNA ; 1 : 2U de pol I ; 2 : 1U de pol I; 3 : 0,5 U de pol I ; 4 : 0,2U de pol I; 5 : 0,1U de pol I; 6 : 0,1U de pol I + 500 ng PCNA.

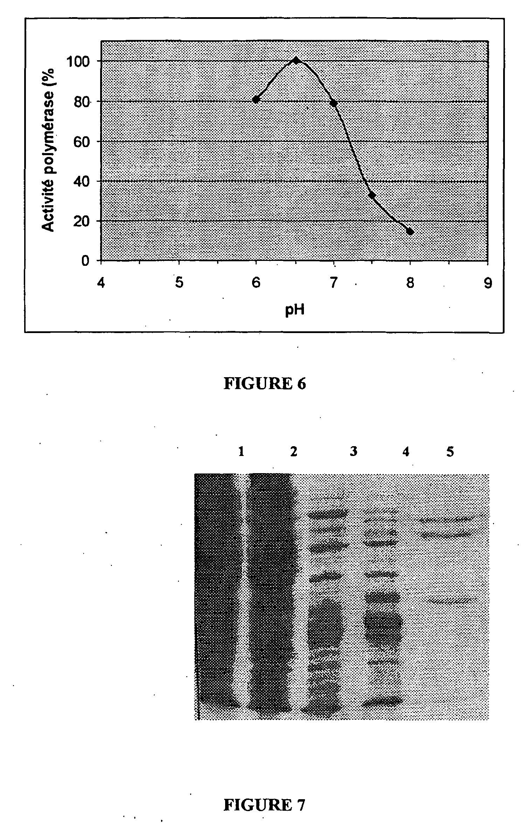

Figure 5 : Caractérisation des deux sous-unités polB et polC recombinant obtenues par co-expression dans *E. coli* HMS174(DE3)
   Visualisation des extraits obtenus sur SDS-PAGE à 10 % :
   - ligne 1 : LMW ;
   - lignes 2, 3, 4 : clones co-exprimant les 2 sous-unités (polB et polC) de l'ADN polymérase II induit 15 h à 37°C après dénaturation thermique 15 min à 80°C
   - ligne 5 : témoin HMS induit 15 h à 37°C après dénaturation thermique 15 min à 80°C ;
   - ligne 6 : LMW ("Low Molecular Weight", bas poids moléculaires).
Figure 6 : Activité polymérase de l'ADN polymérase Il en fonction du pH exprimée en pourcentage de l'activité obtenue pour le pH optimal à 6,5.
Figure 7 : Caractérisation de la protéine de réplication A recombinante exprimée dans *E*. *coli* B121 pLys S(DE3)
   Gel d'électrophorèse SDS-PAGE à 10 % :
   - ligne 1 : non induit 15 h à 37°C ;
   - ligne 2 : induit 15 h à 37°C ;
   - ligne 3 : non induit 15 h à 37°C après dénaturation thermique 15 min à 75°C ;
   - ligne 4 : induit 15 h à 37°C après dénaturation thermique 15 min à 75°C ;
   - ligne 5: HMW(pharmacia).

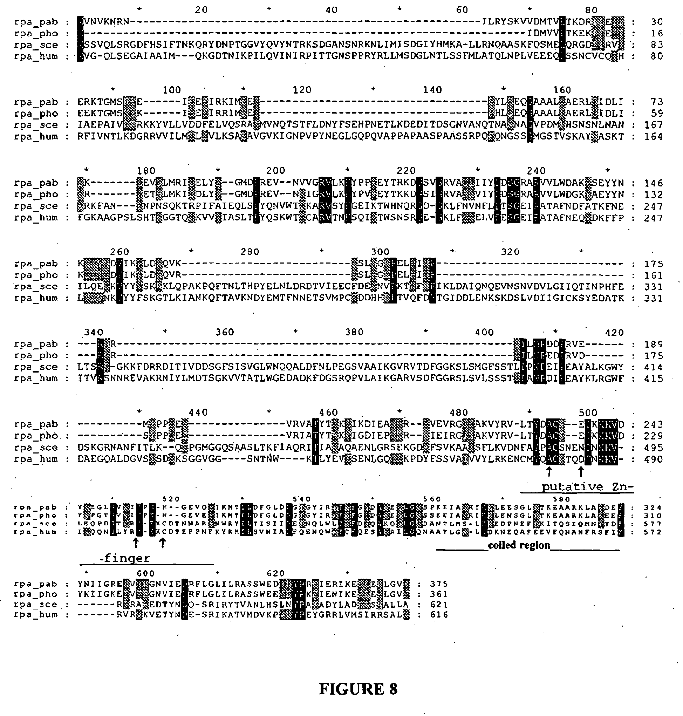

Figure 8 : Alignement des séquences de RPA de *Pyrococcus abyssi, Pyrococcus horikochii* et de la RP70 de *Saccharomyces cerevisiae* et d'origine humaine.

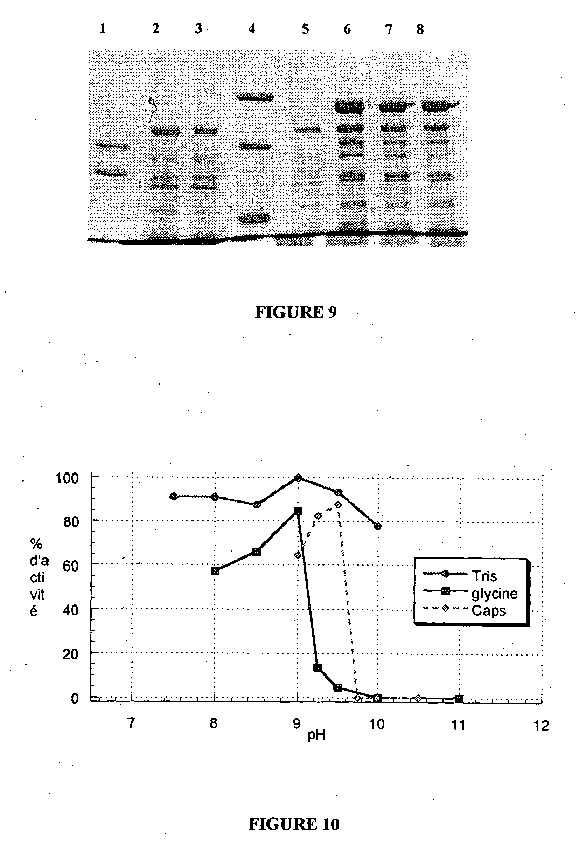

Figure 9 : Caractérisation de l'ADN polymérase I recombinante exprimée dans *E*. *coli* B121(DE3) pLys S
   Gel d'électrophorèse :
   - ligne 1 : LMW ;
   - ligne 2 : Témoin : *E. coli* BL21(DE3) pLysS non induit ;
   - ligne 3 : Témoin : E. *coli* BL21(DE3) pLysS ;
   - ligne 4 : LMW ;
   - ligne 5 : ADN polymérase I non induit ;
   - ligne 6 : ADN polymérase I induit 15 h à 37°C induit 15 h, IPTG 0; 1mM ;
   - ligne 7 : ADN polymérase I induit 15 h à 37°C induit 15 h, IPTG 0,5mM ;
   - ligne 8 : ADN polymérase I induit 15 h à 37°C induit 15 h, IPTG 1 mM.
Figure 10: Effet du pH dans différents tampons sur l'activité polymérase de l'ADN polymérase 1 recombinante.

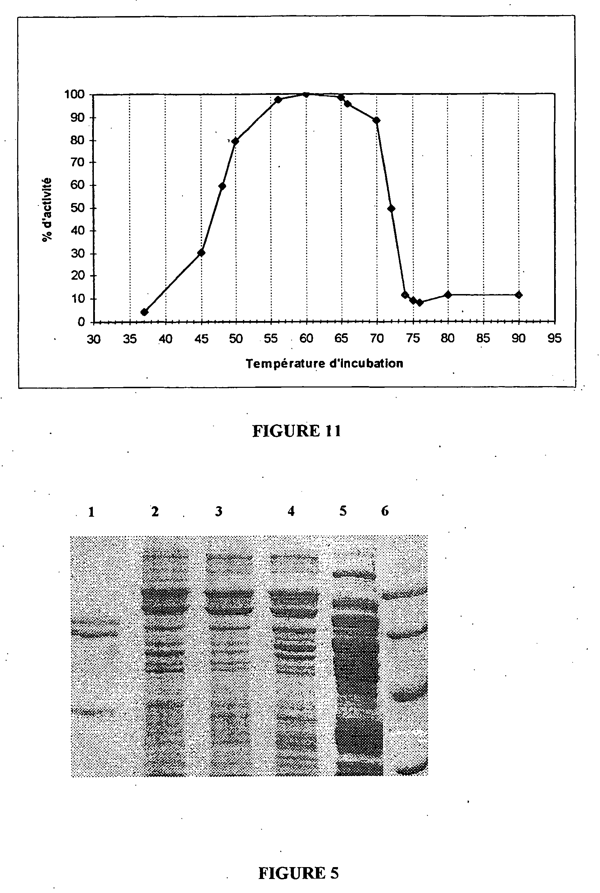

Figure 11 : Activité selon la température d'incubation de l'ADN polymérase 1 recombinante exprimée en pourcentage de l'activité obtenue à la température optimale.

### Procédures expérimentales

### 1. Cellules

La souche de Pyrococcus *abyssi* ORSAY utilisée est la souche déposée le 9 Avril 1999 à la Collection Nationale de Culture de Microorganismes, INSTITUT PASTEUR, Paris, France, sous le numéro I-2180.

### 2. Culture des cellules

La souche de Pyrococcus *abyssi* ORSAY est cultivée dans les conditions et sur un milieu de culture tels que décrit par Erauso et all. (Arch. Microbiol.,160, 338-349, 1993 ; J. Bact., 178 (11), 3232-3237, 1996).

### 3. Purification de Pyrococcus abyssi

Après culture, les cellules de Pyrococcus *abyssi* sont récoltées par centrifugation à basse vitesse et à 4°C, selon le protocole décrit dans l'ouvrage de Sambrook et al., 1989.

### 4. Préparation de l'ADN

L'ADN sous forme soluble est préparé selon la méthode de lyse alcaline décrite dans l'ouvrage de Sambrook et al., 1989, suivie de deux extractions au phénol/chloroforme, deux extractions au chloroforme, une élimination des ARN par traitement à la RNAse puis une concentration de l'ADN par précipitation éthanolique. L'ADN est repris en TRIS-EDTA (20 mM - 1 mM) et dosé.

L'ADN immobilisé en bloc d'agarose est préparé de la manière suivante : après une culture dans les conditions décrites plus haut jusqu'à une DO 600 nm de 0,3, les cellules sont refroidies dans la glace puis récupérées par centrifugation à basse vitesse, à 4°C. Le culot de cellules est lavé deux fois dans le milieu de culture, une fois en 50 mM EDTA pH 8,0, une fois en EDTA 125 mM pH 8,0, et centrifugé après chaque lavage. Les cellules sont alors resuspendues en 125 mM EDTA pH 8,0 à 37°C puis un volume égal d'agarose à bas point de fusion (Sigma Type VII) préparé à 1,6 % en eau déionisée et tempéré à 37°C. Le mélange est coulé dans des moules de 0,1 ml et laissé à température ambiante pour permettre la gélification des inclusions cellules-agarose. Les blocs d'inclusion sont démoulés et traités en tampon ESP (0,5 M EDTA pH 9,0, 1 % Lauroyl sarcosine et 1 mg/ml Protéinase K) pendant 48 heures, à 37°C, puis conservés à 4°C. Les blocs d'inclusion sont lavés en TRIS-EDTA (10 mM - 1 mM) + 0,250 mg/ml PMSF, de manière extensive (4 à 6 fois 2 à 4 heures) avant utilisation.

### EXEMPLES : SEQUENCAGE ET ANALYSE DU GENOME DE PYROCOCCUS ABYSSI

### Exemple 1 : STRATEGIE DE SEQUENCAGE

Le séquençage du génome de P. *abyssi,* dont la taille était estimée à 1,8 Mb, a été réalisé par une approche globale ne nécessitant pas la constitution a priori d'une cartographie de préséquençage, ni l'établissement préalable, toujours laborieux, d'un contig de sous-clones de grande taille. La stratégie retenue fait intervenir une phase aléatoire caractérisée par le séquençage d'extrémités appariées (Roach et al., Genomics, 26 : 345, 1995), provenant de clones sélectionnés au hasard, suivie d'une phase dirigée assurant la complétion de la séquence. L'opération a été réalisée en 3 temps :
1. Construction d'une banque plasmidique (12 000 clones) dans pBAM3/DH10B, 1a taille moyenne d'insertion de 5 à 6kb, a été choisie comme compromis entre les exigences d'efficacité de clonage liées à la production à haut débit d'une part, les performances d'assemblage et le souci d'éviter une sur-représentation des séquences du plasmide pGT5 (3,4 kb) d'autre part.
   Un échantillon de 20 µg de l'ADN, ont été soumis à une digestion partielle par l'enzyme CviJI (CHIMERx-Ozyme), dans un volume réactionnel de 300 µl. La température d'incubation à été réduite à 15°C, afin de mieux contrôler la progression de la réaction. Six prélèvements de 50 µl ont été effectués (après 2mn, 5mn, 10mn, 20mn, 45mn et 90mn d'incubation) et la réaction a été immédiatement stoppée par addition d'EDTA et de SDS (20 mM et 0,2 % final, respectivement). Après analyse par électrophorèse d'une aliquote (2 µl), les prélèvements correspondant aux états de digestion partielle jugés adéquats ont été rassemblés, leur ADN précipité à l'éthanol, puis soumis à une séparation préparative sur gel d'agarose à bas point de fusion 0,4 % (LMP, Seaplaque GTG, FMC). La bande de gel correspondant à la gamme de taille de fragments comprise entre 5 et 6 kb a été découpée et l'ADN purifié par fusion de l'agarose LMP (5 mn à 65°C) puis incubation de 4 h à 40°C en présence de β-agarase (Biolabs, 1 unité pour 100 µl d'agarose). L'ADN extrait a été précipité à l'éthanol et resuspendu dans 20 µl d'eau stérile et dosé. 500 ng de ces fragments d'ADN ont été ligaturés à un excès molaire de 5 fois d'un vecteur plasmidique à bouts francs, comme par exemple Bluescript Il SK+ (Stratagene) coupé par Smal, déphophorylé. Après électroporation dans des bactéries DH10B et sélection sur milieu solide complémenté en carbéniciline (100 µg/ml), Xgal (40 µg/ml)et IPTG (20 µg/ml), 12 000 colonies blanches ont été repiquées en microplaques de 96 puits.
   Le séquençage de 19300 extrémités de ces clones plasmidiques est responsable de la majeure partie de l'assemblage.
2. Construction d'une banque cosmidique (4000 clones de taille d'insert 36 à 48 kb)
   L'ADN de la souche ORSAY, immobilisé en bloc d'agarose, a été libéré par fusion (à 65°C) et digestion de l'agarose par la béta-agarase, puis digéré partiellement par l'enzyme Sau3A selon la procédure décrite ci-dessus. La gamme des fragments de taille supérieure à 35 kb a été purifiée comme précédemment par électrophorèse préparative sur gel d'agarose LMP et l'ADN précipité à l'éthanol. Après déphosphorylation de leurs extrémités, ces fragments ont été ligaturés, au site BamHI, à un excès de 10 fois des bras d'un vecteur cosmidique, comme par exemple SuperCos1 (Stratagene). Après encapsidation in vitro, infection de la souche DH10B (Life technologies) et étalement sur boîtes de Pétri complémentées en carbéniciline (100 µg/ml), environ 4 000 clones cosmidiques ont été repiqués en microplaques de 96 puits.
   1635 séquences d'extrémités ont été produites à partir de ces clones cosmides et ont permis l'extension du squelette d'assemblage sur de plus longues distances. Environ 500 d'entre elles ont été réalisées sur des cosmides sélectionnés par hybridation à des sondes PCR issues des extrémités des contigs au cours de l'assemblage.
   Toutes les réactions de séquence correspondant à la phase aléatoire ont été obtenues par amorces fluorescentes (marquées à l'IRD700 et IRD800) situées aux bornes des sites de clonage et analysées sur LI-COR 4200 par le logiciel Image Analysis 3.0.
   L'assemblage a été réalisé en utilisant les logiciels PHRED et PHRAP développés par P. Green et coll. (Ewing et al., Genome Res., 8 : 175, 1998) et les données générées exploitées interactivement à l'aide de CONSED (Gordon et al., Genome Res., 8 : 195, 1998). A ce stade, 14 discontinuités de clonage subsistaient, pour une couverture en séquence d'environ 98 %. La comparaison des données de séquences avec le profil de digestion de l'ADN génomique pour l'enzyme NotI a permis d'étendre le squelette d'assemblage sur l'ensemble de la molécule. La couverture de ces 14 discontinuités de clonage (de 0,6 à 5 kb) ainsi que le renforcement des liens faibles ont été effectués par séquençage des produits obtenus par LA-PCR (Long and Acurate Polymerase Chain Reaction).
3. Etape de finition
   La phase aléatoire (« shotgun ») une fois terminée, le séquençage s'est poursuivi de manière dirigée afin d'assurer la continuité et la fiabilité de la séquence consensus, le taux d'erreur devant être en tout point inférieur à 10⁻⁴. Elle s'est déroulée en deux étapes :
   a) la liaison des contigs de séquence ;
   b) le lissage du consensus, devant satisfaire aux règles de qualités suivantes :
      - valeur de qualité en tout point du consensus, édité par CONSED, égale ou supérieure à 40 (qui correspond à une probabilité de 10⁴ pour 1),
      - 3 lectures au moins à chaque position dont au moins une par brin d'ADN, ou à défaut 3 lectures selon 2 chimies différentes (amorces marquées ou terminateurs marqués),
      - les 3 lectures ci-dessus doivent être issues du séquençage d'au moins 2 clones différents sauf dans le cas où les séquences sont réalisées directement sur génome.

Les amorces nucléotidiques ont été déterminées de manière semi-automatique à l'aide du logiciel PRIMO (Ping LI et al., Genomics, 40, 476-485, 1997).

Toutes les séquences de finition ont été réalisées en terminateurs fluorescents et analysées sur séquenceur ABI 377.

Le séquençage direct sur le génome de P. *abyssi* a été utilisé pour résoudre certaines lacunes de clonage et confirmer quelques sites variables et polymorphismes suspectés.

Une validation supplémentaire de l'assemblage réalisée par comparaison de la carte de restriction déduite de la séquence assemblée avec le profil de digestion observé sur l'ADN de *P. abyssi* pour les enzymes NotI et AscI. Une validation plus fine a été effectuée par « Southern blotting » de l'ADN de la souche ORSAY digéré par les erizymes BamHI, BgIII, EcoRI, HindIII, PstI eet Xbal, et hybridé à 33 sondes obtenues par LA6PCR, correspondant notamment aux 14 discontinuités de clonage observées.

Une vérification ultime du lissage a été réalisée par analyse des données d'annotation (intégrité des cadres de lecture).

### Exemple 2 : ANNOTATION DES SEQUENCES

L'annotation de la séquence de Pyrococcus *abyssi* suit un schéma simple: détermination des segments codants, détermination de régions codantes par similarité, estimation d'un modèle statistique d'une région codante, évaluation du potentiel codant des différentes région, détermination de la fonction de ces segments par comparaison au séquences contenues dans les banques de données de séquences du domaine public.

Une heuristique simple a été utilisée : elle consiste à déterminer toutes les segments maximaux allant d'un codon initiateur à un codon stop d'une longueur supérieure à 100 codons sens. On trouve 2342 tels cadres de lecture ouverts, qui ont été ensuite comparés à la banque de séquence protéique Swissprot (Bairoch A et Apweiler R., Nucleic Acids Res. 1999 27 : 49-54) par l'algorithme de blastp (Altschul et al., J. Mol. Biol. 1990, 215 : 403) de manière à construire une collection de séquences peptidiques présentant une similarité significative (plus de 30 % d'identité sur une longueur supérieure à 80 acides aminés) avec des protéines déjà connues. On trouve ainsi 883 régions significativement similaires, correspondant 719 cadres de lecture ouverts différentes. Un modèle de chaîne de Markov cachée à alors été dérivé des régions du génome encodant ces segments similaires. Ce modèle de Markov caché a alors servi à réévaluer chaque cadre de lecture ouvert au moyen du programme glimmer (Salzberg et al., Nucl. Ac. Res., 1998 26(2):544). A la fin de ce processus 1623 gènes potentiels sont proposés. Une fonction a été assignée à 790 d'entre eux en comparant les séquences peptidiques déduites de ces gènes avec la base de séquence COG (Tatusov et al., Science 1997, 278:631) obtenue au site ftp://ftp.ncbi.nlm.nih.gov/pub/COG, la banque Swissprot (Bairoch A et Apweiler R., Nucleic Acids Res., 1999 27 : 49-54), les séquences contenues dans la banque de séquence TIGR database (domaine public, obtenue aux sites :
ftp://ftp.tigr.org/pub/data/a_fulgidus/GAF.pep.gz,
ftp://ftp.tigr.org/pub/data/h_influenzae/GHI.pep.gz,
ftp://ftp.tigr.org/pub/data/h_pylori/GHP.pep.gz,
ftp://ftp.tigr.org/pub/data/m_janashii/GMH.pep.gz,
ftp://ftp.tigr.org/pub/data/m_genitalium/GMG.pep.gz), et dans la cyanobase
(ftp://ftp.kazusa.or.jp/pub/cyano/cyano.p.aa.gz).

### Banques de données

Le logiciel BLAST (domaine public, Altschul et al., 1990) a été utilisé pour recherche d'homologies entre une séquence et des banques de données protéiques ou nucléiques. Les principales banques de données publiques ont été utilisées.

Les résultats de recherche d'homologies entre une séquence selon l'invention et des banques de données protéiques ou nucléiques sont présentés et résumés dans les tableaux 1 et 2 ci-après.

### Tableau 1 : Liste des fonctions putatives des protéines de séquences SEQ ID N° 1 à SEQ ID N° 791 codées par leur séquence nucléotidique respective ORF 1 à ORF 758 du génome de Pyrococcus abyssi ORSAY

### Légende du tableau 1 :

L'ensemble des fonctions putatives associées aux protéines codées par les ORF 1 à ORF 791 a été obtenu par une recherche d'homologie utilisant le logiciel BLASTP (Altschul et al., 1990). Seules ont été prises en compte les homologies présentant un degré d'identité présentant une valeur de P calculée par blastp inférieure à 10⁻⁵. La description de la fonction la plus probable est donnée dans la colonne Fonction ; la séquence protéique associée à cette fonction est indiquéee dans la colonne SEQ ID.

**TABLEAU 1**

| SEQ ID | FONCTION |
|---|---|
| SEQ ID N° 2 | TPR-repeat protéines |
| SEQ ID N° 3 | 4-hydroxybenzoate octaprenyltranférase |
| SEQ ID N° 4 | Zn-dépendantes hydrolases, incluant les glyoxylases |
| SEQ ID N° 5 | Histidinol-phosphate aminotransférase |
| SEQ ID N° 6 | 30S ribosomal protéine S15 |
| SEQ ID N° 7 | Exonucléase ADN Simple-brin-specifique |
| SEQ ID N° 8 | Protéine de membrane intégrale Predite |
| SEQ ID N° 9 | D-STEREOSPECIDIC AMINOPEPTIDASE (EC 3.4.11.19) |
| SEQ ID N° 10 | AGMATINASE or ARGINASE (EC 3.5.3.11 or 3.5.3.1) |
| SEQ ID N° 11 | proteine de division cellulaire (membrane) |
| SEQ ID N° 12 | Pyruvate kinase |
| SEQ ID N° 13 | Fe-S oxidoréductases |
| SEQ ID N° 14 | Hypoxanthine-guanine phosphoribosyltransferase |
| SEQ ID N° 15 | Alanyl-tRNA synthétase |
| SEQ ID N° 16 | FACTOR C DE REPLICATION, sous-unité |
| SEQ ID N° 17 | FACTOR C DE REPLICATION REPLICATION FACTOR C, grande sous-unité |
| SEQ ID N° 18 | PROTEINE HYPOTHETIQUE SIMILAIRE A LA PHOSPHOSERINE PHOSPHATASE (EC 3.1.3.3) |
| SEQ ID N° 19 | Domaine transmembranaire Glutathione-dependent Na/H antiporters - transmembrane |
| SEQ ID N° 20 | Aminotransférases Pyridoxal phosphate-dependante |
| SEQ ID N° 21 | PROTEINE FIXANT LA PENICILLINE SIMILAIRE A LA BETA-LACTAMASE (EC 3.5.2.6) |
| SEQ ID N° 22 | Dihydroorotase |
| SEQ ID N° 23 | Protéines périplasmiques fixant un dipeptide(et nickel)-binding |
| SEQ ID N° 24 | Oligopeptide/nickel permeases |
| SEQ ID N° 25 | Permeases, primarily amino acid and peptide |
| SEQ ID N° 26 | ATPase subunits of ABC transporters |
| SEQ ID N° 27 | ATPase subunits of ABC transporters |
| SEQ ID N° 28 | BIOTIN-[ACETYL-COA-CARBOXYLASE] LIGASE (EC 6.3.4.15) |
| SEQ ID N° 29 | Molybdenum-binding periplasmic protein !! |
| SEQ ID N° 30 | ATPase subunits of ABC transporters |
| SEQ ID N° 31 | ATPase subunits of ABC transporters |
| SEQ ID N° 32 | Dehydrogenases (flavoproteins) |
| SEQ ID N° 33 | Molybdopterin converting factor |
| SEQ ID N° 34 | Recombinase (RecA) and ATPase domain of putative ATP-dependent protease |
| SEQ ID N° 35 | Recombinase (RecA) and ATPase domain of putative ATP-dependent protease |
| SEQ ID N° 36 | FUCULOSE-PHOSPHATE ALDOLASE (EC 4.1.2.17) |
| SEQ ID N° 37 | ALPHA-AMYLASE ou 4-ALPHA-GLUCANOTRANSFERASE (EC 3.2.1.1 ou 2.4.1.25) |
| SEQ ID N° 38 | Permeases, mainly amino acid |
| SEQ ID N° 39 | Sugar permeases |
| SEQ ID N° 40 | AMYLOPULLULANASE (EC 3.2.1.1 / 3.2.1.41) |
| SEQ ID N° 41 | ATPase subunits of ABC transporters. |
| SEQ ID N° 42 | Superfamily II DNA and RNA helicases |
| SEQ ID N° 43 | Permeases |
| SEQ ID N° 44 | Glutamine amidophosphoribosyltransferase and other predicted amidophosphoribosyltransferases |
| SEQ ID N° 45 | Zn proteases and inactivated homologs |
| SEQ ID N° 46 | Zn proteases and inactivated homologs |
| SEQ ID N° 47 | Pyridoxal phosphate-dependent enzymes |
| SEQ ID N° 48 | Recombinase (RecA) and ATPase domain of putative ATP-dependent protease |
| SEQ ID N° 49 | 3'-phosphoadenosine 5'-phosphosulfate sulfotransferase (PAPS reductase) |
| SEQ ID N° 50 | Predicted exopolyphosphatase |
| SEQ ID N° 51 | Dihydropteroate synthase |
| SEQ ID N° 52 | Predicted dehydrogenase |
| SEQ ID N° 53 | NAD(FAD)-utilizing dehydrogenases |
| SEQ ID N° 54 | ELONGATION FACTOR 2 |
| SEQ ID N° 55 | Inositol monophosphatase and related sulfite synthesis enzyme |
| SEQ ID N° 56 | Superfamily II DNA and RNA helicases |
| SEQ ID N° 57 | Succinyl-CoA synthetase, alpha subunit-related enzymes |
| SEQ ID N° 58 | Predicted phosphatases |
| SEQ ID N° 59 | Predicted phosphoesterase . |
| SEQ ID N° 60 | CBS domain proteins |
| SEQ ID N° 61 | Ferredoxins |
| SEQ ID N° 62 | Ferredoxins |
| SEQ ID N° 63 | D-amino acid dehydrogenase |
| SEQ ID N° 64 | PP-loop superfamily ATPase |
| SEQ ID N° 65 | CTP SYNTHASE (EC 6.3.4.2) |
| SEQ ID N° 66 | Predicted permeases |
| SEQ ID N° 67 | Dehydrogenases with different specificities (related to short-chai alcohol dehydrogenases) |
| SEQ ID N° 68 | NAD-dependent K+ and Na+ uptake proteins |
| SEQ ID N° 69 | High-affinity branched amino acid transport system ATPase |
| SEQ ID N° 70 | rRNA (adenosine-N6,N6-)-dimethyltransferase |
| SEQ ID N° 71 | INTEGRASE / RECOMBINASE |
| SEQ ID N° 72 | Glyceraldehyde-3-phosphate dehydrogenase |
| SEQ ID N° 73 | Methylated DNA-protein cysteine methyltransferase |
| SEQ ID N° 74 | Uncharacterized ACR (EMAP domain) |
| SEQ ID N° 75 | HYPOTHETICAL TYROSINE SPECIFIC PROTEIN PHOSPHATASE (EC 3.1.3.48) |
| SEQ ID N° 76 | RIBOKINASE (EC 2.7.1.15) |
| SEQ ID N° 77 | SAM-dependent methyltransferases |
| SEQ ID N° 78 | SAM-dependent methyltransferases |
| SEQ ID N° 79 | Isopropylmalate/homocitrate synthases |
| SEQ ID N° 80 | 3-isopropylmalate dehydratase/aconitase large subunit (domain) |
| SEQ ID N° 81 | 3-isopropylmalate dehydratase/aconitase small subunit (domain) |
| SEQ ID N° 82 | Isopropylmalate dehydrogenase |
| SEQ ID N° 83 | Acetylglutamate semialdehyde dehydrogenase |
| SEQ ID N° 84 | Acetylglutamate kinase |
| SEQ ID N° 85 | ACETYLORNITHINE DEACETYLASE or SUCCINYL-DIAMINOPIMELATE DESUCCINYLASE (EC 3.5.1.16 or 3.5.1.18) |
| SEQ ID N° 86 | Transketolase/pyruvate dehydrogenase E1 subunit |
| SEQ ID N° 87 | Transketolase/pyruvate dehydrogenase E1 subunit |
| SEQ ID N° 88 | 7-PHOSPHO-2-DEHYDRO-3-DEOXY-D-ARABINO-HEPTONATE synthase (DAHP synthase)/3-deoxy-D-manno-octulosonic acid 8-phosphate synthetase |
| SEQ ID N° 89 | 3-dehydroquinate synthetase |
| SEQ ID N° 90 | 3-dehydroquinate dehydratase |
| SEQ ID N° 91 | Shikimate 5-dehydrogenase |
| SEQ ID N° 92 | High-affinity branched amino acid transport system ATPase |
| SEQ ID N° 93 | Carbohydrate and amino acid permeases |
| SEQ ID N° 94 | Carbohydrate and amino acid permeases |
| SEQ ID N° 95 | 5-enolpyruvylshikimate-3-phosphate synthase |
| SEQ ID N° 96 | Chorismate synthase |
| SEQ ID N° 97 | INTRACELLULAR PROTEASE I (EC 3.4.-.-) |
| SEQ ID N° 98 | Fe-S oxidoreductases |
| SEQ ID N° 99 | DNA primase (bacterial type) and small primase-like proteins |
| SEQ ID N° 100 | Phosphgluçomutase/phosphomannomutase |
| SEQ ID N° 101 | Thymidylate kinase |
| SEQ ID N° 102 | Signal recognition particle GTPase |
| SEQ ID N° 103 | Uncharacterized ACR! 3 |
| SEQ ID N° 104 | Glutamyl- and glutaminyl-tRNA synthetases |
| SEQ ID N° 105 | Cation transporters |
| SEQ ID N° 106 | Dehydrogenases with different specificities (related to short-chai alcohol dehydrogenases) |
| SEQ ID N° 107 | Uncharacterized ACR |
| SEQ ID N° 108 | Predicted Zn-dependent hydrolases (beta-lactmase superfamily) |
| SEQ ID N° 109 | Ferredoxin oxidoreductases |
| SEQ ID N° 110 | Ferredoxin oxidoreductases |
| SEQ ID N° 111 | Ferredoxin oxidoreductases |
| SEQ ID N° 112 | Ferredoxin oxidoreductases |
| SEQ ID N° 113 | Ferredoxin oxidoreductases |
| SEQ ID N° 114 | Ferrichrome transport proteins |
| SEQ ID N° 115 | ATPase subunits of ABC transporters |
| SEQ ID N° 116 | RIBONUCLEASE H (EC 3.1.24.4) |
| SEQ ID N° 117 | Pseudouridine synthase |
| SEQ ID N° 118 | SAM-dependent methyltransferases |
| SEQ ID N° 119 | 30S ribosomal protein S13 |
| SEQ ID N° 120 | 30S ribosomal protein S4 and related proteins |
| SEQ ID N° 121 | 30S ribosomal protein S11 |
| SEQ ID N° 122 | 50S ribosomal protein L13 |
| SEQ ID N° 123 | 30S ribosomal protein S9 |
| SEQ ID N° 124 | Enolase |
| SEQ ID N° 125 | 30S ribosomal protein S2 |
| SEQ ID N° 126 | Threonine dehydratase/Threonine synthase |
| SEQ ID N° 127 | Galactokinase |
| SEQ ID N° 128 | Aspartokinases |
| SEQ ID N° 129 | UDP-N-acetyl-D-mannosaminuronic acid dehydrogenase/UDP-glucose 6-dehydrogenase |
| SEQ ID N° 130 | UDP-N-acetylgIucosamine 2-epimerase |
| SEQ ID N° 131 | Glycyl-tRNA synthetase |
| SEQ ID N° 132 | HYPOTHETICAL PROTEIN |
| SEQ ID N° 133 | Predicted ATPases |
| SEQ ID N° 134 | Glutathione-dependent Na/H antiporters - transmembrane domain |
| SEQ ID N° 135 | Glutamate dehydrogenase |
| SEQ ID N° 136 | Carbonic anhydrases homologous to acetyltransferases of the isoleucine patch superfamily |
| SEQ ID N° 137 | Uncharacterized ACR (translation?) |
| SEQ ID N° 138 | Zn finger protein (hydrogenase expression regulation?) |
| SEQ ID N° 139 | ATPases involved in chromosome partitioning |
| SEQ ID N° 140 | Hydrogenase formation factor |
| SEQ ID N° 141 | Serine/Threonine protein kinases |
| SEQ ID N° 142 | S1-type RNA-binding domain |
| SEQ ID N° 143 | PROTEASOME (Multicatalytic endopeptidase complex), alpha |
| | subunit (EC 3.4.99.46) |
| SEQ ID N° 144 | RNase PH (tRNA nucleotidyltransferase) |
| SEQ ID N° 145 | DNA-DEPENDENT RNA POLYMERASE, subunit B (EC 2.7.7.6) |
| SEQ ID N° 146 | DNA-DEPENDENT RNA POLYMERASE, subunit A' (EC 2.7.7.6) |
| SEQ ID N° 147 | DNA-DEPENDENT RNA POLYMERASE, subunit A" (EC 2.7.7.6) |
| SEQ ID N° 148 | Transcription terminationlantitermination factor |
| SEQ ID N° 149 | 30S ribosomal protein S12 |
| SEQ ID N° 150 | 30S ribosomal protein S7 |
| SEQ ID N° 151 | Uncharacterized ACR |
| SEQ ID N° 152 | ATPase components of ABC transporters with duplicated ATPase domains |
| SEQ ID N° 153 | Predicted integral membrane protein |
| SEQ ID N° 154 | HYPOTHETICAL PROTEIN SIMILAR TO ENDOGLUCANASE |
| SEQ ID N° 155 | Cytosine deaminase and related predicted metal-dependent hydrolases |
| SEQ ID N° 156 | ENDONUCLEASE III (EC 4.2.99.18 (anc. 3.1.25.2)) |
| SEQ ID N° 157 | Predicted permeases |
| SEQ ID N° 158 | ATPases involved in DNA repair |
| SEQ ID N° 159 | ELONGATION FACTOR 1-ALPHA |
| SEQ ID N° 160 | 30S ribosomal, protein S10 |
| SEQ ID N° 161 | Arginyl-tRNA synthetase |
| SEQ ID N° 162 | Sugar kinases |
| SEQ ID N° 163 | NADH dehydrogenase I chain K |
| SEQ ID N° 164 | Hydrogenase subunits |
| SEQ ID N° 165 | NADH-ubiquinone oxidoreductase |
| SEQ ID N° 166 | Fe-S oxidoreductases |
| SEQ ID N° 167 | Formate hydrogenlyase, subunit 5 |
| SEQ ID N° 168 | Formate hydrogenlyase, subunit 5 |
| SEQ ID N° 169 | Ferredoxins |
| SEQ ID N° 170 | ATP-dependent proteases, serine protease domain |
| SEQ ID N° 171 | Thioredoxiri reductase |
| SEQ ID N° 172 | Pyridoxal phosphate-dependent aminotransferases |
| SEQ ID N° 173 | SAM-dependent methyltransferases |
| SEQ ID N° 174 | Fermentative lactate dehydrogenase and related dehydrogenases |
| SEQ ID N° 175 | Predicted Zn-dependent hydrolases (beta-lactmase superfamily) |
| SEQ ID N° 176 | RIBOSE-5-PHOSPHATE ISOMERASE (EC 5.3.1.6) |
| SEQ ID N° 177 | Predicted rRNA methylase |
| SEQ ID N° 178 | Histidinol-phosphate aminotransferase |
| SEQ ID N° 179 | Anaerobic dehydrogenases, typically selenocysteine-containing |
| SEQ ID N° 180 | NADPH-dependent glutamate synthase beta chain and related oxidoreductases |
| SEQ ID N° 181 | Predicted phosphate-utilizing enzyme involved in purine/histidine biosynthesis |
| SEQ ID N° 182 | Glutamine amidotransferase (possibly involved in histidine and purine biosynthesis) |
| SEQ ID N° 183 | Permeases, mainly amino acid |
| SEQ ID N° 184 | ATPase subunits of ABC transporters |
| SEQ ID N° 185 | GMP synthase - Glutamine amidotransferase domain |
| SEQ ID N° 186 | Predicted membrane GTPase |
| SEQ ID N° 187 | Glycine decarboxylase complex H subunit |
| SEQ ID N° 188 | PP-loop superfamily ATPase |
| SEQ ID N° 189 | (Predicted) ATPases involved in pili biogenesis |
| SEQ ID N° 190 | S1-type RNA-binding domain |
| SEQ ID N° 191 | 3-methyl-2-oxobutanoate hydroxymethyltransferase |
| SEQ ID N° 192 | Hydrogenase formation/expression factors |
| SEQ ID N ° 193 | Fe-S oxidoreductases 4 |
| SEQ ID N° 194 | Predicted dehydrogenases and related proteins |
| SEQ ID N° 195 | Predicted dehydrogenases and related proteins |
| SEQ ID N° 196 | Diaminopimelate/ornithine desuccinylases/deacetylases |
| SEQ ID N° 197 | Superfamily II DNA and RNA helicases |
| SEQ ID N° 198 | Superfamily II DNA and RNA helicases |
| SEQ ID N° 199 | Carbamate kinase |
| SEQ ID N° 200 | Flavoproteins, dehydrogenase subunits |
| SEQ ID N° 201 | SAM-dependent methyltransferases |
| SEQ ID N° 202 | Cystathionine beta-lyases/cystathionine gamma-synthases |
| SEQ ID N° 203 | 5-methyltetrahydrofolate-homocysteine methyltransferase (methionine synthetase) |
| SEQ ID N° 204 | Permeases (major facilitator family) |
| SEQ ID N° 205 | Homoserine dehydrogenase |
| SEQ ID N° 206 | Isoleucyl-tRNA synthetase |
| SEQ ID N° 207 | Helix-turn-helix domain-containing transcriptional regulators |
| SEQ ID N° 208 | Cation transport ATPases |
| SEQ ID N° 209 | Superfamily II DNA and RNA helicases |
| SEQ ID N° 210 | Oligopeptide/nickel permeases |
| SEQ ID N° 211 | ATPase subunits of ABC transporters |
| SEQ ID N° 212 | ATPase subunits of ABC transporters |
| SEQ ID N° 213 | ENDOGLUCANASE (EC 3.2.1.4) |
| SEQ ID N° 214 | (Predicted) ATPases involved in pili biogenesis |
| SEQ ID N° 215 | Ferredoxins |
| SEQ ID N° 216 | Flavodoxins/hemoproteins |
| SEQ ID N° 217 | Ferredoxins |
| SEQ ID N° 218 | Formate hydrogenlyase, subunit 5 |
| SEQ ID N° 219 | Recombinase (RecA) and ATPase domain of putative ATP-dependent protease |
| SEQ ID N° 220 | Nucleoside-diphosphate-sugar pyrophosphorylases involved in lipopolysaccharide biosynthesis/translation initiation factor eIF2B subunits |
| SEQ ID N° 221 | Lysyl/asparaginyl/aspartyl-tRNA synthetases |
| SEQ ID N° 222 | Arylsulfatase regulator (Fe-S oxidoreductase) |
| SEQ ID N° 223 | 3-OCTAPRENYL-4-HYDROXYBENZOATE CARBOXY-LYASE or PHENYLACRYLIC ACID DECARBOXYLASE (EC 4.1.I.) |
| SEQ ID N° 224 | Diadenosine 5',5"'-P1,P4-tetraphosphate (Ap4A) asymmetrical hydrolase, other predicted hydrolasesof the HIT family and galactose-1-phosphate uridylyltransferase |
| SEQ ID N° 225 | Predicted permease |
| SEQ ID N° 226 | Ferrichrome transport proteins |
| SEQ ID N° 227 | Iron, hemin, cobalamine permeases |
| SEQ ID N° 228 | ATPase components of ABC transporters with duplicated ATPase domains |
| SEQ ID N° 229 | Phosphate permease |
| SEQ ID N° 230 | Phosphate permease |
| SEQ ID N° 231 | ABC transporter ATPase subunit |
| SEQ ID N° 232 | Zn-dependent hydrolases, including glyoxylases |
| SEQ ID N° 233 | HYPOTHETICAL THERMONUCLEASE(EC 3.1.31.1) |
| SEQ ID N° 234 | PHP superfamily hydrolase |
| SEQ ID N° 235 | Predicted membrane GTPase |
| SEQ ID N° 236 | Ferredoxin oxidoreductases |
| SEQ ID N° 237 | SAM-dependent methyltransferases |
| SEQ ID N° 238 | SAM-dependent methyltransferases |
| SEQ ID N° 239 | Predicted GTPases |
| SEQ ID N° 240 | ACETYLORNITHINE DEACETYLASE or SUCCINYL-DIAMINOPIMELATE DESUCCINYLASE (EC 3.5.1.16 or 3.5.1.18) |
| SEQ ID N° 241 | Permeases |
| SEQ ID N° 242 | Predicted Zn-dependent hydrolases (beta-lactmase superfamily) |
| SEQ ID N° 243 | Phosphoribosylformimino-5-aminoimidazole carboxamide isomerase |
| SEQ ID N° 244 | Adenylate/cytidylate kinase |
| SEQ ID N° 245 | Queuine tRNA-ribosyltransferase |
| SEQ ID N° 246 | Superfamily II DNA and RNA helicases |
| SEQ ID N° 247 | Asparagine synthase (glutamine-hydrolyzing) |
| SEQ ID N° 248 | Helix-turn-helix domain-containing transcriptional regulators |
| SEQ ID N° 249 | Permeases |
| SEQ ID N° 250 | TRANSLATION INITIATION FACTOR IF-2 HOMOLOG |
| SEQ ID N° 251 | Thymidylate kinase |
| SEQ ID N° 252 | Predicted Zn-dependent proteases (possible chaperone function) |
| SEQ ID N° 253 | Uncharacterized ACR (translation initiation regulator?) |
| SEQ ID N° 254 | SAM-dependent methyltransferases |
| SEQ ID N° 255 | Permeases |
| SEQ ID N° 256 | PROLYL ENDOPEPTIDASE (EC 3.4.21.26) |
| SEQ ID N° 257 | Adenylate/cytidylate kinase |
| SEQ ID N° 258 | Deacetylase |
| SEQ ID N° 259 | Predicted transporters! |
| SEQ ID N° 260 | UDP-N-acetyl-D-mannosaminùronic acid dehydrogenase/UDP-glucose. 6-dehydrogenase |
| SEQ ID N° 261 | Nucleoside-diphosphate-sugar pyrophosphorylases involved in lipopolysaccharide biosynthesis/translation initiation factor eIF2B subunits |
| SEQ ID N° 262 | Glycosyltransferases, typically involved in cell wall biogenesis |
| SEQ ID N° 263 | Carbonic anhydrases homologous to acetyltransferases of the isoleucine patch superfamily |
| SEQ ID N° 264 | Predicted pyridoxal phosphate-dependent enzyme apparently involved in regulation of cell wall biogenesis |
| SEQ ID N° 265 | Predicted dehydrogenases and related proteins |
| SEQ ID N° 266 | UDP-N-acetyl-D-mannosaminuronic acid dehydrogenase/UDP-glucose 6-dehydrogenase |
| SEQ ID N° 267 | Adenosine-5'-phosphosulfate 3'-phosphotransferase (adenylylsulfate kinase) |
| SEQ ID N° 268 | Predicted permeases |
| SEQ ID N° 269 | Predicted transporters! |
| SEQ ID N° 270 | Nucleoside-diphosphate-sugar pyrophosphorylases involved in lipopolysaccharide biosynthesis/translation initiation factor eIF2B subunits |
| SEQ ID N° 271 | dDTP-GLUCOSE 4,6-DEHYDRATASE(EC 4.2.1.46) |
| SEQ ID N° 272 | Nucleoside-diphosphate-sugar epimerases |
| SEQ ID N° 273 | HYPOTHETICAL GLYCOSYLTRANSFERASE (EC 2.4.1.-) |
| SEQ ID N° 274 | Glycosyltransferases, typically involved in cell wall biogenesis |
| SEQ ID N° 275 | (Predicted) ATPases involved in pili biogenesis |
| SEQ ID N° 276 | Uncharacterized ACR - SIR2 homolog!!!! |
| SEQ ID N° 277 | Hydrogenase subunits |
| SEQ ID N° 278 | Glutathione-dependent Na/H antiporters - transmembrane domain |
| SEQ ID N° 279 | Predicted ATPase |
| SEQ ID N° 280 | DNA repair exonuclease |
| SEQ ID N° 281 | ATPase involved in DNA repair |
| SEQ ID N° 282 | MANNOSE-6-PHOSPHATE ISOMERASE / MANNOSE-1-PHOSPHATE GUANYLYL TRANSFERASE (EC 5.3.1.8/2.7.7.22) |
| SEQ ID N° 283 | PHOSPHOMANNOMUTASE or PHOSPHOGLUCOMUTASE (EC 5.4.2.8 or 5.4.2.2) |
| SEQ ID N° 284 | High-affinity branched amino acid transport system ATPase |
| SEQ ID N° 285 | Uncharacterized ACR (translation initiation regulator?) |
| SEQ ID N° 286 | Integral membrane protein |
| SEQ ID N° 287 | HYPOTHETICAL PROTEIN WITH SIMILARITY TO GLYCOSYLTRANSFERASE |
| SEQ ID N° 288 | Uncharacterized ACR! 2 |
| SEQ ID N° 289 | Adenylosuccinate lyase |
| SEQ ID N° 290 | Predicted rRNA methylase |
| SEQ ID N° 291 | Dihydrodipicolinate synthetase/N-acetylneuraminate lyase |
| SEQ ID N° 292 | Predicted ATPases, distantly related to replication factors |
| SEQ ID N° 293 | GTPase involved in cell division, distantly related to tubulin |
| SEQ ID N° 294 | ATPases involved in chromosome partitioning |
| SEQ ID N° 295 | Succinyl-CoA synthetase, alpha subunit-related enzymes |
| SEQ ID N° 296 | Ferredoxin oxidoreductases |
| SEQ ID N° 297 | Hydrogenase formation factor |
| SEQ ID N° 298 | HYPOTHETICAL PROTEIN SIMILAR TO GLUCANASE |
| SEQ ID N° 299 | Permeases |
| SEQ ID N° 300 | HMP-P kinase (thiamine biosynthesis) |
| SEQ ID N° 301 | ACR non caractérisée |
| SEQ ID N° 302 | Séryl-tRNA synthétase |
| SEQ ID N° 303 | Perméases |
| SEQ ID N° 304 | Enzymes thiamine pyrophosphate-dépendante [acétolactate synthase, pyruvate déhydrogenase (cytochrome), 2-succinyl-6-hydroxy-2,4-cyclohexadiene-1-carboxylate synthase, glyoxylate carboligase] |
| SEQ ID N° 305 | Kétol-acid reductoisomérase (biosynthèse d'isoleucine) |
| SEQ ID N° 306 | 2-ISOPROPYLMALATE SYNTHASE (EC 4.1.3.12) |
| SEQ ID N° 307 | 3-isopropylmalate dehydratase/aconitase large subunit (domain) |
| SEQ ID N° 308 | 3-isopropylmalate déhydratase/aconitase petite sousunité (domaine) |
| SEQ ID N° 309 | Isopropylmalate/homocitrate synthases |
| SEQ ID N° 310 | Déhydratases (dihydroxy acid/phosphogluconate) |
| SEQ ID N° 311 | Acétyltransférases |
| SEQ ID N° 312 | 3-oxoacyl-[acyl-carrier-protein] synthase III |
| SEQ ID N° 313 | Acétyl-CoA acétyltransférases |
| SEQ ID N° 314 | Perméases |
| SEQ ID N° 315 | Predicted GTPases |
| SEQ ID N° 316 | N6-adénine-spécifique DNA methylase |
| SEQ ID N° 317 | Phosphate perméase |
| SEQ ID N° 318 | Cysteinyl-tRNA synthetase |
| SEQ ID N° 319 | NAD(FAD)-utilizing dehydrogenases |
| SEQ ID N° 320 | Uncharacterized ACR |
| SEQ ID N° 321 | Serine-pyruvate aminotransferase |
| SEQ ID N° 322 | Predicted cytidylyltransferases |
| SEQ ID N° 323 | ATPases involved in chromosome partitioning |
| SEQ ID N° 324 | Predicted metal-dependent hydrolases! |
| SEQ ID N° 325 | CBS domain proteins |
| SEQ ID N° 326 | Signal peptidase I |
| SEQ ID N° 327 | Lipopolysaccharide N-acetylglucosaminyltransferase |
| SEQ ID N° 328 | Fermentative lactate dehydrogenase and related dehydrogenases |
| SEQ ID N° 329 | Predicted permeases |
| SEQ ID N° 330 | Serine/Threonine protein kinases |
| SEQ ID N° 331 | Sensory transduction histidine kinases and response regulators (two-component signal transduction systems) |
| SEQ ID N° 332 | Chemotaxis protein |
| SEQ ID N° 333 | Sodium/calcium antiporter |
| SEQ ID N° 334 | Predicted Zn-dependent hydrolases (beta-lactmase superfamily) |
| SEQ ID N° 335 | Predicted glutamine amidotransferase |
| SEQ ID N° 336 | Serine/threonine protein kinases |
| SEQ ID N° 337 | HYPOTHETICAL LYSOPHOSPHOLIPASE HOMOLOG |
| SEQ ID N° 338 | Nucleoside-diphosphate-sugar pyrophosphorylases involved in lipopolysaccharide biosynthesis/translation initiation factor eIF2B subunits |
| SEQ ID N° 339 | (Predicted) hydrolases of the dehalogenase-related superfamily |
| SEQ ID N° 340 | RIBONUCLEOTIDE DIPHOSPHATE REDUCTASE (EC 1.17.4.1) |
| SEQ ID N° 341 | FAD-dependent oxidoreductases (ubiquinone biosynthesis) |
| SEQ ID N° 342 | Membrane Zn protease (prediction) |
| SEQ ID N° 343 | Predicted GTPases |
| SEQ ID N° 344 | Phosphoribosylcarboxyaminoimidazole (NCAIR) mutase |
| SEQ ID N° 345 | Phosphoribosylaminoimidazole (AIR) synthetase |
| SEQ ID N° 346 | Dehydrogenases with different specificities (related to short-chain alcohol dehydrogenases) |
| SEQ ID N° 347 | ATPases involved in chromosome partitioning |
| SEQ ID N° 348 | Predicted ATPases of the PP superfamily |
| SEQ ID N° 349 | Acetyltransferases |
| SEQ ID N° 350 | TRNA INTRON ENDONUCLEASE (EC 3.1.27.9) |
| SEQ ID N° 351 | Histidyl-tRNA synthetase |
| SEQ ID N° 352 | Preprotein translocase subunit |
| SEQ ID N° 353 | HYPOTHETICAL ENDONUCLEASE IV (EC 3.1.21.2) |
| SEQ ID N° 354 | INORGANIC PYROPHOSPHATASE (EC 31.6.1.1) |
| SEQ ID N° 355 | DNA-DEPENDENT RNA POLYMERASE, subunit E (EC 2.7.7.6) |
| SEQ ID N° 356 | Cation transporters |
| SEQ ID N° 357 | Putative transcription factors |
| SEQ ID N° 358 | Tryptophanyl-tRNA synthetase |
| SEQ ID N° 359 | Phosphorybosylpyrophosphate synthetase |
| SEQ ID N° 360 | Nucleoside-diphosphate-sugar pyrophosphorylases involved in lipopolysaccharide biosynthesis/translation initiation factor eIF2B subunits |
| SEQ ID N° 361 | Uncharacterized ACR |
| SEQ ID N° 362 | ENOLASE (EC 4.2.1.11) |
| SEQ ID N° 363 | DNA-DIRECTED POLYMERASE I (EC 2.7.7.7) |
| SEQ ID N° 364 | Fe-S oxidoreductases |
| SEQ ID N° 365 | Predicted dehydrogenases and related proteins |
| SEQ ID N° 366 | Predicted dehydrogenases and related proteins |
| SEQ ID N° 367 | Predicted sugar kinase |
| SEQ ID N° 368 | Dihydroorotase |
| SEQ ID N° 369 | Flavodoxins/hemoproteins |
| SEQ ID N° 370 | Molybdenum cofactor biosynthesis enzyme and related Fe-S oxidoreductases |
| SEQ ID N° 371 | 3'-phosphoadenosine 5'-phosphosulfate sulfotransferase (PAPS reductase) |
| SEQ ID N° 372 | Metal-dependent protease |
| SEQ ID N° 373 | Deoxycytidine deaminase |
| SEQ ID N° 374 | 50S ribosomal protein L1 |
| SEQ ID N° 375 | 30S ribosomal protein S2 |
| SEQ ID N° 376 | Superfamily II DNA and RNA helicases |
| SEQ ID N° 377 | Glycine dehydrogenase (decarboxylating) |
| SEQ ID N° 378 | Glycine dehydrogenase (decarboxylating) |
| SEQ ID N° 379 | Pseudouridine synthase |
| SEQ ID N° 380 | Integral membrane protein |
| SEQ ID N° 381 | Preprotein translocase subunit |
| SEQ ID N° 382 | Preprotein translocase subunit |
| SEQ ID N° 383 | NAD-dependent K+ or Na+ uptake system component |
| SEQ ID N° 384 | Pseudouridine synthase |
| SEQ ID N° 385 | ATP synthase E subunit |
| SEQ ID N° 386 | ATP synthase beta subunit/transription termination factor rho |
| SEQ ID N° 387 | Flavoproteins, dehydrogenase subunits |
| SEQ ID N° 388 | Sensory transduction histidine kinases and response regulators (two-component signal transduction systems) |
| SEQ ID N° 389 | Alanyl-tRNA synthetase |
| SEQ ID N° 390 | Oligopeptide/nickel permeases. |
| SEQ ID N° 391 | Permeases, primarily amino acid and peptide |
| SEQ ID N° 392 | ATPase subunits of ABC transporters |
| SEQ ID N° 393 | ATPase subunits of ABC transporters |
| SEQ ID N° 394 | Phosphoribosylforinylglycinamidine synthase I |
| SEQ ID N° 395 | Phosphoribosylformylglycinamidine synthase II |
| SEQ ID N° 396 | SAM-dependent methyltransferases |
| SEQ ID N° 397 | SAM-dependent methyltransferases |
| SEQ ID N° 398 | PHOSPHOSERINE PHOSPHATASE 1 (EC 3.1.3.3) |
| SEQ ID N° 399 | TRIOSEPHOSPHATE ISOMERASE (EC 5.3.1.1) |
| SEQ ID N° 400 | Flavodoxins/hemoproteins |
| SEQ ID N° 401 | NADPH-dependent glutamate synthase beta chain and related oxidoreductases |
| SEQ ID N° 402 | Fe-S oxidoreductases |
| SEQ ID N° 403 | D-ARABINO 3-HEXULOSE 6-PHOSPHATE FORMALDEHYDE LYASE (EC 4.1.2.) |
| SEQ ID N° 404 | Predicted phosphatase |
| SEQ ID N° 405 | Acetyltransferases |
| SEQ ID N° 406 | Helix-turn-helix domain-containing transcriptional regulators |
| SEQ ID N° 407 | Succinyl-CoA synthetase, alpha subunit-related enzymes |
| SEQ ID N° 408 | Uncharacterized ACR (repair enzyme?) |
| SEQ ID N° 409 | SAM-dependent methyltransferases |
| SEQ ID N° 410 | Uncharacterized ACR |
| SEQ ID N° 411 | Pyridoxal-phosphate-dependent aminotransferases (2-amino-3-oxobutanoate synthase/8-amino-7-oxononanoate synthase) |
| SEQ ID N° 412 | Alanyl-tRNA synthetase |
| SEQ ID N° 413 | Glycosyltransferases, typically involved in cell wall biogenesis |
| SEQ ID N° 414 | Glutathione-dependent Na/H antiporters - transmembrane domain |
| SEQ ID N° 415 | IMP dehydrogenase/GMP reductase |
| SEQ ID N° 416 | Valyl-tRNA synthetase |
| SEQ ID N° 417 | Phosphoribosyl.glycinamide formyltransferase II |
| SEQ ID N° 418 | Phosphoribosylamine-glycine ligase |
| SEQ ID N° 419 | Predicted ATPases, distantly related to replication factors |
| SEQ ID N° 420 | Uncharacterized ACR |
| SEQ ID N° 421 | N6-adenine-specific DNA methylase |
| SEQ ID N° 422 | DnaJ-type Zn finger domain |
| SEQ ID N° 423 | Phospho-N-acetylmuramoyl-pentapeptide transferase/Undécaprenylphosphate alpha-N-acetylglucosaminyltransferase |
| SEQ ID N° 424 | Glutamine synthase |
| SEQ ID N° 425 | Predicted ATPases, distantly related to replication factors |
| SEQ ID N° 426 | UDP-GLUCOSE 4-EPIMERASE (EC 5.1.3.2) |
| SEQ ID N° 427 | Lipopolysaccharide N-acetylglucosaminyltransferase |
| SEQ ID N° 428 | Putative translation factor |
| SEQ ID N° 429 | Adenylosuccinate synthase |
| SEQ ID N° 430 | Molybdopterin biosynthesis enzymes |
| SEQ ID N° 431 | Translation initiation factor eIF-2B alpha subunit |
| SEQ ID N° 432 | Sensory transduction histidine kinases and response regulators (two-component signal transduction systems) |
| SEQ ID N° 433 | ATP-DEPENDENT PROTEASE LA (EC 3.4.21.53) |
| SEQ ID N° 434 | Glycosyltransferases, typically involved in cell wall biogenesis |
| SEQ ID N° 435 | (Predicted) hydrolases of the dehalogenase-related superfamily |
| SEQ ID N° 436 .. | Predicted nucleotidyltransferases |
| SEQ ID N° 437 | CARBOXYPEPTIDASE (EC 3.4.17.19) |
| SEQ ID N° 438 | Membrane protease subunits, stomatin/prohibitin homologs |
| SEQ ID N° 439 | Response regulators |
| SEQ ID N° 440 | PROTEIN-GLUTAMATE METHYLESTERASE (EC 3.1.1.61) |
| SEQ ID N° 441 | Chemotaxis protein histidine kinase and related kinases |
| SEQ ID N° 442 | Sensory transduction histidine kinases and response regulators (two-component signal transduction systems) |
| SEQ ID N° 443 | Periplasmic dipeptide(and nickel)-binding proteins |
| SEQ ID N° 444 | Oligopeptide/nickel permeases |
| SEQ ID N° 445 | Permeases, primarily amino acid and peptide |
| SEQ ID N° 446 | ATPase subunits of ABC transporters |
| SEQ ID N° 447 | ATPase subunits of ABC transporters |
| SEQ ID N° 448 | Glucosamine-fructose-6-phosphate aminotransferase |
| SEQ ID N° 449 | BETA-GALACTOSIDASE (EC 3.2.1.23) |
| SEQ ID N° 450 | Uncharacterized, His-rich protein |
| SEQ ID N° 451 | Predicted GTPase |
| SEQ ID N° 452 | Predicted exopolyphosphatase |
| SEQ ID N° 453 | S-adenosylhomocysteine hydrolase |
| SEQ ID N° 454 | Molecular chaperone, HSP90 family |
| SEQ ID N° 455 | Recombinase (RecA) and ATPase domain of putative ATP-dependent protease |
| SEQ ID N° 456 | (Predicted) ATPases involved in pili biogenesis |
| SEQ ID N° 457 | Anaerobic dehydrogenases, typically selenocysteine-containing |
| SEQ ID N° 458 | Ferredoxins |
| SEQ ID N° 459 | Hydrogenase subunits |
| SEQ ID N° 460 | Hydrogenase subunits |
| SEQ ID N° 461 | NADH-ubiquinone oxidoreductase |
| SEQ ID N° 462 | Formate hydrogenlyase, subunit 5 |
| SEQ ID N° 463 | Ferredoxins |
| SEQ ID N°464 | Fe-S oxidoreductases |
| SEQ ID N° 465 | Hydrogenase subunits |
| SEQ ID N° 466 | Lipopolysaccharide N-acetylglucosaminyltransferase |
| SEQ ID N° 467 | GLYCOSYLTRANSFERASE (EC 2.4.1.-) |
| SEQ ID N° 468 | Topoisomerase type IA |
| SEQ ID N° 469 | Succinyl-CoA synthetase, alpha subunit-related enzymes |
| SEQ ID N° 470 | Uncharacterized ACR |
| SEQ ID N° 471 | METHIONINE AMINOPEPTIDASE (EC 3.4.11.18) |
| SEQ ID N° 472 | HYPOTHETICAL PROTEIN WITH SIMILARITY TO ENDONUCLEASE III |
| SEQ ID N° 473 | Molybdopterin biosynthesis enzyme |
| SEQ ID N° 474 | Predicted permeases |
| SEQ ID N° 475 | Predicted permeases |
| SEQ ID N° 476 | Alanyl-tRNA synthetase |
| SEQ ID N° 477 | Pyruvate kinase |
| SEQ ID N° 478 | Uncharacterized ACR |
| SEQ ID N° 479 | 50S ribosomal protein L16 |
| SEQ ID N° 480 | Thioredoxin-like oxidoreductases |
| SEQ ID N° 481 | Predicted amidohydrolase |
| SEQ ID N° 482 | 2-hydroxyhepta-2,4-diene-1,7-dioate isomerase |
| SEQ ID N° 483 | RNA-binding proteins (RRM domain)!! 1 |
| SEQ ID N° 484 | KLBA HOMOLOG |
| SEQ ID N° 485 | DNA-DEPENDENT RNA POLYMERASE, subunit M (EC 2.7.7.6) |
| SEQ ID N° 486 | PROLIFERATING CELL NUCLEAR ANTIGEN (PCNA) |
| SEQ ID N° 487 | Ferredoxin oxidoreductases |
| SEQ ID N° 488 | Ferredoxins |
| SEQ ID N° 489 | Ferredoxin oxidoreductases |
| SEQ ID N° 490 | Ferredoxin oxidoreductases |
| SEQ ID N° 491 | Ferredoxins |
| SEQ ID N° 492 | Ferredoxin oxidoreductases |
| SEQ ID N° 493 | Ferredoxin oxidoreductases |
| SEQ ID N° 494 | AAA superfamily and related ATPases |
| SEQ ID N° 495 | Nucleoside diphosphate kinase |
| SEQ ID N° 496 | Threonyl-tRNA synthetase |
| SEQ ID N° 497 | Transcription termination/antitermination factor |
| SEQ ID N° 498 | Aspartate carbamoyltransferase, catalytic chain |
| SEQ ID N° 499 | Uroporphyrin-III C-methyltransferase |
| SEQ ID N° 500 | Ornithine carbamoyltransferase |
| SEQ ID N° 501 | Orotidine-5'-phosphate decarboxylase |
| SEQ ID N° 502 | Predicted cytidylyltransferases |
| SEQ ID N° 503 | ALCOHOL DEHYDROGENASE HYPOTHETICAL BUTANOL DÉHYDROGENASE (EC 1.1.1.) |
| SEQ ID N° 504 | Na/H antiporter |
| SEQ ID N° 505 | Histidinol-phosphate aminotransferase |
| SEQ ID N° 506 | Amino acid permeases |
| SEQ ID N° 507 | Iron, hemin, cobalamine permeases |
| SEQ ID N° 508 | High-affinity branched amino acid transport system ATPase |
| SEQ ID N° 509 | SAM-dependent methyltransferases |
| SEQ ID N° 510 | Zn proteases and inactivated homologs |
| SEQ ID N° 511 | Zn proteases and inactivated homologs |
| SEQ ID N° 512 | Arsenical pump-driving ATPase |
| SEQ ID N° 513 | Hydrogenase expression-formation factor |
| SEQ ID N° 514 | Hydrogenase maturation factor |
| SEQ ID N° 515 | Superfamily I DNA helicases and helicase subunits (yjhR - C-terminal domain only) |
| SEQ ID N° 516 | (Predicted) hydrolases of the dehalogenase-related superfamily |
| SEQ ID N° 517 | HYPOTHETICAL GLUTAMATE DECARBOXYLASE (EC 4.1.1.) |
| SEQ ID N° 518 | DOLICHOL MANNOSYL TRANSFERASE RELATED PROTEIN |
| SEQ ID N° 519 | Asparagine synthase (glutamine-hydrolyzing) |
| SEQ ID N° 520 | Succinyl-CoA synthetase, alpha subunit-related enzymes |
| SEQ ID N° 521 | PROLINE PEPTIDASE (EC 3.4.-.-) |
| SEQ ID N° 522 | Glycine cleavage system T protein (aminomethyltransferase) |
| SEQ ID N° 523 | Transcription activator!!! |
| SEQ ID N° 524 | Transcription activator!!! |
| SEQ ID N° 525 | Thiamin-phosphate pyrophosphorylase |
| SEQ ID N° 526 | HMP-P kinase (thiamine biosynthesis) |
| SEQ ID N ° 527 | Phosphate permease |
| SEQ ID N° 528 | Purine nucleoside phosphorylase |
| SEQ ID N° 529 | Permeases |
| SEQ ID N° 530 | Uncharacterized ACR |
| SEQ ID N° 531 | PHOSPHOMANNOMUTASE or PHOSPHOGLUCOMUTASE (EC 5.4.2.8 or 5.4.2.2) |
| SEQ ID N° 532 | ATPase components of ABC transporters with duplicated ATPase domains |
| SEQ ID N° 533 | Thiol antioxidant enzymes |
| SEQ ID N° 534 | Aspartokinases |
| SEQ ID N° 535 | Aspartokinases |
| SEQ ID N° 536 | Homoserine kinase |
| SEQ ID N° 537 | Threonine dehydratase/Threonine synthase |
| SEQ ID N° 538 | Aspartate-semialdehyde dehydrogenase |
| SEQ ID N° 539 | 3-phosphoglycerate kinase |
| SEQ ID N° 540 | ABC transporter ATPase subunit |
| SEQ ID N° 541 | Pseudourydilate synthase I (tRNA psi55) |
| SEQ ID N° 542 | Molybdenum cofactor biosynthesis enzyme and related Fe-S oxidoreductases |
| SEQ ID N° 543 | ATPase subunits of ABC transporters |
| SEQ ID N° 544 | Ferredoxins |
| SEQ ID N° 545 | Biotin carboxylase/D-alanine-D-alanine ligase (ATP-grasp domain) |
| SEQ ID N° 546 | Superfamily II DNA and RNA helicases |
| SEQ ID N° 547 | Prolyl-tRNA synthetase |
| SEQ ID N° 548 | TATA BOX BINDING PROTEIN |
| SEQ ID N° 549 | Tyrosyl-tRNA synthetase |
| SEQ ID N° 550 | SAM-dependent methyltransferases |
| SEQ ID N° 551 | Flavodoxins/hemoproteins |
| SEQ ID N° 552 | NADPH-dependent glutamate synthase beta chain and related oxidoreductases |
| SEQ ID N° 553 | BETA-GLYCOSIDASE (EC 3.2.1.) |
| SEQ ID N° 554 | Predicted Zn-dependent hydrolases (beta-lactmase superfamily) |
| SEQ ID N° 555 | Predicted sugar kinases |
| SEQ ID N° 556 | Predicted Zn-dependent hydrolases (beta-lactmase superfamily) |
| SEQ ID N° 557 | SAM-dependent methyltransferases |
| SEQ ID N° 558 | Acetyl-CoA carboxylase beta subunit |
| SEQ ID N° 559 | Biotin carboxyl carrier protein of acetyl-CoA carboxylase |
| SEQ ID N° 560 | DIPEPTIDASE (EC 3.4.13.) |
| SEQ ID N° 561 | Leucyl-tRNA synthetase |
| SEQ ID N° 562 | Flavodoxins/hemoproteins |
| SEQ ID N° 563 | Ferredoxins |
| SEQ ID N° 564 | Methylviologen-reducing hydrogenase alpha subunit |
| SEQ ID N° 565 | AAA superfamily and related ATPases |
| SEQ ID N° 566 | Malic enzyme |
| SEQ ID N° 567 | ATPases involved in chromosome partitioning |
| SEQ ID N° 568 | Uncharacterized ACR |
| SEQ ID N° 569 | Serine-pyruvate aminotransferase |
| SEQ ID N° 570 | Predicted transposases |
| SEQ ID N° 571 | Histidinol-phosphate aminotransferase |
| SEQ ID N° 572 | Superfamily II DNA and RNA helicases (recQ family) |
| SEQ ID N° 573 | GTPase involved in cell division, distantly related to tubulin |
| SEQ ID N° 574 | Uracil phosphoribosyltransferase |
| SEQ ID N° 575 | Permeases (major facilitator family) |
| SEQ ID N° 576 | D-amino acid dehydrogenase |
| SEQ ID N° 577 | ATPase subunits of ABC transporters |
| SEQ ID N° 578 | Sodium- and chloride-dependent transporters |
| SEQ ID N° 579 | Translation elongation factor P/translation initiation factor eIF5-a |
| SEQ ID N° 580 | ABC transporter ATPase subunit |
| SEQ ID N° 581 | Uncharacterized ACR (pps1 protein homolog) |
| SEQ ID N° 582 | Thioredoxin-like oxidoreductases |
| SEQ ID N° 583 | PEPTIDYL-PROLYL CIS-TRANS ISOMERASE (EC 5.2.1.8) |
| SEQ ID N° 584 | PROTEASOME (Multicatalytic endopeptidase complex), beta subunit 2 (EC 3.4.99.46) |
| SEQ ID N° 585 | Predicted Zn-dependent hydrolases (beta-lactmase superfamily) |
| SEQ ID N° 586 | Threonine dehydratase/Threonine synthase |
| SEQ ID N° 587 | Glutamate 5-kinase |
| SEQ ID N° 588 | Periplasmic dipeptide(and nickel)-binding proteins |
| SEQ ID N° 589 | Oligopeptide/nickel permeases |
| SEQ ID N° 590 | Permeases, primarily amino acid and peptide |
| SEQ ID N° 591 | ATPase subunits of ABC transporters |
| SEQ ID N° 592 | ATPase subunits of ABC transporters |
| SEQ ID N° 593 | HYPOTHETICAL NUCLEASE SIMILAR TO DNA STRUCTURE-SPECIFIC ENDONUCLEASE |
| SEQ ID N 594 | NADH dehydrogenase I chain K |
| SEQ ID N° 595 | Hydrogenase subunits |
| SEQ ID N° 596 | Fe-S oxidoreductases |
| SEQ ID N° 597 | Formate hydrogenlyase, subunit 5 |
| SEQ ID N° 598 | Formate hydrogenlyase, subunit 5 |
| SEQ ID N° 599 | NADH-ubiquinone oxidoreductase |
| SEQ ID N° 600 | Ferredoxins |
| SEQ ID N° 601 | 3'-phosphoadenosine 5'-phosphosulfate sulfotransferase (PAPS reductase) |
| SEQ ID N° 602 | Flavoprotein involved in panthothenate metabolism |
| SEQ ID N° 603 | MOLYBDENUM COFACTOR BIOSYNTHESIS HOMOLOG |
| SEQ ID N° 604 | L asparaginase II |
| SEQ ID N° 605 | Glu-tRNAGln amidotransferase B subunit (PET112 homolog) |
| SEQ ID N° 606 | Glycerol dehydrogenase |
| SEQ ID N° 607 | TRANSCRIPTION INITIATION FACTOR TFIIB |
| SEQ ID N° 608 | Lipoate-protein ligase A |
| SEQ ID N° 609 | Uncharacterized protein involved in glycerol metabolism (homolog of Drosophila rhomboid) |
| SEQ ID N° 610 | Pyridoxal phosphate-dependent aminotransferases |
| SEQ ID N° 611 | High-affinity branched amino acid transport system ATPase |
| SEQ ID N° 612 | Predicted intégral membrane protein |
| SEQ ID N° 613 | Uncharacterized ACR |
| SEQ ID N° 614 | ATPase subunits of ABC transporters |
| SEQ ID N° 615 | Thiamine biosynthesis enzyme |
| SEQ ID N° 616 | NAD(FAD)-utilizing dehydrogenases |
| SEQ ID N° 617 | Membrane protease subunits, stomatin/prohibitin homologs |
| SEQ ID N° 618 | Dihydroorotate dehydrogenase |
| SEQ ID N° 619 | Succinyl-CoA synthetase, alpha subunit-related enzymes |
| SEQ ID N° 620 | RNA phosphate cyclase |
| SEQ ID N° 621 | Predicted phosphoesterase |
| SEQ ID N° 622 | (Predicted) ATPases involved in pili biogenesis |
| SEQ ID N° 623 | Predicted rRNA methylase |
| SEQ ID N° 624 | Zn proteases and inactivated homologs |
| SEQ ID N° 625 | Zn proteases and inactivated homologs |
| SEQ ID N° 626 | PROTEASE IV (EC 3.4.-.-) |
| SEQ ID N° 627 | Predicted permease |
| SEQ ID N° 628 | NTP pyrophosphohydrolases (MutT family) including oxidative damage repair enzymes |
| SEQ ID N° 629 | Tryptophan synthase beta chain |
| SEQ ID N° 630 | Glycosyltransferases, typically involved in cell wall biogenesis |
| SEQ ID N° 631 | Predicted Zn-dependent proteases (possible chaperone function) |
| SEQ ID N° 632 | DOLICHOL PHOSPHATE MANNOSE SYNTHASE RELATED PROTEIN |
| SEQ ID N° 633 | Thymidine phosphorylase |
| SEQ ID N° 634 | ATPases involved in chromosome partitioning |
| SEQ ID N° 635 | Predicted Zn-dependent hydrolases (beta-lactmase superfamily) |
| SEQ ID N° 636 | Predicted ATPases of the PP superfamily |
| SEQ ID N° 637 | Molybdenum cofactor biosynthesis enzyme and related Fe-S oxidoreductases |
| SEQ ID N° 638 | Diadenosine tetraphosphatase and related serine/threonine protein phosphatases |
| SEQ ID N° 639 | DNA LIGASE (EC 6.5.1.1) |
| SEQ ID N° 640 | Permeases (chloride channel?) |
| SEQ ID N° 641 | Molybdopterin biosynthesis enzyme |
| SEQ ID N° 642 | Glycine hydroxymethyltransferase |
| SEQ ID N° 643 | (Predicted) hydrolases of the dehalogenase-related superfamily |
| SEQ ID N° 644 | Signal recognition particle GTPase |
| SEQ ID N° 645 | Predicted GTPase |
| SEQ ID N° 646 | Predicted ATPases of the PP superfamily |
| SEQ ID N° 647 | Adenine/guanine phosphoribosyltransferases |
| SEQ ID N° 648 | Fe-S oxidoreductases 4 |
| SEQ ID N° 649 | GTPases - translation elongation factors + sulfate adenylate transferase subunit 1 |
| SEQ ID N° 650 | INDOLE-3-GLYCEROL PHOSPHATE SYNTHASE (EC 4.1.1.48) |
| SEQ ID N° 651 | Anthranilate phosphoribosyltransferase |
| SEQ ID N° 652 | Anthranilate/paraaminobenzoate synthase component I/Isochorismate synthase |
| SEQ ID N° 653 | Anthranilate synthase/paraaminobenzoate synthases |
| SEQ ID N° 654 | Anthranilate isomerase |
| SEQ ID N° 655 | Tryptophan synthase beta chain |
| SEQ ID N° 656 | Tryptophan synthase alpha chain |
| SEQ ID N° 657 | CBS domain proteins |
| SEQ ID N° 658 | Sensory transduction histidine kinases and response regulators (two-component signal transduction systems) |
| SEQ ID N° 659 | High-affinity branched amino acid transport system ATPase |
| SEQ ID N° 660 | Predicted integral membrane protein |
| SEQ ID N° 661 | Molecular chaperone (heat shock protein) |
| SEQ ID N° 662 | Predicted transposases |
| SEQ ID N° 663 | Acetyltransferases including spermidine N1-acetyltransferase |
| SEQ ID N° 664 | AAA superfamily and related ATPases |
| SEQ ID N° 665 | SAM-dependent methyltransferases |
| SEQ ID N° 666 | HYPOTHETICAL PROTEIN SIMILAR TO ENDOGLUCANASE |
| SEQ ID N° 667 | Molybdenum cofactor biosynthesis enzyme |
| SEQ ID N° 668 | ATPases involved in chromosome partitioning |
| SEQ ID N° 669 | AAA superfamily and related ATPases |
| SEQ ID N° 670 | ATPases involved in DNA repair |
| SEQ ID N° 671 | Succinyl-CoA synthetase, alpha subunit-related enzymes |
| SEQ ID N° 672 | Succinyl-CoA synthetase, alpha subunit-related enzymes |
| SEQ ID N° 673 | dGTP triphosphohydrolases and other D superfamily phosphohydrolases |
| SEQ ID N° 674 | IMP dehydrogenase/GMP reductase |
| SEQ ID N° 675 | 50S ribosomal protein L3 |
| SEQ ID N° 676 | 50S ribosomal protein L4 |
| SEQ ID N° 677 | 50S ribosomal protein L2 |
| SEQ ID N° 678 | 30S ribosomal protein S19 |
| SEQ ID N° 679 | 30S ribosomal protein S3 |
| SEQ ID N° 680 | 30S ribosomal protein S17 |
| SEQ ID N° 681 | 50S ribosomal protein L24 |
| SEQ ID N° 682 | 50S ribosomal protein L5 |
| SEQ ID N° 683 | 30S ribosomal protein S8 |
| SEQ ID N° 684 | 50S ribosomal protein L18 |
| SEQ ID N° 685 | 30S ribosomal protein S5 |
| SEQ ID N° 686 | 50S ribosomal protein L15 |
| SEQ ID N° 687 | Preprotein translocase subunit |
| SEQ ID N° 688 | UDP-GLUCOSE 4-EPIMERASE (EC 5.1.3.2) |
| SEQ ID N° 689 | ATPase subunits of ABC transporters |
| SEQ ID N° 690 | High-affinity branched amino acid transport system ATPase |
| SEQ ID N° 691 | TYPE I SITE SPECIFIC ENDONUCLEASE, subunit M (EC 3.1.21.3) |
| SEQ ID N° 692 | TYPE I SITE SPECIFIC ENDONUCLEASE, subunit S (EC 3.1.21.3) |
| SEQ ID N° 693 | TYPE I SITE SPECIFIC ENDONUCLEASE, subunit R (EC 3.1.21.3) |
| SEQ ID N° 694 | Amino acid permeases |
| SEQ ID N° 695 | REPLICATION FACTOR A RELATED PROTEIN |
| SEQ ID N° 696 | Permeases |
| SEQ ID N° 697 | HYPOTHETICAL PROTEIN 2-ACETYL-1-ALKYLGLYCEROPHOSPHOCHOLINE ESTERASE (EC 3.1.1.) |
| SEQ ID N° 698 | Dehydrogenases with different specificities (related to short-chai alcohol dehydrogenases) |
| SEQ ID N° 699 | XAA-PRO DIPEPTIDASE (EC 3.4.13.9) |
| SEQ ID N° 700 | Recombinase (RecA) and ATPase domain of putative ATP-dependent protease |
| SEQ ID N° 701 | Pseudouridine synthase |
| SEQ ID N° 702 | CBS domain proteins |
| SEQ ID N° 703 | Membrane Zn protease (prediction) |
| SEQ ID N° 704 | (Predicted) hydrolases of the dehalogenase-related superfamily |
| SEQ ID N° 705 | PROTEASOME (Multicatalytic endopeptidase complex), beta subunit 1(EC 3.4.99.46) |
| SEQ ID N° 706 | Glutamine amidophosphoribosyltransferase and other predicted amidophosphoribosyltransferases |
| SEQ ID N° 707 | Lysyl/asparaginyl/aspartyl-tRNA synthetases |
| SEQ ID N° 708 | NAD-dependent potassium channels - NAD-binding domains |
| SEQ ID N° 709 | SAM-dependent methyltransferases |
| SEQ ID N° 710 | Spermidine synthase |
| SEQ ID N° 711 | Pyridoxal-dependent aminotransferases |
| SEQ ID N° 712 | Permeases, mainly amino acid |
| SEQ ID N° 713 | ATP-dependent Zn proteases |
| SEQ ID N° 714 | Predicted GTPases |
| SEQ ID N° 715 | NAD synthase |
| SEQ ID N° 716 | Thiol-disulfide isomerase and thioredoxins |
| SEQ ID N° 717 | Adenine-specific DNA methyltransferases |
| SEQ ID N° 718 | High-affinity branched amino acid transport system ATPase |
| SEQ ID N° 719 | Purine nucleoside phosphorylase |
| SEQ ID N° 720 | OPERON POLYMERASE Il SEQ ID N° 1 - CELL DIVISION CONTROL PROTEIN |
| SEQ ID N° 721 | DNA-DIRECTED POLYMERASE II, subunit 1 (EC 2.7.7.7) |
| SEQ ID N° 722 | OPERON POLYMERASE II SEQ ID N° 4 - CONSERVED HYPOTHETICAL PROTEIN |
| SEQ ID N° 723 | OPERON POLYMERASE II SEQ ID N° 5 - RECOMBINASE |
| SEQ ID N° 724 | SAM-dependent methyltransferases |
| SEQ ID N° 725 | Molybdenum cofactor biosynthesis enzyme and related Fe-S oxidoreductases |
| SEQ ID N° 726 | IMP dehydrogenase/GMP reductase |
| SEQ ID N° 727 | Predicted ATPase (PP family) |
| SEQ ID N° 728 | |
| SEQ ID N° 729 | L asparaginase II |
| SEQ ID N° 730 | Helix-turn-helix domain-containing transcriptional regulators |
| SEQ ID N° 731 | Diadenosine 5',5"' -P1,P4-tetraphosphate (Ap4A) asymmetrical hydrolase, other predicted hydrolasesof the HIT family and galactose-1-phosphate uridylyltransferase |
| SEQ ID N° 732 | Dinucleotide-utilizing enzymes involved in molibdopterin and thiamin biosynthesis |
| SEQ ID N° 733 | Threonine dehydratase/Threonine synthase |
| SEQ ID N° 734 | Pseudouridine synthase |
| SEQ ID N° 735 | DOLICHOL-PHOSPHATE MANNOSYLTRANSFERASE (EC 2.4.1.83) |
| SEQ ID N° 736 | Superfamily II DNA and RNA helicases (recQ family) |
| SEQ ID N° 737 | DNA-DEPENDENT RNA POLYMERASE, subunit L (EC 2.7.7.6) |
| SEQ ID N° 738 | Site-specific DNA methylase |
| SEQ ID N° 739 | Phosphoglyceromutase/phosphopentomutase |
| SEQ ID N° 740 | Cobalamin-5-phosphate synthase |
| SEQ ID N° 741 | Helix-turn-helix domain-containing transcriptional regulators |
| SEQ ID N° 742 | Predicted oxidoreductases (related to aryl-alcohol dehydrogenases) |
| SEQ ID N° 743 | Permeases, mainly amino acid |
| SEQ ID N° 744 | Permeases, mainly amino acid |
| SEQ ID N° 745 | ATPase subunits of ABC transporters |
| SEQ ID N° 746 | Thioredoxin-like oxidoreductases |
| SEQ ID N° 747 | Chaperonin (HSP60 family) |
| SEQ ID N° 748 | Fumarate/succinate/L-aspartate dehydrogenases |
| SEQ ID N° 749 | Quinolinate synthase |
| SEQ ID N° 750 | Nicotinate-nucleotide pyrophosphorylase |
| SEQ ID N° 751 | GTPase involved in cell division, distantly, related to tubulin |
| SEQ ID N° 752 | Transcription antiterminator |
| SEQ ID N° 753 | 50S ribosomal protein L11 |
| SEQ ID N° 754 | Permeases (major facilitator family) |
| SEQ ID N° 755 | Lysyl/asparaginyl/aspartyl-tRNA synthetases |
| SEQ ID N° 756 | Hydrogenase expression factor |
| SEQ ID N° 757 | Permeases, primarily amino acid and peptide |
| SEQ ID N° 758 | Methionyl-tRNA synthetase |
| SEQ ID N° 759 | Periplasmic phosphate-binding protein |
| SEQ ID N° 760 | ALKALINE PHOSPHATASE (EC 3.1.3.1) |
| SEQ ID N° 761 | CELL DIVISION CONTROL PROTEIN |
| SEQ ID N° 762 | Fermentative lactate dehydrogenase and related dehydrogenases |
| SEQ ID N° 763 | BETA-MANNOSIDASE (EC 3.2..1.25) |
| SEQ ID N° 764 | H(+)-TRANSPORTING ATP SYNTHASE, subunit A(EC 3.6.1.34) |
| SEQ ID N° 765 | Zn-dependent alcohol dehydrogenases and related dehydrogenases |
| SEQ ID N° 766 | Superfamily II DNA and RNA helicases |
| SEQ ID N° 767 | Pyridoxal phosphate-dependent aminotransferases |
| SEQ ID N° 768 | Geranylgeranyl pyrophosphate synthase |
| SEQ ID N° 769 | Predicted rRNA methylase |
| SEQ ID N° 770 | Predicted hydrolases (HAD superfamily) |
| SEQ ID N° 771 | 50S ribosomal protein L22 |
| SEQ ID N° 772 | Phosphoribosylaminoimidazolesuccinocarboxamide (SAICAR) synthase |
| SEQ ID N° 773 | Translation initiation factor eIF-2B alpha subunit |
| SEQ ID N° 774 | DNA-dependent DNA polymerase elongation subunit (family B) |
| SEQ ID N° 775 | GLYCEROL KINASE (EC 2.7.1.30) |
| SEQ ID N° 776 | DNA-DEPENDENT RNA POLYMERASE, subunit D (EC 2.7.7.6) |
| SEQ ID N° 777 | ALPHA-GLUCAN PHOSPHORYLASE (EC 2.4.1.1) |
| SEQ ID N° 778 | Hydrogenase subunits |
| SEQ ID N° 779 | GMP synthase - PP-ATPase domain |
| SEQ ID N° 780 | Superfamily II DNA and RNA helicases |
| SEQ ID N° 781 | Isopropylmalate dehydrogenase |
| SEQ ID N° 782 | Phenylalanyl-tRNA synthetase alpha subunit |
| SEQ ID N° 783 | Phenylanyl-tRNA synthetase beta subunit |
| SEQ ID N° 784 | Adenine/guanine phosphoribosyltransferases |
| SEQ ID N° 785 | Thiamin-phosphate pyrophosphorylase |
| SEQ ID N° 786 | Nucleoside-diphosphate-sugar pyrophosphorylases involved in lipopolysaccharide biosynthesis/translation initiation factor eIF2B subunits |
| SEQ ID N° 787 | 50S ribosomal protein L14 |
| SEQ ID N° 788 | Periplasmic serine proteases |
| SEQ ID N° 789 | Pyridoxal phosphate-dependent aminotransferases |
| SEQ ID N° 790 | Anaerobic dehydrogenases, typically selenocysteine-containing |
| SEQ ID N° 791 | Facteur d'initiation de la traduction eIF-2B sous-unité alpha |
| SEQ ID N° 792 | Hypothetical tRNAnucleotidyultransferase containing NTP transf 2 domain |
| SEQ ID N° 793 | RapA-like bacterial aspartate phosphatase domain containing protein |
| SEQ ID N° 794 | Hypothetical metallo-dependent hydrolase (beta-lactamase superfamily) |
| SEQ ID N° 795 | Hypothetical integral membrane protein containing DUF6 domain |
| SEQ ID N° 796 | Deoxyribose-phosphate aldolase domain containing protein |
| SEQ ID N° 797 | Hypothetical 3-isopropylmalate de hydratase |
| SEQID N° 798 | Hypothetical archaeosine tRNA-ribosyltransferase |
| SEQ ID N° 799 | AAA superfamily,and related ATPases |
| SEQ ID N° 800 | Hypothetical 3-isopropylmalate dehydratase. |

### TABLEAU 2 : Homologie des séquences peptidiques SEQ ID N° 2 à SEQ ID N° 800 avec une séquence disponible dans une banque de données dont la fonction également connue permet d'assigner à chacune de ces séquences SEQ ID N° 2 à SEQ ID N° 800 sa fonction la plus probable.

Les homologies ont été réalisées avec le logiciel BLASTP (Altschul et al. 1990) avec pour paramètres les paramètres par défaut. La séquence la plus homologue est décrite par son numéro d'accession (colonne numéro d'accession) dans la référence de la banque où elle est disponible (colonne banque) avec l'espèce animale à laquelle appartient cette séquence (colonne espèce). Le pourcentage d'homologie est évalué par le logiciel BLAST sur une séquence de longueur donnée.

**TABLEAU 2**

| **SEQ ID** | **HOMOLOGIE** | **NUMERO ACCESSION** | **BANQUE DE DONNEES** | **ESPECE ANIMALE** |
|---|---|---|---|---|
| SEQ ID N° 2 | 29.91 % sur 117 | SLL0499 | cyanobase | *Synechocystis sp. |
| SEQ ID N° 3 | 31.72 % sur 268 | Q57727 | Swissprot | Methanococcus jannaschii |
| SEQ ID N° 4 | 44.83 % sur 29 | P75849 | Swissprot | Escherichia coli |
| SEQ ID N° 5 | 71.43 % sur 21 | Q58365 | Swissprot | Methanococcus jannaschii |
| SEQ ID N° 6 | 76.25 % sur 80 | P54012 | Swissprot | Methanococcus jannaschii |
| SEQ ID N° 7 | 38.78 % sur 49 | Q58241 | Swissprot | Methanococcus jannaschii. |
| SEQ ID N° 8 | 30.88 % sur 68 | P28636 | Swissprot | Escherichia coli |
| SEQ ID N° 9 | 87 % sur 361 | 057784 | Swissprot | Pyrococcus horikoshii |
| SEQ ID N° 10 | 85 % sur 283 | AB009467 | Genbank | Pyrococcus horikoshii |
| SEQ ID N° 11 | 37.93 % sur 58 | Q57862 | Swissprot | Methanococcus jannaschii |
| SEQ ID N° 12 | 42.86 % sur 105 | P73534 | Swissprot | *Synechocystis sp. |
| SEQ ID N° 12 | 88 % sur 807 | AB009467-9 | Genbank | Pyrococcus horikoshii |
| SEQ ID N° 13 | 58.09 % sur 136 | Q57722 | Swissprot | Methanococcus jannaschii |
| SEQ ID N° 14 | 30.14 % sur 73 | P47696 | Swissprot | Mycoplasma genitalium |
| SEQ ID N° 15 | 48.94 % sur 47 | Q57984 | Swissprot | Methanococcus jannaschii |
| SEQ ID N° 16 | 62 % sur 707 | AB009468 | Genbank | Pyrococcus horikoshii |
| SEQ ID N° 17 | 83 % sur 480 | AB009468 | Genbank | Pyrococcus horikoshii |
| SEQ ID N° 18 | 95 % sur 300 | AB009468 | Genbank | Pyrococcus horikoshii |
| SEQ ID N° 19 | 42.31 % sur 26 | P44933 | Swissprot | Haemophilus influenzae |
| SEQ ID N° 20 | 31.87 % sur 182 | P18335 | Swissprot | Escherichia coli |
| SEQ ID N° 21 | 83 % sur 286 | AP000001 | Genbank | Pyrococcus horikoshii |
| SEQ ID N° 22 | 47.50 % sur 40 | P77671 | Swissprot | Escherichia coli |
| SEQ ID N° 23 | 25.86 % sur 116 | P23847 | Swissprot | Escherichia coli |
| SEQ ID N° 24 | 50.38 % sur 133 | P37316. | Swissprot | Escherichia coli |
| SEQ ID N° 25 | 47.22 % sur 216 | P37315 | Swissprot | Escherichia coli |
| SEQ ID N° 26 | 35.16 % sur 91 | P73450 | Swissprot | *Synechocystis sp. |
| SEQ ID N° 27 | 30.70 % sur 114 | P73450 | Swissprot | *Synechocystis sp. |
| SEQ ID N° 28 | 72 % sur 237 | AB009469 | Genbank | Pyrococcus horikoshii |
| SEQ ID N° 29 | 54.55 % sur 110 | MJ1186 | TIGR database | Methanococcus jannaschii |
| SEQ ID N° 30 | 36.96 % sur 92 | P44513 | Swissprot | Haemophilus influenzae |
| SEQ ID N° 31 | 38.10 % sur 126 | P73450 | Swissprot | *Synechocystis sp. |
| SEQ ID N° 32 | 36.84 % sur 76 | Q57952 | Swissprot | Methanococcus jannaschii |
| SEQ ID N° 33 | 43.44 % sur 122 | P30749 | Swissprot | Escherichia coli |
| SEQ ID N° 34 | 51.55 % sur 97 | MJ1359 | TIGR database | Methanecoccus jannaschii |
| SEQ ID N° 35 | 45.95 % sur 37 | MJ1359 | TIGR database | Methanococcus jannaschii |
| SEQ ID N° 36 | 83 % sur 186 | AB009471 | Genbank | Pyrococcus horikoshii |
| SEQ ID N° 37 | 71 % sur 659 | D88253 | Swissprot | Thermococcus litoralis |
| SEQ ID N° 38 | 36.96 % sur 92 | P02916 | Swissprot | Escherichia coli |
| SEQ ID N° 39 | 43.24 % sur 74 | P07622 | Swissprot | Escherichia coli |
| SEQ ID N° 40 | 73 % sur 849 | AF016588 | Genbank | Pyrococcus furiosus |
| SEQ ID N° 41 | 40.00 % sur 120 | P73450 | Swissprot | *Synechocystis sp. |
| SEQ ID N° 42 | 24.74 % sur 97 | Q58900 | Swissprot | Methanococcus jannaschii |
| SEQ ID N° 43 | 28.05 % sur 82 | P23910 | Swissprot | Escherichia coli |
| SEQ ID N° 44 | 63.33 % sur 120 | Q57657 | Swissprot | Methanococcus jannaschii |
| SEQ ID N° 45 | 32.93 % sur 82 | P24231 | Swissprot | Escherichia coli |
| SEQ ID N° 46 | 37.88 % sur 66 | P24231 | Swissprot | Escherichia coli |
| SEQ ID N° 48 | 61.54 % sur 130 | Q49593 | Swissprot | Methanococcus jannaschii |
| SEQ ID N° 49 | 44.85 % sur 165 | Q60377 | Swissprot | Methanococcus jannaschii |
| SEQ ID N° 50 | 57.81% sur 64 | Q59027 | Swissprot | Methanococcus jannaschii |
| SEQ ID N° 51 | 54.39 % sur 57 | Q57571 | Swissprot | Methanococcus jannaschii |
| SEQ ID N° 52 | 28.00 % sur 100 | P47285 | Swissprot | Mycoplasma genitalium |
| SEQ ID N° 53 | 50.00 % sur 38 | SLL1913 | cyanobase | *Synechocystis sp. |
| SEQ ID N° 54 | 94 % sur 735 | AB009528 | Genbank | Pyrococcus horikoshii |
| SEQ ID N° 55 | 32.00 % sur 75 | MJ0917 | TIGR database | Methanococcus jannaschii |
| SEQ ID N° 56 | 39.44 % sur 71 | P21693 | Swissprot | Escherichia coli |
| SEQ ID N° 57 | 33.70 % sur 92 | P76594 | Swissprot | Escherichia coli |
| SEQ ID N° 58 | 35.14 % sur 37 | P32662 | Swissprot | Escherichia coli |
| SEQ ID N° 59 | 46.99 % sur 83 | Q58040 | Swissprot | Methanococcus jannaschii |
| SEQ ID N° 60 | 34.55 % sur 55 | Q58069 | Swissprot | Methanococcus jannaschii |
| SEQ ID N° 62 | 39.47 % sur 38 | P32708 | Swissprot | Escherichia coli |
| SEQ ID N° 63 | 40.74 % sur 27 | P29011 | Swissprot | Escherichia coli |
| SEQ ID N° 64 | 41.03 % sur 39 | P47612 | Swissprot | Mycoplasma genitalium |
| SEQ ID N° 65 | 95 % sur 537 | AB009526 | Genbank | Pyrococcus horikoshii |
| SEQ ID N° 66 | 27.68 % sur 177 | Q58119 | Swissprot | Methanococcus jannaschii |
| SEQ ID N° 67 | 29.63 % sur 54 | P39345 | Swissprot | Escherichia coli |
| SEQ ID N° 68 | 29.05 % sur 210 | P23849 | Swissprot | Escherichia coli |
| SEQ ID N° 69 | 31.53 % sur 111 | P22730 | Swissprot | Escherichia coli |
| SEQ ID N° 70 | 43.10 % sur 58 | P06992 | Swissprot | Escherichia coli |
| SEQ ID N° 71 | 78 % sur 157 | AB009526 | Genbank | Pyrococcus horikoshii |
| SEQ ID N° 71 | 86 % sur 53 | 1BQ8 | Swissprot | Pyrococcus furiosusi |
| SEQ ID N° 72 | 61.11 % sur 54 | Q58546 | Swissprot | Methanococcus jannaschii |
| SEQ ID N° 73 | 35.14 % sur 111 | MJ1529 | TIGR database | Methanococcus jannaschii |
| SEQ ID N° 74 | 31.82 % sur 66 | P43828 | Swissprot | Haemophilus influenzae |
| SEQ ID N° 75 | 53 % sur 82 | ACYP-ARCFU | Swissprot | Escherichia coli |
| SEQ ID N° 75 | 78 % sur 135 | AB009468 | Genbank | Pyrococcus horikoshii |
| SEQ ID N° 76 | 78 % sur 292 | AB009527 | Genbank | Pyrococcus horikoshii |
| SEQ ID N° 77 | 48.98 % sur 98 | Q57598 | Swissprot | Methanococcus jannaschü |
| SEQ ID N° 78 | 40.38 % sur 52 | Q57836. | Swissprot | Methanococcus jannaschii |
| SEQ ID N° 79 | 48.51 % sur 101 | Q57926 | Swissprot | Methanococcus jannaschii |
| SEQ ID N° 80 | 45.65 % sur 92 | P49367 | Swissprot | Saccharomyces cerevisiae |
| SEQ ID N° 81 | 47.76 % sur 67 | P49367 | Swissprot | Saccharomyces cerevisiae |
| SEQ ID N° 82 | 50.00 % sur 98 | Q58130 | Swissprot | Methanococcus jannaschii |
| SEQ ID N° 83 | 38.64 % sur 44 | Q01.217 | Swissprot | Saccharomyces cerevisiae |
| SEQ ID N° 84 | 38.89 % sur 36 | Q60382 | Swissprot | Methanococcus jannaschii |
| SEQ ID N° 85 | 80 % sur 323 | AB009524 | Genbank | Pyrococcus horikoshii |
| SEQ ID N° 86 | 52.00 % sur 25 | P33570 | Swissprot | Escherichia coli |
| SEQ ID N° 87 | 50.54 % sur 93 | Q58092 | Swissprot | Methanococcus jannaschii |
| SEQ ID N° 88 | 57.82 % sur 147 | SLL0934 | cyanobase | *Synechocystis sp. |
| SEQ ID N° 89 | 45.35 % sur 86 | P08566 | Swissprot | Saccharomyces cerevisiae |
| SEQ ID N° 90 | 47.62 % sur 42 | P08566 | Swissprot | Saccharomyces cerevisiae |
| SEQ ID N° 91 | 42.86 % sur 35 | P08566 | Swissprot | Saccharomyces cerevisiae |
| SEQ ID N° 92 | 38.16 % sur 76 | P22731 | Swissprot | Escherichia coli |
| SEQ ID N° 93 | 31.37 % sur 51 | P47366 | Swissprot | Mycoplasma genitalium |
| SEQ ID N° 94 | 33.33 % sur 45 | P23200 | Swissprot | Escherichia coli |
| SEQ ID N° 95 | 37.21 % sur 43 | P08566 | Swissprot | Saccharomyces cerevisiae |
| SEQ ID N° 96 | 49.45 % sur 91 | Q58575 | Swissprot | Methanococcus jannaschii |
| SEQ ID N° 97 | 87 % sur 166 | AB009524 | Genbank | Pyrococcus horikoshii |
| SEQ ID N° 98 | 32.50 % sur 40 | Q58275 | Swissprot | Methanococcus jannaschii |
| SEQ ID N° 99 | 66.88 % sur 154 | MJ1206 | TIGR database | Methanococcus jannaschii |
| SEQ ID N° 100 | 34.44 % sur 90 | SLL1496 | cyanobase | *Synechocystis sp. |
| SEQ ID N° 101 | 60.00 % sur 40 | P47252 | Swissprot | Mycoplasma genitalium |
| SEQ ID N° 102 | 39.44 % sur 180 | P07019 | Swissprot | Escherichia coli |
| SEQ ID N° 103 | 45.24 % sur 42 | Q58075 | Swissprot | Methanococcus jannaschii |
| SEQ ID N° 104 | 49.06 % sur 53 | P04805 | Swissprot | Escherichia coli |
| SEQ ID N° 105 | 53.33 % sur 60 | Q57898 | Swissprot | Methanococcus jannaschii |
| SEQ ID N° 106 | 35.09 % sur 57 | P39333 | Swissprot | Escherichia coli |
| SEQ ID N° 107 | 28.74 % sur 87 | SLL1118 | cyanobase | *Synechocystis sp. |
| SEQ ID N° 108 | 59.18 % sur 49 | MJ1163 | TIGR-database | Methanococcus jannaschii |
| SEQ ID N° 109 | 45.76 % sur 59 | MJ0266 | TIGR database | Methanococcus jannaschii |
| SEQ ID N° 110 | 52.31 % sur 65 | MJ0536 | TIGR database | Methanococcus jannaschii |
| SEQ ID N° 111 | 31.88 % sur 69 | MJ0267 | TIGR database | Methanococcus jannaschii |
| SEQ ID N° 112 | 37.68 % sur 69 | MJ0266 | TIGR database | Methanococcus jannaschii |
| SEQ ID N° 113 | 70.71 % sur 99 | MJ0536 | TIGR database | Methanococcus jannaschii |
| SEQ ID N° 114 | 26.19 % sur 42 | Q57550 | Swissprot | Methanococcus jannaschii |
| SEQ ID N° 115 | 45.98 % sur 87 | P73450 | Swissprot | *Synechocystis sp. |
| SEQ ID N° 116 | 70 % sur 220 | AB009522 | Genbank | Pyrococcus horikoshii |
| SEQ ID N° 117 | 44.29 % sur 70 | P09171 | Swissprot | Escherichia coli |
| SEQ ID N° 118 | 41.3.0 % sur 46 | P39406 | Swissprot | Escherichia coli |
| SEQ ID N° 119 | 47.73 % sur 44 | P02369 | Swissprot | Escherichia coli |
| SEQ ID N° 120 | 40.38 % sur 52 | P47553 | Swissprot | Mycoplasma genitalium |
| SEQ ID N° 121 | 57.14 % sur 42 | P02366 | Swissprot | Escherichia coli |
| SEQ ID N° 122 | 57.14% sur 63 | P54023 | Swissprot | Methanococcus jannaschii |
| SEQ ID N° 123 | 42.86 % sur 28 | P02363 | Swissprot | Escherichia coli |
| SEQ ID N° 124 | 39.71 % sur 68 | P47647 | Swissprot | Mycoplasma genitalium |
| SEQ ID N° 125 | 34.29 % sur 70 | P02351 | Swissprot | Escherichia coli |
| SEQ ID N° 126 | 37.93 % sur 29 | P05792 | Swissprot | Escherichia coli |
| SEQ ID N° 127 | 39.53 % sur 86 | Q58487 | Swissprot | Methanococcus jannaschii |
| SEQ ID N° 128 | 36.36 % sur 55 | Q60352 | Swissprot | Methanococcus jannaschii |
| SEQ 1D N° 129 | 48.32 % sur 149 | P27829 | Swissprot | Escherichia coli |
| SEQ ID N° 130 | 65.06 % sur 166 | MJ1504 | TIGR database | Methanococcus jannaschii |
| SEQ ID N° 131 | 49.23 % sur 65 | P47493 | Swissprot | Mycoplasma genitalium |
| SEQ ID N° 132 | 79 % sur 917 | AP000006 | Genbank | Pyrococcus horikoshii |
| SEQ ID N° 133 | 32.81 % sur 64 | Q57809 | Swissprot | Methanococcus jannaschii |
| SEQ ID N° 134 | 23.91 % sur 138 | MJ1275 | TIGR database | Methanococcus jannaschii |
| SEQ ID N° 135 | 55.49 % sur 164 | P54386 | Swissprot | *Synechocystis sp. |
| SEQ ID N° 136 | 37.11 % sur 159 | P39206 | Swissprot | Escherichia coli |
| SEQ ID N° 137 | 53.16 % sur 190 | Q58767 | Swissprot | Methanococcus jattnaschü |
| SEQ ID N° 138 | 26.32 % sur 76 | P24189 | Swissprot | Escherichia coli |
| SEQ ID N° 139 | 37.63 % sur 93 | P21590 | Swissprot | Escherichia coli |
| SEQ ID N° 140 | 34.72 % sur 72 | P24193 | Swissprot | Escherichia coli |
| SEQ ID N° 141 | 30.00 % sur 50 | MJ1073 | TIGR database | Methanococcus jannaschü |
| SEQ ID N° 142 | 46.05 % sur 76 | Q57885 | Swissprot | Methanococcus jannaschü |
| SEQ ID N° 143 | 95% sur 260 | AP000006 | Genbank | Pyrococcus horikoshü |
| SEQ ID N° 144 | 37.84 % sur 37 | P05055 | Swissprot | Escherichia coli |
| SEQ ID N° 145 | 97 % sur 1117 | AP000006 | Genbank | Pyrococcus horikoshii |
| SEQ ID N° 146 | 97 % sur 907 | Swissprot | Genbank | Pyrococcus horikoshii |
| SEQ ID N° 147 | 96 % sur 397 | AP000006-255 | Genbank | Pyrococcus horikoshii |
| SEQ ID N° 148 | 40.00 % sur 50 | Q58447 | Swissprot | Methanococcus jannaschii |
| SEQ ID N° 149 | 76.72 % sur 116 | P54062 | Swissprot | Methanococcus jannaschii |
| SEQ ID N° 150 | 32.47 % sur 77 | P02359 | Swissprot | Escherichia coli |
| SEQ ID N° 151 | 29.55 % sur 44 | Q57261 | Swissprot | Escherichia coli |
| SEQ ID N° 152 | 47.17 % sur 53 | P37797 | Swissprot | Escherichia coli |
| SEQ ID N°153. | 27.27 % sur 44 | P31993 | Swissprot | Escherichia coli |
| SEQ ID N° 154 | 94% sur 351 | AP000006 | Genbank | Pyrococcus horikoshii |
| SEQ ID N° 156 | 90% sur 222 | AP000006 | Genbank | Pyrococcus horikoshii |
| SEQ ID N° 157 | 24.53 % sur 265 | P32159 | Swissprot | Escherichia coli |
| SEQ ID N° 158 | 41.18 % sur 34 | HP1393 | TIGR database | Helicobacter pylori |
| SEQ ID N° 159 | 97 % sur 428 | AP000006 | Genbank | Pyrococcus horikoshii |
| SEQ ID N° 160 | 38.46 % sur 52 | P02364 | Swissprot | Escherichia coli |
| SEQ ID N° 161 | 42.17 % sur 83 | P11875 | Swissprot | Escherichia coli |
| SEQ ID N° 162 | 40.00 % sur 35 | P05054 | Swissprot | Escherichia coli |
| SEQ ID N° 163 | 56.10 % sur 41 | MJ 1310 | TIGR database | Methanococcus jannaschii |
| SEQ ID N° 164 | 47.83 % sur 69 | MJ1309 | TIGR database | Methanococcus jannaschii |
| SEQ ID N° 165 | 42.86 % sur 35 | P16430 | Swissprot | Escherichia coli |
| SEQ ID N° 166 | 40.54 % sur 74 | MJ0516 | TIGR database | Methanococcus jannaschii |
| SEQ ID N° 167 | 35.90 % sur 78 | P16431 | Swissprot | Escherichia coli |
| SEQ ID N° 168 | 33.73 % sur 83 | P16431 | Swissprot | Escherichia coli |
| SEQ ID N° 169 | 34.15 % sur 41 | P16432 | Swissprot | Escherichia coli |
| SEQ ID N° 170 | 42.31 % sur 78 | P43864 | Swissprot | Haemophilus influenzae |
| SEQ ID N° 171 | 57.30 % sur 89 | P09625 | Swissprot | Escherichia coli |
| SEQ ID N° 172 | 48.86 % sur 88 | P18335 | Swissprot | Escherichia coli |
| SEQ ID N° 173 | 61.73 % sur 81 | Q58293 | Swissprot | Methanococcus jannaschii |
| SEQ ID N° 174 | 46.15 % sur 65 | P05459 | Swissprot | Escherichia coli |
| SEQ ID N° 175 | 40.85 % sur 71 | Q57749 | Swissprot | Methanococcus jannaschii |
| SEQ ID N° 176 | 90 % sur 229 | AP000006 | Genbank | Pyrococcus horikoshii |
| SEQ ID N° 177 | 48.53 % sur 68 | P36929 | Swissprot | Escherichia coli |
| SEQ ID N° 178 | 31.87 % sur 91 | Q58365 | Swissprot | Methanococcus jannaschii |
| SEQ ID N° 179 | 39.22 % sur 51 | P20099 | Swissprot | Escherichia coli |
| SEQ ID N° 181 | 74.92 % sur 319 | Q58090 | Swissprot . | Methanococcus jannaschii |
| SEQ ID N° 182 | 51.06 % sur 47 | Q59055 | Swissprot | Methanococcus jannaschii |
| SEQ ID N° 183 | 28.35 % sur 127 | P16701 | Swissprot | Escherichia coli |
| SEQ ID N° 184 | 43.33 % sur 120 | P73450 | Swissprot | *Synechocystis sp. |
| SEQ ID N° 185 | 58.06 % sur 62 | P44335 | Swissprot | Haemophilus influenzae |
| SEQ ID N° 186 | 34.85 %.sur 66 | Q57986 | Swissprot - | Methanococcus jannaschii |
| SEQ ID N° 187 | 49.17 % sur 120 | P23884 | Swissprot | Escherichia coli |
| SEQ ID N° 188 | 39.72 % sur 141 | Q58341 | Swissprot | Methanococcus jannaschii |
| SEQ ID N° 189 | 37.14 % sur 70 | Q58683 | Swissprot | Methanococcus jannaschü |
| SEQ ID N° 190 | 68.48 % sur 92 | Q57581 | Swissprot | Methanococcus jannaschii |
| SEQ ID N° 191 | 46.09 % sur 128 | P31057 | Swissprot | Escherichia coli |
| SEQ ID N° 192 | 29.90 % sur 97 | Q58048 | Swissprot | Methanococcus jannaschii |
| SEQ ID N° 193 | 65.42 % sur 107 | Q58708 | Swissprot | Methanococcus jannaschii |
| SEQ ID N° 194 | 28.33 % sur 120 | Q57999 | Swissprot | Methanococcus jannaschii |
| SEQ ID N° 195 | 38.33 % sur 60 | Q57999 | Swissprot | Methanococcus jannaschii |
| SEQ ID N° 196 | 36.84 % sur 95 | P23908 | Swissprot | Escherichia coli |
| SEQ ID N° 197 | 38.33 % sur 60 | Q58900 | Swissprot | Methanococcus jannaschii |
| SEQ ID N° 198 | 23.48 % sur 115 | P30015 | Swissprot | Escherichia coli |
| SEQ ID N° 199 | 50.69 % sur 144 | Q46807 | Swissprot | Escherichia coli |
| SEQ ID N° 201 | 40.00 % sur 55 | P27851 | Swissprot | Escherichia coli |
| SEQ ID N° 202 | 50.00 % sur 84 | P00935 | Swissprot | Escherichia coli |
| SEQ ID N° 203 | 35.56 % sur 45 | Q58868 | Swissprot | Methanococcus jannaschii |
| SEQ ID N° 204 | 38.24 % sur 102 | P07117 | Swissprot | Escherichia coli |
| SEQ ID N° 205 | 46.48 % sur 71 | MJ1602 | TIGR database | Methanococcus jannaschii |
| SEQ ID N° 206 | 42.86 % sur 84 | P47587 | Swissprot | Mycoplasma genitalium |
| SEQ ID N° 207 | 43.75 % sur 64 | P37309 | Swissprot | Escherichia coli |
| SEQ ID N° 208 | 40.49 % sur 326 | P37617 | Swissprot | Escherichia coli |
| SEQ ID N° 209 | 33.33 % sur 48 | P53819 | Swissprot | Saccharomyces cerevisiae |
| SEQ ID N° 210 | 38.64 % sur 88 | P37316 | Swissprot | Escherichia coli |
| SEQ ID N° 211 | 31.82 % sur 44 | P73450 | Swissprot | *Synechocystis sp. |
| SEQ ID N° 212 | 35.48 % sur 93 | P73450 | Swissprot | *Synechocystis sp. |
| SEQ ID N° 213 | 52 % sur 397 | AP000005 | Genbank | Pyrococcus horikoshii |
| SEQ ID N° 214 | 44.71 % sur 85 | Q58412 | Swissprot | Methanococcus jannaschii |
| SEQ ID N° 215 | 66.67 % sur 15 | SLL0031 | cyanobase | *Synechocystis sp. |
| SEQ ID N° 216 | 56.67 % sur 30 | MJ1446 | TIGR database | Methanococcus jannaschii |
| SEQ ID N° 217 | 42.42 % sur 33 | Q60340 | Swissprot | Methanococcus jannaschii |
| SEQ ID N° 218 | 40.98 % sur 61 | P16431 | Swissprot | Escherichia coli |
| SEQ ID N° 219 | 31.43 % sur 70 | P74391 | Swissprot | *Synechocystis sp. |
| SEQ ID N° 220 | 49.12 % sur 57 | MJ1101 | TIGR database | Methanococcus jannaschii |
| SEQ ID N° 221 | 40.00 % sur 120 | P17242 | Swissprot | Escherichia coli |
| SEQ ID N° 222 | 35.56 % sur 45 | Q58317 | Swissprot | Methanococcus jannaschii |
| SEQ ID N° 223 | 83 % sur 181 | AP000004 | Genbank | Pyrococcus horikoshii |
| SEQ ID N° 224 | 35.63 % sur 87 | P47378 | Swissprot | Mycoplasma genitalium |
| SEQ ID N° 225 | 35.09 % sur 114 | MJ1177 | TIGR database | Methanococcus jannaschii |
| SEQ ID N° 226 | 68.03 % sur 122 | Q57550 | Swissprot | Methanococcus jannaschii |
| SEQ ID N° 227 | 31.36 % sur 169 | P15030 | Swissprot | Escherichia coli |
| SEQ ID N° 228 | 48.00 % sur 50 | P37797 | Swissprot | Escherichia coli |
| SEQ ID N° 229 | 31.00 % sur 100 | P07653 | Swissprot | Escherichia coli |
| SEQ ID N° 230 | 44.44 % sur 117 | P07654 | Swissprot | Escherichia coli |
| SEQ.ID N° 231 | 29.59 % sur 98 | Q60350 | Swissprot | Methanococcus jannaschii |
| SEQ ID N° 232 | 52.17 % sur 23 | Q58298 | Swissprot | Methanococcus jannaschii |
| SEQ ID N° 233 | 78 % sur 176 | AP0000005 | Genbank | Pyrococcus horikoshii |
| SEQ ID N° 234 | 27.78% sur 36 | P47255 | Swissprot | Mycoplasma genitalium |
| SEQ ID N° 235 | 45.19 % sur 270 | Q57986 | Swissprot | Methanococcus jannaschii |
| SEQ ID N° 236 | 51.22 % sur 41 | Q57724 | Swissprot | Methanococcus jannaschii |
| SEQ ID N° 237 | 32.32 % sur 99 | P55135 | Swissprot | Escherichia coli |
| SEQ ID N° 2.38 | 33.33 % sur 63 | P27851 | Swissprot | Escherichia coli |
| SEQ ID N° 239 | 37.31 % sur 67 | P25519 | Swissprot | Escherichia coli |
| SEQ ID N° 240 | 92 % sur 354 | AP000005 | Genbank | Pyrococcus horikoshii |
| SEQ ID N° 241 | 26.17 % sur 107 | SLL1204 | cyanobase | *Synechocystis sp. |
| SEQ ID N° 242 | 45.38 % sur 130 | Q58897 | Swissprot | Methanococcus jannaschii |
| SEQ ID N° 243 | 36.36 % sur 33 | Q58927 | Swissprot | Methanococcus jannaschii |
| SEQ ID N° 244 | 45.35 % sur 86 | P05082 | Swissprot | Escherichia coli |
| SEQ ID N° 245 | 60.00 % sur 115 | Q57878 | Swissprot | Methanococcus jannaschü |
| SEQ ID N° 246 | 44.30 % sur 158 | P30015 | Swissprot | Escherichia coli |
| SEQ ID N° 247 | 29.47 % sur 95 | P22106 | Swissprot | Escherichia coli |
| SEQ ID N° 248 | 54.17 % sur 24 | MJ1325 | TIGR database | Methanococcus jannaschii |
| SEQ ID N° 249 | 24.30 % sur 107 | SLL1204 | cyanobase | *Synechocystis sp. |
| SEQ ID N° 250 | 76 % sur 1040 | AP000005 | Genbank | Pyrococcus horikoshii |
| SEQ ID N° 251 | 54.17 % sur 24 | Q57741 | Swissprot | Methanococcus jannaschii |
| SEQ ID N° 252 | 44.00 % sur 100 | P23894 | Swissprot | Escherichia coli |
| SEQ ID N° 253 | 73.33 % sur 30 | YLR143W | sgd | Saccharomyces cerevisiae |
| SEQ ID N° 254 | 46.43 % sur 28 | P23003 | Swissprot | Escherichia coli |
| SEQ ID N° 255 | 24.14 % sur 58 | P28246 | Swissprot | Escherichia coli |
| SEQ ID N° 256 | 84 % sur 612 | JC4084 | Swissprot | Pyrococcus furiosus |
| SEQ ID N° 257 | 59.14 % sur 93 | Q58071 | Swissprot | Methanococcus jannaschii |
| SEQ ID N° 258 | 54.98 % sur 231 | Q57955 | Swissprot | Methanococcus jannaschii |
| SEQ ID N° 259 | 37.66 % sur 77 | MJ1068 | TIGR database | Methanococcus jannaschii |
| SEQ ID N° 260 | 29.03 % sur 93 | P27829 | Swissprot | Escherichia coli |
| SEQ ID N° 261 | 30.51 % sur 59 | MJ1101 | TIGR database | Methanococcus jannaschii |
| SEQ ID N° 262 | 38.30 % sur 47 | P11290 | Swissprot | Escherichia coli |
| SEQ ID N° 263 | 52.08 % sur 48 | SLL1636 | cyanobase | *Synechocystis sp. |
| SEQ ID N° 264 | 40.14 % sur 142 | P27833 | Swissprot | Escherichia coli |
| SEQ ID N° 265 | 37.50 % sur 56 | P42599 | Swissprot | Escherichia coli |
| SEQ ID N° 266 | 43.04 % sur 79 | P27829 | Swissprot | Escherichia coli |
| SEQ ID N° 267 | 52.05 % sur 171 | SLR0676 | cyanobase | *Synechocystis sp. |
| SEQ ID N° 268 | 22.94 % sur 109 | SLR1262 | cyanobase | *Synechocystis sp. |
| SEQ ID N° 269 | 26.47 % sur 204 | MJ1068 | TIGR database | Methanococcus jannaschii |
| SEQ ID N° 270 | 47.37 % sur 57 | MJ1101 | TIGR database | Methanococcus jannaschii |
| SEQ ID N° 271 | 85 % sur 334 | AP000002 | Genbank | Pyrococcus horikoshii |
| SEQ ID N° 272 | 46.85 % sur 111 | SLL1395 | cyanobase | *Synechocystis sp. |
| SEQ ID N° 273 | 28 % sur 353 | AE001082 | Genbank | Archaeoglobus fulgidus |
| SEQ ID N° 274 | 41.82 % sur 55 | SLR1065 | cyanobase | *Synechocystis sp. |
| SEQ ID N° 275 | 38.37 % sur 86 | Q58412 | Swissprot | Methanococcus jannaschii |
| SEQ ID N° 277 | 53.49 % sur 43 | MJ1309 | TIGR database | Methanococcus jannaschii |
| SEQ ID N° 278 | 24.79 % sur 117 | P39830 | Swissprot | Escherichia coli |
| SEQ ID N° 279 | 43.27 % sur 245 | Q58824 | Swissprot | Methanococcus jannaschii |
| SEQ ID N° 280 | 36.36 % sur 88 | MJ1323 | TIGR database | Methanococcus jannaschii |
| SEQ ID N° 281 | 37.08 % sur 89 | MJ1322 | TIGR database | Methanococcus jannaschii |
| SEQ ID N° 282 | 92 % sur 464 | AP000004 | Genbank | Pyrococcus horikoshii |
| SEQ ID N° 283 | 92 % sur 455 | AP000004 | Genbank | Pyrococcus horikoshii |
| SEQ ID N° 284 | 29.21 % sur 89 | P22730 | Swissprot | Escherichia coli |
| SEQ ID N° 285 | 46.43 % sur 56 | P42631 | Swissprot | Escherichia coli |
| SEQ ID N° 286 | 28.24 % sur 85 | MJ1078 | TIGR database | Methanococcus jannaschii |
| SEQ ID N° 287 | 47 % sur 374 | AB009527 | Genbank | Pyrococcus horikoshii |
| SEQ ID N° 288 | 38.46 % sur 52 | SLL1306 | cyanobase | *Synechocystis sp. |
| SEQ ID N° 289 | 58.76 % sur 177 | Q58339 | Swissprot | Methanococcus jannaschii |
| SEQ ID N° 290 | 46.25 % sur 80 | P36929 | Swissprot | Escherichia coli |
| SEQ ID N° 291 | 39.06 % sur 64 | P05640 | Swissprot | Escherichia coli |
| SEQ ID N° 292 | 59.64 % sur 166 | SLR1416 | cyanobase | *Synechocystis sp. |
| SEQ ID N° 293 | 44.62 % sur 65 | P47466 | Swissprot | Mycoplasma genitalium |
| SEQ ID N° 294 | 21.78 % sur 101 | P47706 | Swissprot | Mycoplasma genitalium |
| SEQ ID N° 295 | 36.19 % sur 105 | P76594 | Swissprot | Escherichia coli |
| SEQ ID N° 296 | 35.80 % sur 81 | Q57724 | Swissprot | Methanococcus jannaschii |
| SEQ ID N° 297 | 46.32 % sur 95 | P24193 | Swissprot | Escherichia coli |
| SEQ ID N° 298 | 89 % sur 333 | AP000003 | Genbank | Pyrococcus horikoshii |
| SEQ ID N° 299 | 29.35 % sur 92 | P37021 | Swissprot | Escherichia coli |
| SEQ ID N° 300 | 43.33 % sur 30 | Q57688 | Swissprot | Methanococcus jannaschii |
| SEQ ID N° 301 | 40.74 % sur 54 | P32698 | Swissprot | Escherichia coli |
| SEQ ID N° 302 | 46.43 % sur 196 | P09156 | Swissprot | Escherichia coli |
| SEQ ID N° 303 | 39.71 % sur 68 | MJ1317 | TIGR database | Methanococcus jannaschii |
| SEQ ID N° 304 | 41.07 % sur 112 | P30146 | Swissprot | Escherichia coli |
| SEQ ID N° 305 | 44.87 % sur 78 | P05793 | Swissprot | Escherichia coli |
| SEQ ID N° 306 | 58 % sur 488 | AE000772 | Genbank | Aquifex aeolicus |
| SEQ ID N° 307 | 33.33 % sur 144 | P49367 | Swissprot | Saccharomyces cerevisiae |
| SEQ ID N° 308 | 43.82 % sur 89 | P49367 | Swissprot | Saccharomyces cerevisiae |
| SEQ ID N° 309 | 31.11 % sur 90 | MJ1231 | TIGR database | Methanococcus jannaschii |
| SEQ ID N° 310 | 37.21% sur 86 | P25530 | Swissprot | Escherichia coli |
| SEQ ID N° 311 | 20.00 % sur 90 | P43577 | Swissprot | Saccharomyces cerevisiae |
| SEQ ID N° 312 | 29.41 % sur 68 | P24249 | Swissprot | Escherichia coli |
| SEQ ID N° 313 | 46.73 % sur 107 | MJ1549 | TIGR database | Methanococcus jannaschü |
| SEQ ID N° 314 | 31.25 % sur 64 | P25744 | Swissprot | Escherichia coli |
| SEQ ID N° 315 | 40.58 % sur 69 | Q49435 | Swissprot | Mycoplasma genitalium |
| SEQ ID N° 316 | 46.84 % sur 79 | Q57880 | Swissprot | Methanococcus jannaschii |
| SEQ ID N° 317 | 35.56 % sur 45 | P37308 | Swissprot | Escherichia coli |
| SEQ ID N° 318 | 39.22 % sur 153 | P47495 | Swissprot | Mycoplasma genitalium |
| SEQ ID N° 319 | 34.38 % sur 64 | P08201 | Swissprot | Escherichia coli |
| SEQ ID N° 320 | 51.92 % sur 52 | Q58481 | Swissprot | Methanococcus jannaschii |
| SEQ ID N° 321 | 37.68 % sur 69 | MJ0959 | TIGR database | Methanococcus jannaschii |
| SEQ ID N° 322 | 38.18 % sur 55 | MJ1179 | TIGR database | Methanococcus jannaschii |
| SEQ ID N° 323 | 47.17 % sur 53 | P18197 | Swissprot | Escherichia coli |
| SEQ ID N° 324 | 33.06 % sur 124 | P28910 | Swissprot | Escherichia coli |
| SEQ ID N° 325 | 49.06 % sur 53 | Q59011 | Swissprot | Methanococcus jannaschii |
| SEQ ID N° 326 | 33.33 % sur 51 | P15367 | Swissprot | Saccharomyces cerevisiae |
| SEQ ID N° 327 | 33.33 % sur 99 | SLL0045 | cyanobase | *Synechocystis sp. |
| SEQ ID N° 328 | 44.44 % sur 27 | P05459 | Swissprot | Escherichia coli |
| SEQ ID N° 329 | 30.56 % sur 108 | Q57883 | Swissprot | Methanococcus jannaschii |
| SEQ ID N° 330 | 48.10 % sur 79 | MJ1073 | TIGR database | Methanococcus jannaschii |
| SEQ ID N° 331 | 39.77 % sur 176 | SLR1044 | cyanobase | *Synechocystis sp. |
| SEQ ID N° 332 | 38.46 % sur 104 | P07365 | Swissprot | Escherichia coli |
| SEQ ID N° 333 | 37.61% sur 109 | P45394 | Swissprot | Escherichia coli |
| SEQ ID N° 334 | 41.38 % sur 29 | P47385 | Swissprot, | Mycoplasma genitalium |
| SEQ ID N° 335 | 44.74 % sur 38 | MJ1515 | TIGR database | Methanococcus jannaschii |
| SEQ ID N° 336 | 50.00 % sur 70 | Q58530 | Swissprot | Methanococcus jannaschii |
| SEQ ID N° 337 | 36 % sur 245 | 028521 | Swissprot | Archaeoglobus fulgidus |
| SEQ ID N° 338 | 45.45 % sur 44 | MJ1101 | TIGR database | Methanococcus jannaschii |
| SEQ.ID N° 339 | 28.95 % sur 76 | P15302 | Swissprot | Escherichia coli |
| SEQ ID N° 340 | 66 % sur 1420 | U78098 | Swissprot | Pyrococcus furiosus |
| SEQ ID N° 341 | 37.50 % sur 56 | Q58915 | Swissprot | Methanococcus jannaschii |
| SEQ ID N° 342 | 42.86 % sur 70 | SLL0862 | cyanobase | *Synechocystis sp. |
| SEQ ID N° 343 | 59.46 % sur 37 | P47571 | Swissprot | Mycoplasma genitalium |
| SEQ ID N° 344 | 55.69 % sur 167 | P21264 | Swissprot | Saccharomyces cerevisiae |
| SEQ ID N° 345 | 47.19 % sur 89 | P07244 | Swissprot | Saccharomyces cerevisiae |
| SEQ ID N° 346 | 51.43 % sur 35 | P25529 | Swissprot | Escherichia coli |
| SEQ ID N° 347 | 42.22 % sur 45 | P53383 | Swissprot | *Synechocystis sp. |
| SEQ ID N° 348 | 43.06 % sur 72 | Q58558 | Swissprot | Methanococcus jannaschü |
| SEQ ID N° 349 | 44.12 % sur 68 | P09453 | Swissprot | Escherichia coli |
| SEQ ID N° 350 | 85 % sur 170 | AB009474 | Genbank | Pyrococcus horikoshii |
| SEQ ID N° 351 | 35.05 % sur 97 | P04804 | Swissprot | Escherichia coli |
| SEQ ID N° 352 | 36.90 % sur 252 | MJ1562 | TIGR database | Methanococcus jannaschü |
| SEQ ID N° 353 | 88 % sur 281 | AB009529 | Genbank | Pyrococcus horikoshii |
| SEQ ID N° 354 | 94 % sur 178 | AB009529 | Genbank | Pyrococcus horikoshü |
| SEQ ID N° 355 | 92 % sur 82 | AP000006-258 | Genbank | Pyrococcus horikoshii |
| SEQ ID N° 355 | 94 % sur 171 | 059571 | Swissprot | Pyrococcus horikoshii |
| SEQ ID N° 356 | 27.78 % sur 90 | P37310 | Swissprot | Escherichia coli |
| SEQ ID N° 357 | 43.24 % sur 37 | Q57951 | Swissprot | Methanococcus jannaschii |
| SEQ ID N° 358 | 36.77 % sur 155 | Q58810 | Swissprot | Methanococcus jannaschii |
| SEQ ID N° 359 | 38.24 % sur 102 | P47304 | Swissprot | Mycoplasma genitalium |
| SEQ ID N° 360 | 55.74 % sur 61 | MJ1101 | TIGR database | Methanococcus jannaschii |
| SEQ ID N° 361 | 40.28 % sur 72 | P27244 | Swissprot | Escherichia coli |
| SEQ ID N° 362 | 93 % sur 428 | AB009529 | Genbank | Pyrococcus horikoshii |
| SEQ ID N° 363 | 99 % sur 771 | Z54173 | Swissprot | Pyrococcus sp. |
| SEQ ID N° 364 | 54.48 % sur 134 | Q58277 | Swissprot | Methanococcus jannaschii |
| SEQ ID N° 365 | 34.62 % sur 78 | P46853 | Swissprot | Escherichia coli |
| SEQ ID N° 366 | 33.02 % sur 106 | P46853 | Swissprot | Escherichia coli |
| SEQ ID N° 367 | 47.01 % sur 117 | Q58981 | Swissprot | Methanococcus jannaschii |
| SEQ ID N° 368 | 28.36 % sur 67 | Q46806 | Swissprot | Escherichia coli |
| SEQ ID N° 369 | 51.02 % sur 98 | MJ1446 | TIGR database | Methanococcus jannaschü |
| SEQ ID N° 370 | 41.38 % sur 58 | Q58214 | Swissprot | Methanococcus jannaschü |
| SEQ ID N° 371 | 33.33 % sur 54 | Q60377 | Swissprot | Methanococcus jannaschii. |
| SEQ ID N° 372 | 61.70 % sur 94 | Q58530 | Swissprot | Methanococcus jannaschii |
| SEQ ID N° 373 | 35.56 % sur 45 | P28248 | Swissprot | Escherichia coli |
| SEQ ID N° 374 | 35.85 % sur 53 | P02384 | Swissprot | Escherichia coli |
| SEQ ID N° 375 | 50.00 % sur 28 | P32905 | Swissprot | Saccharomyces cerevisiae |
| SEQ ID N° 376 | 65.22 % sur 23 | P32892 | Swissprot . | Saccharomyces cerevisiae |
| SEQ ID N° 377 | 45.78 % sur 83 | P33195 | Swissprot | Escherichia coli |
| SEQ ID N° 378 | 38.16 % sur 152 | P33195 | Swissprot | Escherichia coli |
| SEQ ID N° 379 | 33.80 % sur 71 | Q57612 | Swissprot | Methanocbccus jannaschii |
| SEQ ID N° 380 | 27.96 % sur 93 | MJ1078 | TIGR database | Methanococcus jannaschii |
| SEQ ID N° 381 | 52.46 % sur 122 | MJ1253 | TIGR database | Methanococcus jannaschii |
| SEQ ID N° 382 | 48.05 % sur 154 | Q57575 | Swissprot | Methanococcus jannaschii |
| SEQ ID N° 383 | 34.31 % sur 102 | P23868 | Swissprot | Escherichia coli |
| SEQ ID N° 384 | 22.22 % sur 81 | P33322 | Swissprot | Saccharomyces cerevisiae |
| SEQ ID N° 385 | 43.48 % sur 69 | Q57674 | Swissprot | Methanococcus jannaschii |
| SEQ ID N° 386 | 35.44 % sur 79 | P00824 | Swissprot | Escherichia coli |
| SEQ ID N° 387 | 34.38 % sur 64 | Q58142 | Swissprot | Methanococcus jannaschii |
| SEQ ID N° 388 | 43.82 % sur 89 | SLR1044 | cyanobase | *Synechocystis sp. |
| SEQ ID N° 389 | 32.56 % sur 86 | Q57984 | Swissprot | Methanococcus jannaschii |
| SEQ ID N° 390 | 37.41 % sur 139 | P37316 | Swissprot | Escherichia coli |
| SEQ ID N° 391 | 36.26 % sur 91 | P37315 | Swissprot | Escherichia coli |
| SEQ ID N° 392 | 35.96 % sur 89 | P73450 | Swissprot | *Synechocystis sp. |
| SEQ ID N° 393 | 38.46 % sur 91 | P73450 | Swissprot | *Synechocystis sp. |
| SEQ ID N° 394 | 44.00 % sur 25 | P43847 | Swissprot | Haemophilus influenzae |
| SEQ ID N° 395 | 32.22 % sur 90 | P43847 | Swissprot | Haemophilus influenzae |
| SEQ ID N° 396 | 35.42 % sur 48 | P28637 | Swissprot | Escherichia coli |
| SEQ ID N° 397 | 60.53 % sur 76 | Q57636 | Swissprot | Methanococcus jannaschii |
| SEQ ID N° 398 | 74 % sur 205 | AB009528 | Genbank | Pyrococcus horikoshii |
| SEQ ID N° 399 | 95% sur 231 | AB009528 | Genbank | Pyrococcus horikoshii |
| SEQ ID N° 400 | 37.29 % sur 59 | MJ1446 | TIGR database | Methanococcus jannaschii |
| SEQ ID N° 401 | 32.97 % sur 185 | Q12680 | Swissprot | Saccharomyces cerevisiae |
| SEQ ID N° 402 | 35.94 % sur 64 | P45476 | Swissprot | Escherichia coli |
| SEQ ID N° 403 | 95 % sur 406 | AB009529 | Genbank | Pyrococcus horikoshii |
| SEQ ID N° 404 | 42.22 % sur 45 | P77475 | Swissprot | Escherichia coli |
| SEQ ID N° 405 | 38.05 % sur 113 | Q58604 | Swissprot | Methanococcus jannaschii |
| SEQ ID N° 406 | 47.06 % sur 51 | Q58948 | Swissprot | Methanococcus jannaschii |
| SEQ ID N° 407 | 39.05 % sur 105 | P76594 | Swissprot | Escherichia coli |
| SEQ ID N° 408 | 55.56 % sur 54 | P52061 | Swissprot | Escherichia coli |
| SEQ ID N° 409 | 58.33 % sur 84 | Q59043 | Swissprot | Methanococcus jannaschii |
| SEQ ID N° 410 | 43.33 % sur 30 | Q58452 | Swissprot | Methanococcus jannaschii |
| SEQ ID N° 411 | 38.04 % sur 92 | P12998 | Swissprot | Escherichia coli |
| SEQ ID N° 412 | 46.67 % sur 30 | P47534 | Swissprot | Mycoplasma genitalium |
| SEQ ID N° 413 | 38.78 % sur 49 | SLR2116 | cyanobase | *Synechocystis sp. |
| SEQ ID N° 414 | 41.79 % sur 67 | P44933 | Swissprot | Haemophilus influenzae |
| SEQ ID N° 415 | 68.14 % sur 113 | P44334 | Swissprot | Haemophilus influenzae |
| SEQ ID N° 416 | 36.22 % sur 127 | P47576 | Swissprot | Mycoplasma genitalium |
| SEQ ID N° 417 | 54.76 % sur 210 | P33221 | Swissprot | Escherichia coli |
| SEQ ID N° 418 | 34.78 % sur 92 | P07244 | Swissprot | Saccharomyces cerevisiae |
| SEQ ID N° 419 | 40.07 % sur 297 | SLR1416 | cyanobase | *Synechocystis sp. |
| SEQ ID N° 420 | 31.40 % sur 86 | SLR0510 | cyanobase | *Synechocystis sp. |
| SEQ ID N° 421 | 58.82 % sur 51 | Q58120 | Swissprot | Methanococcus jannaschii |
| SEQ ID N° 422 | 63.70 % sur 146 | MJ1198 | TIGR database | Methanococcus jannaschii |
| SEQ ID N° 423 | 53.33 % sur 45 | P15876 | Swissprot | Escherichia coli |
| SEQ ID N° 424 | 62.12 % sur 66 | P06711 | Swissprot | Escherichia coli |
| SEQ ID N° 425 | 43.81 % sur 210 | SLR1416 | cyanobase | *Synechocystis sp. |
| SEQ ID N° 426 | 82 % sur 314 | AP000002 | Genbank | Pyrococcus horikoshii |
| SEQ ID N° 427 | 35.09 % sur 114 | MJ1178 | TIGR database | Methanococcus jannaschii |
| SEQ ID N° 428 | 34.09 % sur 44 | P45847 | Swissprot | Escherichia coli |
| SEQ ID N° 429 | 47.67 % sur 86 | P12283 | Swissprot | Escherichia coli |
| SEQ ID N° 430 | 41.18 % sur 68 | SLR0427 | cyanobase | *Synechocystis sp. |
| SEQ ID N° 431 | 41.14 % sur 158 | Q57586 | Swissprot | Methanococcus jannaschii |
| SEQ ID N° 432 | 30.73 % sur 205 | SLR1044 | cyanobase | *Synechocystis sp. |
| SEQ ID N° 433 | 71 % sur 1128 | AP000002-147 | Genbank | Pyrococcus horikoshii |
| SEQ ID N° 434 | 37.50 % sur 48 | P11290 | Swissprot | Escherichia coli |
| SEQ ID N° 435 | 38.81 % sur 67 | P33999 | Swissprot | Escherichia coli |
| SEQ ID N° 436 | 62.24 % sur 98 | Q57961 | Swissprot | Methanococcus jannaschii |
| SEQ ID N° 437 | 92 % sur 498 | AP000002 | Genbank | Pyrococcus horikoshii |
| SEQ ID N° 438 | 31.18 % sur 93 | P25661 | Swissprot | Escherichia coli |
| SEQ ID N° 439 | 29.89 % sur 87 | P21712 | Swissprot | Escherichia coli |
| SEQ ID N ° 440 | 96 % sur 368 | AP000002 | Genbank | Pyrococcus horikoshii |
| SEQ ID N° 441 | 39.19 % sur 222 | SLL1296 | cyanobase | *Synechocystis sp. |
| SEQ ID N° 442 | 28.85 % sur 208 | SLR1044 | cyanobase | *Synechocystis sp. |
| SEQ ID N° 443 | 34.48 % sur 29 | P33590 | Swissprot | Escherichia coli |
| SEQ ID N° 444 | 42.05 % sur 88 | P37316 | Swissprot | Escherichia coli |
| SEQ ID N° 445 | 41.56 % sur 77 | P37315 | Swissprot | Escherichia coli |
| SEQ ID N° 446 | 31.82 % sur 44 | P73450 | Swissprot | *Synechocystis sp. |
| SEQ ID N° 447 | 39.02 % sur 82. | P73450 | Swissprot | *Synechocystis sp. |
| SEQ ID N° 448 | .38.24 % sur 68 | Q58815 | Swissprot | Methanococcus jannaschii |
| SEQ ID N° 449 | 70 % sur 784 | AP000002 | Genbank | Pyrococcus horikoshii |
| SEQ ID N° 450 | 33.78 % sur 74 | P35756 | Swissprot | Haemophilus influenzae |
| SEQ ID N° 451 | 63.41 % sur 41 | P47270 | Swissprot | Mycoplasma genitalium |
| SEQ ID N° 452 | 43.55 % sur 62 | Q58395 | Swissprot | Methanococcus jannaschii |
| SEQ ID N° 453 | 63.55 % sur 321 | Q58783 | Swissprot | Methanococcus jannaschii |
| SEQ ID N° 454 | 30.99 % sur 71 | P02829 | Swissprot | Saccharomyces cerevisiae |
| SEQ ID N° 455 | 43.91 % sur 230 | Q58309 | Swissprot | Methanococcus jannaschii |
| SEQ ID N° 456 | 44.23 % sur 52 | Q58191 | Swissprot | Methanococcus jannaschii |
| SEQ ID N° 457 | 34.09 % sur 44 | P20099 | Swissprot | Escherichia coli |
| SEQ ID N° 458 | 41.67 % sur 72 | P32175 | Swissprot | Escherichia coli |
| SEQ ID N° 459 | 37.78 % sur 45 | MJ1309 | TIGR database | Methanococcus jannaschii |
| SEQ ID N° 460 | 53.33 % sur 45 | MJ1309 | TIGR database | Methanococcus jannaschii |
| SEQ ID N° 461 | 33.33 % sur 99 | P16430 | Swissprot | Escherichia coli |
| SEQ ID N° 462 | 52.60 % sur 154 | P16431 | Swissprot | Escherichia coli |
| SEQ ID N° 463 | 32.91 % sur 158 | P16432 | Swissprot | Escherichia coli |
| SEQ ID N° 464 | 48.00 % sur 75 | MJ0516 | TIGR database | Methanococcus jannaschii |
| SEQ ID N° 465 | 40.51 % sur 79 | MJ1309 | TIGR database | Methanococcus jannaschii |
| SEQ ID N° 466 | 30.99 % sur 71 | MJ1059 | TIGR database | Methanococcus jannaschii |
| SEQ ID N° 467 | 53 % sur 385 | 029919 | Swissprot | Archaeoglobus fulgidus |
| SEQ ID N° 468 | 37.04 % sur 81 | P43012 | Swissprot | Haemophilus influenzae |
| SEQ ID N° 469 | 34.26 % sur 108 | P76594 | Swissprot | Escherichia coli |
| SEQ ID N° 470 | 31.88 % sur 69 | Q58337 | Swissprot | Methanococcus jannaschii |
| SEQ ID N° 471 | 91 % sur 295 | AP000003 | Genbank | Pyrococcus horikoshü |
| SEQ ID N° 472 | 68 % sur 131 | AP000003 | Genbank | Pyrococcus horikoshii |
| SEQ ID N° 473 | 34.83 % sur 290 | P12281 | Swissprot | Escherichia coli |
| SEQ ID N° 474 | 32.97 % sur 91 | Q58119 | Swissprot | Methanococcus jannaschii |
| SEQ ID N° 475 | 27.09 % sur 203 | Q58119 | Swissprot | Methanococcus jannaschii |
| SEQ ID N° 476 | 31.82 % sur 88 | Q57984 | Swissprot | Methanococcus jannaschii |
| SEQ ID N° 477 | 50.39 % sur 127 | P21599 | Swissprot | Escherichia coli |
| SEQ ID N° 478 | 34.57 % sur 81 | P24198 | Swissprot | Escherichia coli |
| SEQ ID N° 479 | 54.72 % sur 159 | Q57963 | Swissprot | Methanococcus jannaschii |
| SEQ ID N° 480 | 40.68 % sur 59 | MJ1645 | TIGR database | Methanococcus jannaschii |
| SEQ ID N° 481 | 33.33 % sur 39 | SLL1640 | cyanobase | *Synechocystis sp. |
| SEQ ID N° 482 | 59.38 % sur 96 | MJ1656 | TIGR database | Methanococcus jannaschii |
| SEQ ID N° 483 | 33.33 % sur 57 | P27476 | Swissprot | Saccharomyces cerevisiae |
| SEQ ID N° 484 | 77 % sur 595 | AP000003 | Genbank | Pyrococcus horikoshii |
| SEQ ID N° 485 | 94 % sur 110 | D1030698 | Genbank | Pyrococcus horikoshii |
| SEQ ID N° 486 | 92 % sur 249 | AP000003 | Genbank | Pyrococcus horikoshii |
| SEQ ID N° 487 | 72.73 % sur 99 | MJ0269 | TIGR database | Methanococcus jannaschii |
| SEQ ID N° 488 | 44.00 % sur 50 | Q57563 | Swissprot | Methanococcus jannaschii |
| SEQ ID N° 489 | 61.29 % sur 155 | MJ0267 | TIGR database | Methanococcus jannaschii |
| SEQ ID N° 490 | 62.05 % sur 166 | MJ0266 | TIGR database | Methanococcus jannaschii |
| SEQ ID N° 491 | 42.31 % sur 26 | P16432 | Swissprot | Escherichia coli |
| SEQ ID N° 492 | 72.38 % sur 210 | MJ0267 | TIGR database | Methanococcus jannaschii |
| SEQ ID N° 493 | 67.10 % sur 155 | MJ0266 | TIGR database | Methanococcus jannaschii |
| SEQ ID N° 494 | 72.63% sur 274 | Q58556 | Swissprot | Methanococcus jannaschii |
| SEQ ID N° 495 | 52.38 % sur 105 | P24233 | Swissprot | Escherichia coli |
| SEQ ID N° 496 | 35.94 % sur 64 | P47615 | Swissprot | Mycoplasma genitalium |
| SEQ ID N° 497 | 42.00 % sur 50 | Q58447 | Swissprot | Methanococcus jannaschii |
| SEQ ID N° 498 | 50.75 % sur 134 | P07259 | Swissprot | Saccharomyces cerevisiae |
| SEQ ID N° 499 | 33.33 % sur 48 | Q58181 | Swissprot | Methanococcus jannaschii |
| SEQ ID N° 500 | 48.23 % sur 141 | P04391 | Swissprot | Escherichia coli |
| SEQ ID N° 501 | 53.52 % sur 71 | Q57700 | Swissprot | Methanococcus jannaschii |
| SEQ ID N° 502 | 55.81 % sur 43 | HI1526 | TIGR database | Haemophilus influenzae |
| SEQ ID N° 503 | 84 % sur 375 | AP000003 | Genbank | Pyrococcus horikoshii |
| SEQ ID N° 504 | 41.35 % sur 104 | Q60362 | Swissprot | Methanococcus jannaschii |
| SEQ ID N° 505 | 34.52 % sur 84 | Q58365 | Swissprot | Methanococcus jannaschii |
| SEQ ID N° 506 | 40.65 % sur 123 | HP0942 | TIGR database | Helicobacter pylori |
| SEQ ID N° 507 | 38.61 % sur 101 | P15030 | Swissprot | Escherichia coli |
| SEQ ID N° 508 | 40.00 % sur 45 | P22730 | Swissprot | Escherichia coli |
| SEQ ID N° 509 | 48.21 % sur 56 | Q58293 | Swissprot | Methanococcus jannaschii |
| SEQ ID N° 510 | 33.33 % sur 72 | P24231 | Swissprot | Escherichia coli |
| SEQ ID N° 511 | 25.00 % sur 128 | P24231 | Swissprot | Escherichia coli |
| SEQ ID N° 512 | 46.88 % sur 64 | MJ1142 | TIGR database | Methanococcus jannaschii |
| SEQ ID N° 513 | 40.82 % sur 98 | P24192 | Swissprot | Escherichia coli |
| SEQ ID N° 514 | 60.42 % sur 96 | Q58123 | Swissprot | Methanococcus jannaschii |
| SEQ ID N° 515 | 37.78 % sur 45 | P47386 | Swissprot | Mycoplasma genitalium |
| SEQ ID N° 516 | 40.00 % sur 40 | MJ1437 | TIGR database | Methanococcus jannaschii |
| SEQ ID N° 517 | 89 % sur 381 | AP000004 | Genbank | Pyrococcus horikoshii |
| SEQ ID N° 518 | 40 % sur 233 | 026431 | Swissprot | **Methanobacterium ther. |
| SEQ ID N° 519 | 45.00 % sur 60 | P22106 | Swissprot | Escherichia coli . |
| SEQ ID N° 520 | 26.92 % sur 52 | P76594 | Swissprot | Escherichia coli |
| SEQ ID N° 521 | 83 % sur 351 | AP000005 | Genbank | Pyrococcus horikoshii |
| SEQ ID N° 522 | 40.79 % sur 76 | P27248 | Swissprot | Escherichia coli |
| SEQ ID N° 523 | 27.93 % sur 179 | P44659 | Swissprot | Haemophilus influenzae |
| SEQ ID N° 524 | 31.03 % sur 58 | P44659 | Swissprot | Haemophilus influenzae |
| SEQ ID N° 525 | 41.82 % sur 55 | P30137 | Swissprot | Escherichia coli |
| SEQ ID N° 526 | 36.47 % sur 85 | Q57688 | Swissprot | Methanococcus jannasc hii |
| SEQ ID N° 527 | 31.82 % sur 66 | P37308 | Swissprot | Escherichia coli |
| SEQ ID N° 528 | 54.43 % sur 79 | Q60367 | Swissprot | Methanococcus jannaschii |
| SEQ ID N° 529 | 45.45 % sur 99 | HP1175 | TIGR database | Helicobacter pylori |
| SEQ ID N° 530 | 35.48 % sur 62 | P39364 | Swissprot | Escherichia coli |
| SEQ ID N° 531 | 85 % sur 451 | AP000005 | Genbank | Pyrococcus horikoshii |
| SEQ ID N° 532 | 37.10 % sur 62 | P45535 | Swissprot | Escherichia coli |
| SEQ ID N° 533 | 43.40 % sur 53 | P26427 | Swissprot | Escherichia coli |
| SEQ ID N° 534 | 35.79 % sur 95 | P08660 | Swissprot | Escherichia coli |
| SEQ ID N° 535 | 42.31 % sur 78 | P08660 | Swissprot | Escherichia coli |
| SEQ ID N° 536 | 36.46 % sur 96 | P00547 | Swissprot | Escherichia coli |
| SEQ ID N° 537 | 75.48 % sur 155 | Q58860 | Swissprot | Methanococcus jannaschii |
| SEQ ID N° 538 | 51.76 % sur 85 | Q57658 | Swissprot | Methanococcus jannaschii |
| SEQ ID N° 539 | 39.39 % sur 99 | P11665 | Swissprot | Escherichia coli |
| SEQ ID N° 540 | 44.00 % sur 50 | Q60350 | Swissprot | Methanococcus jannaschii |
| SEQ ID N° 541 | 37.50 % sur 64 | P07649 | Swissprot | Escherichia coli |
| SEQ ID N° 542 | 25.00 % sur 76 | MJ1632 | TIGR database | Methanococcus jannaschii |
| SEQ ID N° 543 | 37.04 % sur 108 | P73450 | Swissprot | *Synechocystis sp. |
| SEQ ID N° 544 | 43.09 % sur 123 | Q58175 | Swissprot | Methanococcus jannaschii |
| SEQ ID N° 545 | 56.90 % sur 58 | P32528 | Swissprot | Saccharomyces cerevisiae |
| SEQ ID N° 546 | 38.89 % sur 36 | P30015 | Swissprot | Escherichia coli |
| SEQ ID N° 547 | 34.58 % sur 107 | P47525 | Swissprot | Mycoplasma genitalium |
| SEQ ID N° 548 | 97 % sur 191 | AP000004 | Genbank | Pyrococcus horikoshii |
| SEQ ID N° 549 | 60.78 % sur 51 | Q57834 | Swissprot | Methanococcus jannaschii |
| SEQ ID N° 550 | 66.13 % sur 124 | MJ1233 | TIGR database | Methanococcus jannaschii |
| SEQ ID N° 551 | 43.75 % sur 48 | MJ1446 | TIGR database | Methanococcus jannaschii |
| SEQ ID N° 552 | 39.67 % sur 121 | Q12680 | Swissprot | Saccharomyces cerevisiae |
| SEQ ID N° 553 | 77 % sur 506 | U60214 | Swissprot | Pyrococcus furiosus |
| SEQ ID N° 554 | 37.78 % sur 45 | P47385 | Swissprot | Mycoplasma genitalium |
| SEQ ID N° 555 | 37.31 % sur 134 | MJ0917 | TIGR database | Methanococcus jannaschii |
| SEQ ID N° 556 | 48.94 % sur 47 | Q57749 | Swissprot | Methanococcus jannaschii |
| SEQ ID N° 557 | 40.38 % sur 52 | P17993 | Swissprot | Escherichia coli |
| SEQ ID N° 558 | 40.00 % sur 75 | Q57417 | Swissprot | *Synechocystis sp. |
| SEQ ID N° 559 | 53.97 % sur 63 | MJ1231 | TIGR database | Methanococcus jannaschii |
| SEQ ID N° 560 | 76 % sur 354 | AP000004 | Genbank | Pyrococcus horikoshii |
| SEQ ID N° 561 | 31.52 % sur 92 | P47508 | Swissprot | Mycoplasma genitalium |
| SEQ ID N° 562 | 63.16 % sur 19 | MJ1446 | TIGR database | Methanococcus jannaschii |
| SEQ ID N° 563 | 50.00 % sur 34 | Q58136 | Swissprot | Methanococcus jannaschii |
| SEQ ID N° 564 | 27.91 % sur 86 | P37181 | Swissprot | Escherichia coli |
| SEQ ID N° 565 | 41.27 % sur 126 | Q58556 | Swissprot | Methanococcus jannaschii |
| SEQ ID N° 566 | 52.61 % sur 211 | P43837 | Swissprot | Haemophilus influenzae |
| SEQ ID N° 567 | 46.00 % sur 150 | P21590 | Swissprot | Escherichia coli |
| SEQ ID N° 568 | 62.70 % sur 185 | MJ1133 | TIGR database | Methanococcus jannaschii |
| SEQ ID N° 569 | 36.63 % sur 172 | MJ0959 | TIGR database | Methanococcus jannaschii |
| SEQ ID N° 570 | 54.05 % sur 37 | Q60328 | Swissprot | Methanococcus jannaschii |
| SEQ ID N° 571 | 28.89 % sur 90 | Q58365 | Swissprot | Methanococcus jannaschii |
| SEQ ID N° 572 | 32.95 % sur 88 | P73421 | Swissprot | *Synechocystis sp. |
| SEQ ID N° 573 | 29.41 % sur 68 | Q57816 | Swissprot | Methanococcus jannaschii |
| SEQ ID N° 574 | 51.67 % sur 60 | P47276 | Swissprot | Mycoplasma genitalium |
| SEQ ID N° 575 | 34.56 % sur 136 | P07117 | Swissprot | Escherichia coli |
| SEQ ID N° 576 | 26.00 % sur 100 | SLR0633 | cyanobase | *Synechocystis sp. |
| SEQ ID N° 577 | 43.55 % sur 62 | P73450 | Swissprot | *Synechocystis sp. |
| SEQ ID N° 578 | 50.00 % sur 68 | Q58715 | Swissprot | Methanococcus jannaschii |
| SEQ ID N° 579 | 54.00 % sur 100 | Q58625 | Swissprot | Methanococcus jannaschii |
| SEQ ID N° 580 | 53.54 % sur 99 | Q60350 | Swissprot | Methanococcus jannaschii |
| SEQ ID N° 581 | 40.98 % sur 61 | Q60349 | Swissprot | Methanococcus jannaschü |
| SEQ ID N° 582 | 51.19 % sur 168 | Q58218 | Swissprot | Methanococcus jannaschü |
| SEQ ID N° 583 | 84 % sur 254 | AP000006 | Genbank | Pyrococcus horikoshü |
| SEQ ID N° 584 | 84 % sur 207 | AP000006 | Genbank | Pyrococcus horikoshü |
| SEQ ID N° 585 | 28.57 % sur 35 | P47385 | Swissprot | Mycoplasma genitalium |
| SEQ ID N° 586 | 25.25 % sur 99 | P05792 | Swissprot | Escherichia coli |
| SEQ ID N° 587 | 38.33 % sur 60 | P73071 | Swissprot | *Synechocystis sp. |
| SEQ ID N° 588 | 29.31 % sur 58 | P23847 | Swissprot | Escherichia coli |
| SEQ ID N° 589 | 47.62 % sur 84 | P37316 | Swissprot | Escherichiacoli |
| SEQ ID N° 590 | 42.96 % sur 135 | P37315 | Swissprot | Escherichia coli |
| SEQ ID N° 591 | 34.02 % sur 97 | P73450 | Swissprot | *Synechocystis sp. |
| SEQ ID N° 592 | 34.83 % sur 89 | P73450 | Swissprot | *Synechocystis sp. |
| SEQ ID N° 593 | 91 % sur 343 | AP000006 | Genbank | Pyrococcus horikoshii |
| SEQ ID N° 594 | 36.36 % sur 44 | MJ1310 | TIGR database | Methanococcus jannaschii |
| SEQ ID N° 595 | 42.86 % sur 49 | MJ1309 | TIGR database | Methanococcus jannaschii |
| SEQ ID N° 596 | 58.70 % sur 92 | MJ0516 | TIGR database | Methanococcus jannaschii |
| SEQ ID N° 597 | 33..75 % sur 80 | P16431 | Swissprot | Escherichia coli |
| SEQ ID N° 598 | 43.93 % sur 107 | P16431 | Swissprot | Escherichia coli |
| SEQ ID N° 599 | 27.68 % sur 112 | P16430 | Swissprot | Escherichia coli |
| SEQ ID N° 600 | 40.00 % sur 50 | P16432 | Swissprot | Escherichia coli |
| SEQ ID N° 601 | 26.53 % sur 49 | P17854 | Swissprot | Escherichia coli |
| SEQ ID N° 602 | 52.38 % sur 84 | Q58323 | Swissprot | Methanococcus jannaschii |
| SEQ ID N° 603 | 94 % sur 432 | AP000006 | Genbank | Pyrococcus horikoshii |
| SEQ ID N° 604 | 35.00 % sur 60 | P00805 | Swissprot | Escherichia coli |
| SEQ ID N° 605 | 60.25 % sur 239 | Q60325 | Swissprot | Methanococcus jannaschii |
| SEQ ID N° 606 | 61.90 % sur 63 | Q58122 | Swissprot | Methanococcus jannaschii |
| SEQ ID N° 607 | 98 % sur 300 | AP000006 | Genbank | Pyrococcus horikoshii |
| SEQ ID N° 608 | 34.57 % sur 81 | P32099 | Swissprot | Escherichia coli |
| SEQ ID N° 610 | 38.79 % sur 165 | P18335 | Swissprot | Escherichia coli |
| SEQ ID N° 611 | 35.09% sur 114 | P22730 | Swissprot | Escherichia coli |
| SEQ ID N° 612 | 31.51 % sur 73 | SLL0760 | cyanobase | *Synechocystis sp. |
| SEQ ID N° 613 | 40.86 % sur 93 | P37002 | Swissprot | Escherichia coli |
| SEQ ID N° 614 | 46.67 % sur 60 | P73450 | Swissprot | *Synechocystis sp. |
| SEQ ID N° 615 | 53.01 %.sur 183 | P30136 | Swissprot | Escherichia coli |
| SEQ ID N° 616 | 35.00 % sur 80 | P08201 | Swissprot | Escherichia coli |
| SEQ ID N° 617 | 35.56 % sur 45 | P25661 | Swissprot | Escherichia coli |
| SEQ ID N° 618 | 47.76 % sur 67 | P25889 | Swissprot | Escherichia coli |
| SEQ ID N° 619 | 40.46 % sur 131 | P76594 | Swissprot | Escherichia coli |
| SEQ ID N° 620 | 51.72 % sur 174 | Q60335 | Swissprot | Methanococcus jannaschii |
| SEQ ID N° 621 | 46.59 % sur 88 | Q58346 | Swissprot | Methanococcus jannaschii |
| SEQ ID N° 622 | 44.16 % sur 77 | Q58412 | Swissprot | Methanococcus jannaschii |
| SEQ ID N° 623 | 48.48 % sur 33 | P36929 | Swissprot | Escherichia coli |
| SEQ ID N° 624 | 34.78 % sur 46 | P24231 | Swissprot | Escherichia coli |
| SEQ ID N° 625 | 31.76 % sur 85 | P24231 | Swissprot | Escherichia coli |
| SEQ ID N° 626 | 74 % sur 334 | AP000006 | Genbank | Pyrococcus horikoshii |
| SEQ ID N° 627 | 27.40 % sur 73 | HP0567 | TIGR database | Helicobacter pylori |
| SEQ ID N° 628 | 59.76 % sur 82 | MJ1149 | TIGR database | Methanococcus jannaschii |
| SEQ ID N° 629 | 44.19 % sur 86 | P00931 | Swissprot | Saccharomyces cerevisiae |
| SEQ ID N° 630 | 60.47 % sur 86 | Q58619 | Swissprot | Methanococcus jannaschii |
| SEQ ID N° 631 | 32.35 % sur 34 | MJ1682 | TIGR database | Methanococcus jannaschii |
| SEQ ID N° 632 | 68 % sur 215 | D1031651 | Genbank | Pyrococcus horikoshii |
| SEQ ID N° 633 | 29.55 % sur 88 | P07650 | Swissprot | Escherichia coli |
| SEQ ID N° 634 | 56.86 % sur 51 | P18197 | Swissprot | Escherichia coli |
| SEQ ID N° 635 | 41.18 % sur 51 | P16692 | Swissprot | Escherichia coli |
| SEQ ID N° 636 | 22.54 % sur 71 | Q58873 | Swissprot | Methanococcus jannaschii |
| SEQ ID N° 637 | 49.44 % sur 89 | Q57567 | Swissprot | Methanococcus jannaschii |
| SEQ ID N° 638 | 64.71 % sur 34 | Q58322 | Swissprot | Methanococcus jannaschii |
| SEQ ID N° 639 | 49 % sur 557 | AE001061 | Genbank | Archaeoglobus fulgidus |
| SEQ ID N° 640 | 38.10 % sur 84 | Q57753 | Swissprot | Methanococcus jannaschü |
| SEQ ID N° 641 | 35.10 % sur 151 | P12281 | Swissprot | Escherichia coli |
| SEQ ID N° 642 | 43.55 % sur 124 | P00477 | Swissprot | Escherichia coli |
| SEQ ID N° 643 | 34.92 % sur 63 | P23306 | Swissprot | Escherichia coli |
| SEQ ID N° 644 | 39.18 % sur 171 | P07019 | Swissprot | Escherichia coli |
| SEQ ID N° 645 | 51.11 % sur 45 | P47270 | Swissprot | Mycoplasma genitalium |
| SEQ ID N° 646 | 42.19 % sur 64 | Q57909 | Swissprot | Methanococcus jannaschii |
| SEQ ID N° 647 | 30.00 % sur 60 | P07672 | Swissprot | Escherichia coli |
| SEQ ID N° 648 | 51.56 % sur 64 | Q57705 | Swissprot | Methanococcus jannaschii |
| SEQ ID N° 649 | 41.67 % sur 36 | P23845 | Swissprot | Escherichia coli |
| SEQ ID N° 650 | 51 % sur 208 | AE000992. | Genbank | Archaeoglobus fulgidus |
| SEQ ID N° 651 | 50.00 % sur 150 | P00904 | Swissprot | Escherichia coli |
| SEQ ID N° 652 | 35.16 % sur 91 | P10377 | Swissprot | Escherichia coli |
| SEQ ID N° 653 | 42.19 % sur 64 | P00937 | Swissprot | Saccharomyces cerevisiae |
| SEQ ID N° 654 | 48.78 % sur 41 | Q57893 | Swissprot | Methanococcus jannaschii |
| SEQ ID N° 655 | 59.52 % sur 168 | P00931 | Swissprot | Saccharomyces cerevisiae |
| SEQ ID N° 656 | 36.61 % sur 112 | P00931 | Swissprot | Saccharomyces cerevisiae |
| SEQ ID N° 657 | 30.77 % sur 52 | Q59011 | Swissprot | Methanococcus jannaschii |
| SEQ ID N° 658 | 30.77% sur 208 | SLR1044 | cyanobase | *Synechocystis sp. |
| SEQ ID N° 659 | 32.74 % sur 113 | P22730 | Swissprot | Escherichia coli |
| SEQ ID N° 660 | 23.19 % sur 69 | P75774 | Swissprot | Escherichia coli |
| SEQ ID N° 661 | 40.54 % sur 37 | Q57733 | Swissprot | Methanococcus jannaschii |
| SEQ ID N° 662 | 32.22 % sur 90 | Q58161 | Swissprot | Methanococcus jannaschii |
| SEQ ID N° 663 | 30.23 % sur 86 | P37354 | Swissprot | Escherichia coli |
| SEQ ID N° 664 | 65.17 % sur 333 | Q58556 | Swissprot | Methanococcus jannaschii |
| SEQ ID N° 665 | 47.42 % sur 97 | MJ0946 | TIGR database | Methanococcus jannaschii |
| SEQ ID N° 666 | 94 % sur 346 | AB009526 | Genbank | Pyrococcus horikoshii |
| SEQ ID N° 667 | 68.00 % sur 150 | Q58535 | Swissprot | Methanococcus jannaschii |
| SEQ ID N° 668 | 43.48 % sur 46 | Q57633 | Swissprot | Methanococcus jannaschii |
| SEQ ID N° 669 | 48.17 % sur 191 | YDR190C | sgd | Saccharomyces cerevisiae |
| SEQ ID N° 670 | 36.97 % sur 119 | P47540 | Swissprot | Mycoplasma genitalium |
| SEQ ID N° 671 | 31.30 % sur 131 | P76594 | Swissprot | Escherichia coli |
| SEQ ID N° 672 | 33.04 % sur 112 | P76594 | Swissprot | Escherichia coli |
| SEQ ID N° 673 | 48.89 % sur 45 | P47699 | Swissprot | Mycoplasma genitalium |
| SEQ ID N° 674 | 38.18 % sur 55 | Q59011 | Swissprot | Methanococcus jannaschii |
| SEQ ID N° 675 | 45.00 % sur 40 | P47397 | Swissprot | Mycoplasma genitalium |
| SEQ ID N° 676 | 53.98 % sur 113 | P54015 | Swissprot | Methanococcus jannaschii |
| SEQ ID N° 677 | 42.03 % sur 138 | P02387 | Swissprot | Escherichia coli |
| SEQ ID N° 678 | 50.70 % sur 71 | P02375 | Swissprot | Escherichia coli |
| SEQ ID N° 679 | 37.14 % sur 70 | P02352 | Swissprot | Escherichia coli |
| SEQ ID N° 680 | 30.23 % sur 43 | P02373 | Swissprot | Escherichia coli |
| SEQ ID N° 681 | 69.03 % sur 113 | P54038 | Swissprot | Methanococcus jannaschii |
| SEQ ID N° 682 | 41.27 % sur 63 | P02389 | Swissprot | Escherichia coli |
| SEQ ID N ° 683 | 43.48 % sur 46 | P02361 | Swissprot | Escherichia coli |
| SEQ ID N° 684 | 62.40 % sur 125 | P54044 | Swissprot | Methanococcus jannaschii |
| SEQ ID N° 685 | 36.51 % sur 63 | P02356 | Swissprot | Escherichia coli |
| SEQ ID N° 686 | 65.85 % sur 41 | P54047 | Swissprot | Methanococcus jannaschii |
| SEQ ID N° 687 | 42.66 % sur 143 | Q60175 | Swissprot | Methanococcus jannaschii |
| SEQ ID N° 688 | 79 % sur 305 | AP000007 | Genbank | Pyrococcus horikoshii |
| SEQ ID N° 689 | 53.06 % sur 49 | P73450 | Swissprot | *Synechocystis sp. |
| SEQ ID N° 690 | 44.68 % sur 47 | P22730 | Swissprot | Escherichia coli |
| SEQ ID N° 691 | 49 % sur 605 | YC20-METJA | Swissprot | Methanococcus jannaschii |
| SEQ ID N° 692 | 34 % sur 446 | Y130-METJA | Swissprot | Methanococcus jannaschii |
| SEQ ID N° 693 | 37 % sur 1212 | Y124-METJA. | Swissprot | Methanococcus jannaschii |
| SEQ ID N° 694 | 34.43 % sur 61 | Q57007 | Swissprot | Haemophilus influenzae |
| SEQ ID N° 695 | 88 % sur 295 | AB009528 | Genbank | Pyrococcus horikoshii |
| SEQ ID N° 696 | 26.09 % sur 115 | MJ1317 | TIGR database | Methanococcus jannaschii |
| SEQ ID N° 697 | 30 % sur 250 | YQKDBASCU | Swissprot | Bacillus subtilis |
| SEQ ID N° 698 | 46.51 % sur 43 | P15047 | Swissprot | Escherichia coli |
| SEQ ID N° 699 | 91 % sur 365 | AB009529 . | Genbank . | Pyrococcus horikoshü |
| SEQ ID N° 700 | 74.07 % sur 189 | MJ1359 | TIGR database | Methanococcusjannaschii |
| SEQ ID N° 701 | 25.19 % sur 131 1 | P33322 | Swissprot | Saccharomyces cerevisiae |
| SEQ ID N° 702 | 28.57 % sur 77 | Q57892 | Swissprot | Methanococcus jannaschü |
| SEQ ID N° 703 | 37.65 % sur 85 | Q58381 | Swissprot | Methanococcus jannaschü |
| SEQ ID N° 704 | 42.03 % sur 69 | P23306 | Swissprot | Escherichia coli |
| SEQ ID N° 705 | 91 % sur 197 | AB009473 | Genbank | Pyrococcus horikoshü |
| SEQ ID N° 706 | 39.44 % sur 71 | Q57657 | Swissprot | Methanococcus jannaschü |
| SEQ ID N° 707 | 52.75 % sur 91 | P17242 | Swissprot | Escherichia coli |
| SEQ ID N° 708 | 24.29 % sur 70 | Q58752 | Swissprot | Methanococcus jannaschü |
| SEQ ID N° 709 | 50.00 % sur 24 | Q58055 | Swissprot | Methanococcus jannaschii |
| SEQ ID N° 710 | 40.19 % sur 107 | P09158 | Swissprot | Escherichia coli |
| SEQ ID N° 711 | 36.84 % sur 95 | P39389 | Swissprot | Escherichia coli |
| SEQ ID N° 712 | 26.19 % sur 84 | P02916 | Swissprot | Escherichia coli |
| SEQ ID N° 713 | 55.88 % sur 170 | P28691 | Swissprot | Escherichia coli |
| SEQ ID N° 714 | 52.00 % sur 25 | P47577 | Swissprot | Mycoplasma genitalium |
| SEQ ID N° 715 | 52.03 % sur 123 | P47623 | Swissprot | Mycoplasma genitalium |
| SEQ ID N° 716 | 43.33 % sur 30 | Q57755 | Swissprot | Methanococcus jannaschii |
| SEQ ID N° 718 | 37.18 % sur 78 | P22731 | Swissprot | Escherichia coli |
| SEQ ID N° 719 | 61.29 % sur 93 | Q60367 | Swissprot | Methanococcus jannaschii |
| SEQ ID N° 720 | 94 % sur 430 | AB009468 | Genbank | Pyrococcus horikoshii |
| SEQ ID N° 721 | 75% sur 622 | AB009468 | Genbank | Pyrococcus horikoshii |
| SEQ ID N° 721 | 83 % sur 1453 | AB009468 | Genbank | Pyrococcus horikoshii |
| SEQ ID N° 722 | 91 % sur 211 | AB009468 | Genbank | Pyrococcus horikoshii |
| SEQ ID N° 723 | 89 % sur 226 | AB009468 | Genbank | Pyrococcus horikoshii |
| SEQ ID N° 724 | 41.75 % sur 103 | Q58293 | Swissprot | Methanococcus jannaschii |
| SEQ ID N° 725 | 51.28 % sur 39 | P30745 | Swissprot | Escherichia coli |
| SEQ ID N° 726 | 35.29 % sur 68 | Q59011 | Swissprot | Methanococcus jannaschii |
| SEQ ID N° 727 | 42.98 % sur 114 | MJ1347 | TIGR database | Methanococcus jannaschii |
| SEQ ID N° 728 | 74 % sur 446 | AB009466 | Genbank | Pyrococcus horikoshii |
| SEQ ID N° 729 | 40.38 % sur 52 | P00805 | Swissprot | Escherichia coli |
| SEQ ID N° 730 | 42.86 % sur 42 | P37309 | Swissprot | Escherichia coli |
| SEQ ID N° 731 | 28.57 % sur 70 | P47378 | Swissprot | Mycoplasma genitalium |
| SEQ ID N° 732 | 56.45 % sur 62 | P38820 | Swissprot | Saccharomyces cerevisiae |
| SEQ ID N° 733 | 55.32 % sur 94 | P76316 | Swissprot | Escherichia coli |
| SEQ ID N° 734 | 35.85 % sur 53 | P33322 | Swissprot | Saccharomyces cerevisiae |
| SEQ ID N° 735 | 75 % sur 352 | AB009466 | Genbank | Pyrococcus horikoshii |
| SEQ ID.N° 736 | 27.14 % sur 70 | P35187 | Swissprot | Saccharomyces cerevisiae |
| SEQ ID N° 737 | 38 % sur 91 | RPOL-SULAC | Swissprot | Sulfolobus acidocaldarius |
| SEQ ID N° 737 | 91 % sur 80 | AP009521 | Genbank | Pyrococcus horikoshii |
| SEQ ID N° 738 | 68.49 % sur 73 | Q57983 | Swissprot | Methanococcus jannaschü |
| SEQ ID N° 739 | 47.69 % sur 65 | Q60326 | Swissprot | Methanococcus jannaschü |
| SEQ ID N° 740 | 39.13 % sur 69 | MJ1438 | TIGR database | Methanococcus jannaschü |
| SEQ ID N° 741 | 39.22 % sur 51 | SLL1957 | cyanobase | *Synechocystis sp. |
| SEQ ID N° 743 | 28.40 % sur 81 | P16701 | Swissprot | Escherichia coli |
| SEQ ID N° 744 | 35.62 % sur 73 | P16701 | Swissprot | Escherichia coli |
| SEQ ID N° 745 | 32.45 % sur 151 | P73450 | Swissprot | *Synechocystis sp. |
| SEQ ID N° 746 | 34.92 % sur 63 | MJ1227 | TIGR database | Methanococcus jannaschii |
| SEQ ID N° 748 | 64.06 % sur 64 | P00363 | Swissprot | Escherichia coli |
| SEQ ID N° 749 | 58.82 % sur 136 | Q57850 | Swissprot | Methanococcus jannaschii |
| SEQ ID N° 750 | 50.70 % sur 71 | Q57916 | Swissprot | Methanococcus jannaschii |
| SEQ ID N° 751 | 35.04 % sur 117 | P47466 | Swissprot | Mycoplasma genitalium |
| SEQ ID N° 752 | 46.90 % sur 145 | Q5781.8 | Swissprot | Methanococcus jannaschii |
| SEQ ID N° 753 | 31.58 % sur 133 | P02409 | Swissprot | Escherichia coli |
| SEQ ID N° 754 | 41.23 % sur 114 | P07117 | Swissprot | Escherichia coli |
| SEQ ID N° 755 | 39.39 % sur 33 | Q58950 | Swissprot | Methanococcus jannaschii |
| SEQ ID N° 756 | 42.59 % sur 54 | Q60337 | Swissprot | Methanococcus jannaschii |
| SEQ ID N° 757 | 41.25 % sur 80 | P37315 | Swissprot | Escherichia coli |
| SEQ ID N° 758 | 37.68 % sur 207 | P43828 | Swissprot | Haemophilus influenzae |
| SEQ ID N° 759 | 47.06 % sur 85 | P06128 | Swissprot | Escherichia coli |
| SEQ ID N° 760 | 38 % sur 415 | PPB4-BACSU | Swissprot | Bacillus subtilis |
| SEQ ID N° 761 | 76 % sur 1118 | D1030638 | Genbank | Pyrococcus horikoshii |
| SEQ ID N° 762 | 34.48 % sur 87 | P05459 | Swissprot | Escherichia coli |
| SEQ ID N° 763 | 85 % sur 483 | AP000002 | Genbank | Pyrococcus horikoshii |
| SEQ ID N° 764 | 80 % sur 1017 | AB009464 | Genbank | Pyrococcus horikoshii |
| SEQ ID N° 765 | 50.00 % sur 28 | P28304 | Swissprot | Escherichia coli |
| SEQ ID N° 766 | 32.61 % sur 46 | Q58352 | Swissprot | Methanococcus jannaschii |
| SEQ ID N° 767 | 38.46 % sur 91 | P18335 | Swissprot | Escherichia coli |
| SEQ ID N° 768 | 43.10 % sur 58 | P22939 | Swissprot | Escherichia coli |
| SEQ ID N° 769 | 37.31 % sur 67 | P36929 | Swissprot | Escherichia coli |
| SEQ ID N° 770 | 26.67 % sur 75 | P47507 | Swissprot | Mycoplasma genitalium |
| SEQ ID N° 771 | 52.63 % sur 76 | P54033 | Swissprot | Methanococcus jannaschii |
| SEQ ID N° 772 | 40.00 % sur 125 | P21155 | Swissprot | Escherichia coli |
| SEQ ID N° 773 | 60.67 % sur 178 | Q57586 | Swissprot | Methanococcus jannaschii |
| SEQ ID N° 774 | 28.00 % sur 75 | Q58295 | Swissprot | Methanococcus jannaschii |
| SEQ ID N° 775 | 64 % sur 501 | p95907 | Swissprot | Sulfolobus solfataricus |
| SEQ ID N° 776 | 92 % sur 261 | 059303 | Swissprot | Pyrococcus horikoshii |
| SEQ ID N° 777 | 86 % sur 838 | D1031563 | Genbank | Pyrococcus horikoshii |
| SEQ ID N° 778 | 33.56 % sur 146 | MJ1309 | TIGR database | Methanococcus jannaschii |
| SEQ ID N° 779 | 50.00 % sur 116 | P44335 | Swissprot | Haemophilus influenzae |
| SEQ ID N° 780 | 49.15 % sur 118 | MJ1512 | TIGR database | Methanococcus jannaschii |
| SEQ ID N° 781 | 53.95 % sur 76 | Q58130 | Swissprot | Methanococcus jannaschii |
| SEQ ID N° 782 | 52.94 % sur 85 | Q57911 | Swissprot | Methanococcus jannaschii |
| SEQ ID N° 783 | 26.56 % sur 64 | P43820 | Swissprot | Haemophilus influenzae |
| SEQ ID N° 784 | 37.84 % sur 74 | Q59049 | Swissprot | Methanococcus jannaschii |
| SEQ ID N° 785 | 38.98 % sur 59 | P41835 | Swissprot | Saccharomyces cerevisiae |
| SEQ ID N° 786 | 31.31 % sur 99 | MJ1117 | TIGR database | Methanococcus jannaschii |
| SEQ ID N° 787 | 53.70 % sur 54 | P47407 | Swissprot | Mycoplasma genitalium |
| SEQ ID N° 788 | 82.01 % sur 278 | Q57601 | Swissprot | Methanococcus jannaschii |
| SEQ ID N° 789 | 44.12 % sur 102 | P18335 | Swissprot | Escherichia coli |
| SEQ ID N° 790 | 44.44 % sur 36 | P20099 | Swissprot | Escherichia coli |
| SEQ ID N° 791 | 46.97 % sur 66 | Q57586 | Swissprot | Methanococcus jannaschii |
| SEQ ID N° 792 | 27.7 % sur 206 | G69401 | PIR | Archeoglobus fulgidus |
| SEQ ID N° 793 | 24.4 % sur 258 | D69689 | PIR | Bacilus subtilis |
| SEQ ID N° 794 | 25.4 % sur 236 | A70455 | PIR | Aquifex aeolicus |
| SEQ ID N° 795 | 23 % sur 289 | B64674 | PIR | Helicobacter pylori |
| SEQ ID N° 796 | 24 % sur 130 | 066540 | Swissprot | Aquifex aeolicus |
| SEQ ID N° 797 | 82 % sur 241 | H69101 | PIR | Pyrococcus horikoshii |
| SEQ ID N° 798 | 26.9 % sur 398 | H69101 | PIR | Methanobacterium ther |
| SEQ ID N° 799 | 29.1 % sur 244 | C69157 | PIR | Methanobacterium ther |
| SEQ ID N° 800 | 46.2 % sur 238 | E69315 | PIR | Archeoglobus fulgidus |

| | | | | |
|---|---|---|---|---|
| *Synechocystis sp. : Synechocystis sp. Souche PCC6803 | | | | |
| **Methanobacterium ther : Methanobacterium thermoautotrophicum. | | | | |

### Exemple 3 : Facteur C de réplication (RFC) de Pyrococcus abyssi (souche Orsay) (EXEMPLE NON-ILLUSTRATIF)

### A) Caractéristiques de la séquence

### 2 sous-unités

Références génome de Pyrococcus *abyssi* souche Orsay :
1. Petite sous-unité : PAB0068 ; activateur 1, facteur C de réplication, petite sous-unité (RFcS).
   Longueur : 4311 paires de bases ; 38 kDa théorique
   Position sur le génome : 103094-107404 (orientation +). Ce fragment ainsi délimité correspond à l'ORF 801.
2. Grande sous-unité : PAB 0069 ; activateur 1, facteur C de réplication, grande sous-unité (RfcL).
   Longueur : 1437 paires de bases ; 53 kDa
   Position sur le génome de séquence SEQ ID N° 1 : 107419-108855 (orientation +). Ce fragment ainsi délimité correspond à l'ORF 805.
   Famille de gènes : [9.1] métabolisme de l'ADN / réplication, recombinaison et réparation de l'ADN.
3. Séquences :
   - la séquence d'acides aminés SEQ ID N° 801 représente la séquence complète (intéines comprises) de la petite sous-unité ;
   - la séquence d'acides aminés SEQ ID N° 802 représente la séquence de la petite sous-unité dans laquelle les deux séquences d' intéines ont été excisées ;
   - les séquences d'acides aminés SEQ ID N° 803 et SEQ ID N° 804 représentent respectivement les deux séquences d'intéines excisées de la séquence SEQ ID N° 802 ;
   - la séquence d'acides aminés SEQ ID N° 805 représente la séquence de la grande sous-unité ;
   - la séquence de l'ORF 802 correspond ainsi à la séquence de l'ORF 801, ci-avant identifié, dans laquelle les séquences codant pour les peptides intéines de séquences SEQ ID N° 803 et N° 804 ont été supprimées ;
   - les séquences des ORF 803 et ORF 804 correspondent respectivement aux séquences codant pour les peptides intéines de séquences SEQ ID N° 803 et N° 804 de l'ORF 801.

### B) Clonage et expression

### Stratégie de clonage :

### Petite sous-unité

1. Excision des intéines : les intéines ont été éliminées par la technique de "slicing by overlap extension" (Ho et al., 1998, DNA and protein engineering techniques, vol 2, pp 50-55).
   Amorces utilisées :
   Site initiateur (Nde I) : 5'-GGCCTCTAATTTCATATGCGTGACATGGAG-3'
   Site de recouvrement, intéine 1 :
      sens direct : 5'-GGCCCCCAGGAGTTGGAAAGACAACCGCCGCTCTGGCCCT-3'
      sens réverse : 5'-AGGGCCAGAGCGGCGGTTGTCTTTCCAACTCCTGGGGGCC-3'
   Site de recouvrement, intéine 2 :
      Sens direct : 5'-ACGTAAGGTTTATTTTAAGCTGCAACTATTCGTCAAAGAT-3'
      Sens réverse : 5'-ATCTTTGACGAATAGTTGCAGCTTAAAATAAACCTTACGT-3'
      Site terminateur (BamHI) : 5'-GGGGGATCCCTTTATTTCTTCTTTCCGATT-3'
2. Sous-clonage et séquençage :
   Vecteur utilisé : pGEMT easy (Promega)
   Souche : *E. coli* DH5α
3. Expression :
   Vecteur utilisé : pET11a
   Souche : *E. coli* MC1061(DE3)

### Grande sous-unité

1. PCR : la PCR est réalisée avec les amorces suivantes :
   Amorces utilisées :
      Site initiateur (Nde I) : 5'-ATAAAGTGATGACATATGCCAGAAGTTCCC-3'
      Site terminateur (BamHI) : 5'-GGCCCTACTGGATCCAGCTACTTCTTTATG-3'.
2. Sous-clonage et séquençage :
   Vecteur utilisé : pBS/SK(-)
   Souche : *E. coli* DH5α
3. Expression :
   Vecteur utilisé : pET26b+
   Souche : *E*. *coli* MC1061(DE3)
   **Expression**
   Co-expression des deux sous-unités dans E. *coli* MC1061(DE3)
   Conditions : induction à D.O.₆₀₀ = 1,0 ; 0,6 mM IPTG ; 4 h à 37°C ; milieu : LB

### C) Purification

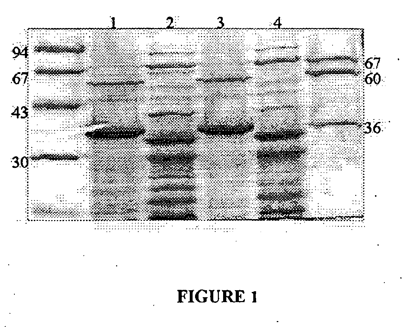

Culot cellulaire repris dans Tris-HCl 50 mM pH 8,0.
Choc thermique : 2 min à 90°C, puis 15 min à 80°C.
Masses molaires apparentes (cf. figure 1) :
Petite sous-unité : 36 kDa
Grande sous-unité : 56 kDa

### D) Caractérisation immuno-biochimique de la grande sous-unité

L'utilisation d'anticorps spécifiques contre la grande sous-unité du RF-C d'eucaryote indique que le site de liaison au PCNA est conservé. Par contre, le site de liaison à l'ADN n'est pas reconnu par l'anticorps.

Anticorps utilisés :
Anticorps A : monoclonal de souris contre le domaine de fixation de l'ADN.
Anticorps B : polyclonal de lapin contre le domaine de fixation du PCNA ; reconnaît, chez l'eucaryote, la zone des acides aminés 478 à 712 de la grande sous-unité du RFC.

**TABLEAU 3 : Pourcentage d'identité des séquences de RFC, petite sous-unité (S) ou grande sous-unité (L) entre différentes espèces d'Euryarchaéotes et de Crénarchaéotes**

| | *Pab S* | *Pho S* | *Mth S* | *Mja S* | *Afu S* | *Pfu L* | *Pab L* | *Pho L* | *Mth L* | *Mja L* | *Afu L* | *Sso S* | *APe S* | *SsO L* | *Ape L* |
|---|---|---|---|---|---|---|---|---|---|---|---|---|---|---|---|
| *Euryarchaeotes* | | | | | | | | | | | | | | | |
| *Pab S* | | | | | | | | | | | | | | | |
| *Pho S* | 96 | | | | | | | | | | | | | | |
| *Mth S* | 55 | 56 | | | | | | | | | | | | | |
| *Mja S* | 66 | 66 | 60 | | | | | | | | | | | | |
| *Afu S* | 56 | 56 | 52 | 65 | | | | | | | | | | | |
| *Pfu L* | 16 | 16 | 14 | 16 | 15 | | | | | | | | | | |
| *Pab L* | 17 | 17 | 14 | 16 | 16 | 81 | | | | | | | | | |
| *Pho L* | 17 | 17 | 14 | 16 | 16 | 81 | 85 | | | | | | | | |
| *Mth L* | 16 | 16 | 15 | 14 | 16 | 30 | 31 | 29 | | | | | | | |
| *MjaL* | 14 | 14 | 13 | 15 | 14 | 36 | 35 | 34 | 37 | | | | | | |
| *Afu L* | 17 | 17 | 15 | 16 | 15 | 37 | 36 | 37 | 30 | 36 | | | | | |

| *Crenarchaeotes* | | | | | | | | | | | | | | | |
|---|---|---|---|---|---|---|---|---|---|---|---|---|---|---|---|
| *Sso* S | 55 | 55 | 49 | 58 | 50 | 17 | 17 | 16 | 15 | 13 | 16 | | | | |
| *Ape S* | 55 | 55 | 47 | 53 | 49 | 15 | 16 | 16 | 15 | 13 | 17 | 59 | | | |
| *Sso L* | 15 | 14 | 14 | 16 | 16 | 28 | 28 | 30 | 24 | 27 | 28 | 18 | 16 | | |
| *Ape L* | 11 | 11 | 12 | 10 | 10 | 26 | 26 | 27 | 24 | 23 | 26 | 11 | 11 | 30 | |

### Exemple 4: Antigène nucléaire de prolifération cellulaire (PCNA) de pyrococcus abyssi (sourche Orsay) (EXEMPLE NON-ILLUSTRATIF)

### A) Caractéristiques de la séquence

Référence Génome de Pyrococcus *abyssi* souche Orsay : PAB 1465 ; antigène nucléaire de prolifération cellulaire (pol 30).

Famille de gènes : [9.1] métabolisme de l'ADN / réplication, recombinaison et réparation de l'ADN.

Longueur : 747 paires de bases ; 28 kDa

Position sur le génome : 1333299-1332553 (orientation -). Ce fragment ainsi délimité correspond à la séquence de l'ORF 806.

Séquences : la séquence d'acides aminés du polypeptide PCNA est représentée par la séquence SEQ ID N° 806.

### b) Clonage et expression

### Stratégie de clonage

La PCR est réalisée avec les amorces nucléotidiques suivantes :
Site initiateur (Nde I) : 5'-AGGTGCAAACATATGCCATTCGAGATAGTC-3'
Site terminateur (Bgl II) : 5'-AGTTAAAGATCTTTACTCCTCAACCCTGGG-3'.
Sous-clonage et séquençage :
Vecteur : pBS/SK-
Souche : *E. coli* DH5α
Clonage : pET26b+ (*E. coli* HMS174(DE3))
Expression :
Conditions : induction à D.O.₆₀₀ = 0,6 ; 1 mM IPTG ; 4 h à 37°C ; milieu : LB

### C) Purification

Extraction dans Tris-HCl 50 mM pH 8,0, NaCl 0,1 M
Choc thermique : 15 min, 75 °C
Chromatographie : Ressource Q (Pharmacia) ; tampon Tris-HCl 50 mM pH 7,5, 1 mM DTT, 0,1 mM EDTA ; gradient de NaCl de 0 à 0,5 M. Superdex 200 (Pharmacia) ; même tampon.

### D) Effets sur l'ADN polymérase I de Pyrococcus abyssi orsay

Tampon : Tris-HCl 50 mM pH 8,8, 1 mM dithiothréitol, 10 mM KCl, 5 mM MgCl₂, 0,4 mg/ml BSA.

Incubation :
Volume réactionnel de 20 µl contenant 0,2 mM de chacun des quatre dNTPs, 0,35 µM de [3H]dTTP (119 Ci/mmole) et 15 fmoles d'ADN de phage lambda amorcé avec un oligonucléotide complémentaire de la région 48502 à 48466 du génome du phage.

Incubation de 20 min à 70°C.

1 Unité d'ADN polymérase est la quantité d'enzyme qui permet l'incorporation de 1 nmole de dNTP par minute, dans ces conditions expérimentales.

**TABLEAU 4 : Caractéristique des polypeptides PCNA pour différentes espèces**

| | Espèce | Longueur (AA) | Poids Moléculaire |
|---|---|---|---|
| YEAST (levure) | *Saccharomyces cerevisiae* | 258 | 28 916 |
| HUMAIN | *Homo sapiens* | 261 | 28 768 |
| METJA | *Methanococcus jannashii* | 247 | 27 637 |
| PHO | *Pyrococcus horikoshii* | 249 | 28 097 |
| PAB | *Pyrococcus abyssi* | 249 | 28 035 |
| PFUR | *Pyrococcus furiosus* | 249 | 28 021 |
| TFU | *Thermococcus fumicolans* | 249 | 28 042 |
| METTH | *Methanobacterium thermoautotrophicum* | 244 | 27 968 |
| ARFUL | *Archaeoglobus fulgidus* | 245 | 27 266 |

**TABLEAU 5 : Pourcentage de similarité des séquences de polypeptide PCNA entre différentes espèces**

| | **HUMAIN** | **YEAST** | **METJA** | **PAB** | **PFUR** | **PHO** | **TFU** | **METTH** | **ARFUL** |
|---|---|---|---|---|---|---|---|---|---|
| **HUMAIN** | 100 | | | | | | | | |
| **YEAST** | 54 | 100 | | | | | | | |
| **METJA** | 40 | 43 | 100 | | | | | | |
| **PAB** | 38 | 41 | 55 | 100 | | | | | |
| **PFUR** | 36 | 40 | 55 | 96 | 100 | | | | |
| **PHO** | 38 | 41 | 56 | 97 | 96 | 100 | | | |
| **TFU** | 38 | 41 | 56 | 94 | 93 | 93 | 100 | | |
| **METTH** | 42 | 41 | 51 | 45 | 46 | 45 | 45 | 100 | |
| **ARFUL** | 36 | 36 | 47 | 42 | 41 | 41 | 41 | 39 | 100 |

**TABLEAU 6 : Pourcentage d'identité des séquences de polypeptide PCNA entre différentes espèces**

| | **HUMAIN** | **YEAST** | **METJA** | **PAB** | **PFUR** | **PHO** | **TFU** | **METTH** | **ARFUL** |
|---|---|---|---|---|---|---|---|---|---|
| **HUMAIN** | 100 | | | | | | | | |
| **YEAST** | 36 | 100 | | | | | | | |
| **METJA** | 27 | 30 | 100 | | | | | | |
| **PAB** | 25 | 26 | 43 | 100 | | | | | |
| **PFUR** | 25 | 27 | 41 | 89 | 100 | | | | |
| **PHO** | 26 | 27 | 43 | 93 | 90 | 100 | | | |
| **TFU** | 26 | 27 | 43 | 84 | 85 | 85 | 100 | | |
| **METTH** | 30 | 25 | 36 | 31 | 33 | 32 | 31 | 100 | |
| **ARFUL** | 24 | 24 | 34 | 27 | 26 | 25 | 27 | 27 | 100 |

Parmi les applications de la PCNA de Pyrococcus *abyssi* selon l'invention, on peut citer son utilisation pour :
- l'amélioration des performances enzymatiques de toute ADN polymérase thermostable ou non, d'eucaryote ou d'archaea , notamment pour
   * l'augmentation de processivité ; et pour
   * l'augmentation de la vitesse de polymérisation;
et comme :
- facteur dans la mise en oeuvre d'une PCR longue distance (synthèse de fragments supérieurs à 100 kb) ;
- facteur pour le séquençage de très longs fragments d'ADN en une réaction ;
- facteur dans la mise en oeuvre d'un système de réplication *in vitro,*
ainsi que pour l'augmentation de la vitesse de croissance de cultures cellulaires eucaryotes exprimant le polypeptide PCNA de Pyrococcus *abyssi.*

Les utilisations dudit polypeptide PCNA, ou le cas échéant de sa séquence codante, comme ci-avant indiquées, font partie de la présente invention.

### Références :

Hingorani, M. M., and M, O. D. (2000) Sliding clamps: A (tail)ored fit Cur. Biol. 10: R25-R29.
Jonsson, Z. O., Hindges, R., and Hubscher, U. (1998) Regulation of DNA replication and repair proteins through interaction with the front side of proliferating cell nuclear antigen EMBO J. 17: 2412-2425.
Kelman, Z. (1997) PCNA: Structure, functions and interactions Oncogene 14: 629-640.
Kelman, Z., and Hurwitz, J. (1998) Protein-PCNA interactions: a DNA-scanning mechanism? Trends Biochem. Sci. 23: 236-238.
Kelman, Z., and O'Donnel, M. (1995) Structural and functional similarities of prokaryotic and eukaryotic DNA polymerase sliding clamps Nucl. Acid Res. 23: 3613-3620.
Krishna, T. S. R., Kong, X. P., Gary, S., Burgers, P. M., and Kuriyan; J. (1994) Crystal structure of the eukaryotic DNA polymerase processivity factor PCNA Cell 79: 1233-1243.
Loor, G., Zhang, S. J., Zhang, P., Toomey, N. L., and Lee, M. Y. W. T. (1997) Identification of DNA replication and cell cycle proteins that interact with PCNA Nucl. Acid' Res. 25: 5041-5046.
Tsurimoto, T. (1998) PCNA, a multifunctional ring on DNA Biochim. Biophys. Acta 1443: 23-39.

### Exemple 5 : ADN polymérase II de Pyrococcus abyssi (souche Orsay)

L'ADN polymérase II issue de *P. abyssi* souche Orsay est composée de deux sous-unités :
- une petite sous-unité (gène : PAB2266, nom : polB)
- une grande sous-unité (gène : PAB2404, nom : polC).

Le gène polC codant pour la grande sous-unité de l'ADN polymérase II contient un gène codant pour une intéine (555 nucléotides) qui est compris entre les nucléotides 2862 et 3417 de la séquence codant pour le polypeptide de séquence SEQ ID N° 808 (séquence complète comprenant l'intéine).

Cet exemple décrit le clonage et les conditions d'obtention d'une ADN polymérase II recombinante thermostable par co-expression dans *E. coli* des gènes codant pour les deux sous-unités (le gène codant pour la grande sous unité de la pol II ayant été préalablement débarassé de la séquence codant pour l'intéine), sa purification et son utilisation dans différents domaines de la biologie moléculaire.

### A) Méthode de production d'une ADN POLYMERASE II thermostable

### A-1. Séquences des 2 sous-unités de l'ADN polymérase II

Caractéristiques :
# ADN POLYMERASE II : sous-unité 1 (2.7.7.7)
Séquence de la petite sous-unité, de l'ADN polymérase II (2.7.7.7)
Famille de gènes : [9.1] métabolisme de l'ADN / réplication, recombinaison et réparation de l'ADN
Référence Génome de Pyrococcus *abyssi* : PAB2266
Nom : polB
Position sur le génome de séquence SEQ ID N° 1 ; Début : 121402, Fin : 119546 (orientation -).
Ce fragment ainsi délimité correspond à la séquence de l'ORF 807.
Longueur du gène : 1857 pb.
# ADN POLYMERASE II : sous unité 2 (2.7.7.7)
Séquences de la grande sous-unité de l'ADN POLYMERASE II (2.7.7.7)
Famille de gènes : [9.1] métabolisme de l'ADN / réplication, recombinaison et réparation de l'ADN
Référence Génome de Pyrococcus *abyssi* Souche Orsay : PAB2404
Nom : poIC
Position sur le génome ; Début : 119543, Fin : 115179 (orientation -)
Ce fragment ainsi délimité correspond à l'ORF 808.
Longueur du gène : 4365 pb.

Séquences :
- la séquence d'acides aminés SEQ ID N° 807 représente la séquence de la sous-unité 1 de l' ADB polymérase II ;
- la séquence d'acides aminés SEQ ID N° 808 représente la séquence complète (intéine comprise) de la sous-unité 2 de l'ADN polymérase II ;
- la séquence d'acides aminés SEQ ID N° 809 représente la séquence de la sous-unité 2 de l'ADN polymérase II, intéine excisée (l'ORF 809 correspond à sa séquence nucléique codante) ;
- la séquence d'acides aminés SEQ ID N° 810 représente la séquence de l'intéine de la sous-unité 2 de l'ADN polymérase Il (l'ORF 810 correspond à sa séquence, nucléique codante).

### A-2. Clonage des 2 sous-unités de l'ADN polymérase II

Le clonage des 2 sous-unités de l'ADN polymérase II a été réalisé séparément dans des vecteurs d'expression de résistance à des antibiotiques différents.

# Clonage de la sous-unité 1 (pol B) dans le vecteur d'expression pET 11a
- Obtention de l'ADN génomique de P. *abyssi* souche Orsay
   La souche *P*. *abyssi* souche Orsay est cultivée en anaérobie à 95°C pendant 16 heures sur un milieu contenant 2 g/l peptone, 0,5 g/l extrait de levure, 10 g/l de soufre, 30 g/l sel de mer. La culture est réalisée en flacon de 100ml contenant 30 ml de milieu. A l'issue de la culture, les cellules sont récupérées par centrifugation, puis resuspendues dans 500 µl de tampon TE (tris 10 mM, EDTA 1 mM, pH 8,3). 500 µl de sarcosyl 10 % et 20 µl de protéinase K (20 mg/ml) sont ajoutés. Le mélange est ensuite incubé 1 h 30 à 50°C. A l'issue de la réaction, une extraction au phénol-chloroforme (v/v), suivie d'une précipitation à l'éthanol est réalisée nous permettant d'obtenir environ 1 mg d'ADN génomique.
- Amplification du gène par PCR et clonage dans un vecteur d'expression
   Des amorces basées sur la séquence du gène polB ont été synthétisées pour amplifier le gène par PCR (DNA thermal cycler, Stratagène). Les séquences des amorces utilisées sont les suivantes :
   Site initiateur (Nde I) : 5'-CAAAGGAGGTTGCT**CATATG**GATGAATTGGTTAAGG-3'
   Site terminateur (BamH1) : 5'-TTCCTTTGGA**GGATCC**ATCAACACCACCCGCTG-3'

Les conditions de la réaction de PCR sont les suivantes :

| | |
|---|---|
| ADN génomique | 20 ng |
| amorces | 100pmol de chaque |
| dNTP | 10 nmol de chaque (dATP, dCTP, dGTP, dTTP) |
| Tampon 10x Pfu polymérase | 5 µl |
| Pfu polymérase (Stratagene) | 1 U |
| H2O | qsp 50 µl |

Le mélange réactionnel est ensuite incubé : 15 min à 94°C, puis 20 fois (30s à 94°C, 30s à 45/50/55°C, 3 min à 72°C) et 15 min à 72°C. Une fois la réaction terminée, le produit PCR est digéré par les enzymes de restriction Nde1 et BamH1 et ligaturé dans le vecteur d'expression pET11a. Le vecteur résultant est ensuite transformé (technique de transformation au Ca C12) dans les cellules d'expression *E. coli* HMS174(DE3). La séquence du gène polB est contrôlé par séquençage complet de l'insert.

### 2- Sous-unité 2 (polC) dans le vecteur pET26b+

- Obtention de l'ADN génomique de *P. abyssi* souche Orsay:
   La souche *P. abyssi* souche Orsay est cultivée en anaérobie à 95°C pendant 16 heures sur un milieu contenant 2 g/l peptone, 0,5g/l extrait de levure, 10 g/l de soufre, 30 g/l sel de mer. La culture est réalisée en flacon de 100 ml contenant 30 ml de milieu. A l'issue de la culture, les cellules sont récupérées par centrifugation, puis resuspendues dans 500 µl de tampon TE (tris 10mM, EDTA 1mM, pH 8,3). 500 µl de sarcosyl 10 % et 20 µl de protéinase K (20 mg/ml) sont ajoutés. Le mélange est ensuite incubé 1 h 30 à 50°C. A l'issue de la réaction, une extraction au phénol-chloroforme (v/v), suivie d'une précipitation à l'éthanol est réalisée nous permettant d'obtenir environ 1 mg d'ADN génomique.
- Amplification du gène par PCR et clonage dans un vecteur d'expression :
   Des amorces basées sur la séquence du gène polB ont été synthétisées pour amplifier le gène par PCR (DNA thermal cycler, Stratagène). Les séquences des amorces utilisées sont les suivantes :
   Site initiateur (Nde I) : 5'-AGCGGGTGGTG**CATAT**GGAGCTTCCAAAGG-3'
   Site terminateur (SalI) : 5'-TCGATGAGTACTAAG**GTCGA**CTTAGTAGATTTCACG-3'

Les conditions de la réaction de PCR sont les suivantes :

| | |
|---|---|
| ADN génomique | 3 µg |
| amorces | 100 pmol de chaque |
| DNTP | 10 nmol de chaque (dATP, dCTP, dGTP, dTTP) |
| Tampon 10X Pfu polymérase | 5 µl |
| Pfu polymérase (Stratagene) | 1 U |
| H2O | qsp 50 µl |

Le mélange réactionnel est ensuite incubé : 15 min à 94°C, puis 20 fois (30s à 94°C, 30s à 45/50/55°C, 3 min à 72°C) et 15 min à 72°C. Une fois la réaction terminée, le produit PCR est digéré par les enzymes de restriction Nde1 et SalI et ligaturé dans le vecteur d'expression pET26b+. Le vecteur résultant est ensuite transformé (technique de transformation au CaC12) dans les cellules E. *coli* DH5α. La séquence du gène polC est contrôlée par séquençage complet de l'insert. Le gène polC est ensuite débarrassé du gène codant pour l'intéine en utilisant la méthode de " splicing by overlap extension " décrite dans l'article Ho, N. S., et al. (1990, DNA and protein engineering using the polymerase chain reaction: splicing by overlap extension. DNA Protein Eng. Tech. 2:50-55).

Pour cela deux amorces nucléotidiques ont été synthétisées :
M4 : 5'-GAGGAGAAACTGTGATGGAGATGAAGACGCTG-3'
M6 : 5'-CTCCATCACAGTTTCTCCTCTTCGCAGCGTGG-3'

Le vecteur résultant pET26b+ contenant le gène polC codant pour la grosse sous-unité de l'ADN polymérase II débarrassé du gène codant pour l'intéine est ensuite transformé dans les cellules d'expression *E. coli* HMS174(DE3). La séquence du gène polC débarassé du gène codant pour l'intéine est contrôlée par séquençage complet de l'insert.

### A-3. Production de l'ADN polymérase II

Deux stratégies ont été utilisées :
# Stratégie N°1 : Expression de chaque sous-unité clonée séparément dans les cellules d'expression *E.coli* HMS174 (DE3). Traitements par sonication puis choc thermique (15 min à 80°C) de chacun des deux clones recombinants puis mélange des sous-unités pour former une polymérase active.
   - Petite sous-unité (gène : PAB2266, nom : polB) :
      Les cellules d'expression *E.coli* HMS174(DE3) contenant le vecteur d'expression pET11a contenant le gène polB sont cultivées à 37°C dans 1 litre de milieu 2xYT (NaCl 10 g/l, extrait de levure 10 g/l, peptone 16 g/l) contenant de l'ampiciline à une concentration de 100 µg/ml.
      Quand la D.O.₆₀₀ ( D;O; pour densité optiqur) atteint 0,6, 1 mM d'IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCl 50 mM, pH 8,2, β-mercaptoéthanol 2 mM, glycérol 10 % . Les cellules sont ensuite soniquées et centrifugées, puis incubées 15 minutes à 80°C. Après centrifugation on récupère 10 ml d'une solution contenant la petite sous-unité (gène : PAB2266, nom : polB) de la polymérase II.
   - Grande sous-unité (gène : PAB2404, nom : poIC) :
      Les cellules d'expression *E. coli* HMS174(DE3) contenant le vecteur d'expression pET26b+ contenant le gène polC débarrassé du gène codant pour l'intéine sont cultivées à 37°C dans 1 litre de milieu 2xYT (NaCl 10 g/l extrait de levure 10 g/l, peptone 16 g/l) contenant de la kanamycine à une concentration de 34 µg/ml. Quand la D.O.₆₀₀ atteint 0,6, 1 mM d'IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCl 50 mM, pH 8,2, β-mercaptoéthanol 2 mM, glycérol 10 %. Les cellules sont ensuite soniquées et centrifugées, puis incubées 15 minutes à 80°C. Après centrifugation on récupère 10 ml d'une solution contenant la grande sous-unité (gène : PAB2404, nom : polC) de la polymérase II.
# Stratégie N° 2 : Co-expression des 2 sous-unités (polB + polC) dans la même cellule d'expression *E. coli* HMS174(DE3), traitements par sonication puis choc thermique (15 min à 80°C).

Les cellules d'expression *E.coli* HMS174(DE3) contenant le vecteur d'expression pET11a contenant le gène polB et le vecteur d'expression pET26b+ contenant le gène polC débarrassé du gène codant pour l'intéine sont cultivées à 37°C dans 1 litre de milieu 2xYT (NaCl 10 g/l, extrait de levure 10 g/l, peptone 16 g/l) contenant de l'ampiciline à une concentration de 100 µg/ml et de la kanamycine à une concentration de 34 µg/ml. Quand la D.O.₆₀₀ atteint 0,6, 1 mM d'IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCl 50 mM, pH 8,2, β-mercaptoéthanol 2 mM, glycérol 10 %. Les cellules sont ensuite soniquées et centrifugées, puis incubées 15 minutes à 80°C. Après centrifugation on récupère 10 ml d'une solution contenant la petite sous-unité (gène : PAB2266, nom : polB) de la polymérase II et la grande sous-unité (gène : PAB2404, nom : polC) de la polymérase II.

### B) Obtention, purification et caractérisation de l'ADN polymérase II

### B-1. Obtention de l'ADN polymérase active

Les fractions obtenues telles que décrit dans les paragraphes A3 (stratégies 1 et 2) sont testées pour leur activité polymérasique.

Le protocole de mesure de l'activité polymérase est le suivant :

Tampon du milieu réactionel :

Tris-HCl 50 mM pH 8,8, 1 mM dithiothréitol, 10 mM KCl, 0,4 mg/ml BSA, MgCl2 2,5mM.

Le volume réactionnel, de 20 µl contient 0,2 mM de chacun des quatre dNTPs (dATP, dCTP, dGTP, dTTP), 0,35 µM de [3H]TTP (119 Ci/mmole) et 6 µg/mL d'ADN de phage M13 amorcé avec un oligonucléotide complémentaire du génome du phage. L'incubation est de 20 min à 70°C. 18 µl de ce mélange réactionnel est déposé sur des filtres GF-C (Whatman) et précipité au TCA 5 %, pyrophosphate de sodium (8,9 g/l). Après 2 lavages à l'éthanol 95 % puis séchage des filtres, la radioactivité est mesurée au compteur à scintillation (Packard).

Une unité d'ADN polymérase est la quantité d'enzyme qui permet l'incorporation de 1 nmole de dNTP par minute, dans ces conditions expérimentales.

Le tableau 7 résume l'ensemble des résultats obtenus sur les différentes fractions testées :

**TABLEAU 7 : Activité polymérase de différentes fractions obtenues avec les stratégies 1 et 2**

| | Activité Spécifique (U/mg de protéines) |
|---|---|
| Sous-unités 1 : PolB (stratégie 1) | 0 |
| Sous-unités 2 : PolC (stratégie 1) | 0,2 |
| PoIB+ polC exprimés séparément puis mélangés (stratégie 1) | 1,3 |
| Co-expression de (polB + polC) dans la même cellule HMS(DE3) (stratégie 2) | 2,5 |
| Fraction témoin HMS(DE3) ne contenant pas les vecteurs exprimant les gènes polB et/ou polC | 0 |

### B-2. Purification de la polymérase II et propriétés

Une culture par co-expression des 2 sous-unités (polB + polC) dans la même cellule d'expression *E*. *coli* HMS174(DE3) a été réalisée.

Les cellules d'expression E. *coli* HMS174(DE3) contenant le vecteur d'expression pET11a contenant le gène polB et le vecteur d'expression pET26b+ contenant le gène polC débarrassé du gène codant pour l'intéine sont cultivées à 37°C dans 1 litre de milieu 2xYT (NaCl 10 g/l, extrait de levure 10 g/l, peptone 16 g/l) contenant de l'ampiciline à une concentration de 100 µg/ml et de la kanamycine à une concentration de 34 µg/ml. Quand la D.O.₆₀₀ atteint 0,6, 1 mM d'IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCl 50 mM, pH 8,2, 2-mercaptoéthanol 2mM, glycérol 10 %. Les cellules sont ensuite soniquées et centrifugées, puis incubées 15 minutes à 80°C. Après centrifugation on récupère 10 ml d'une solution contenant la petite sous-unité (gène : PAB2266, nom : polB) de la polymérase II et la grande sous-unité (gène : PAB2404, nom : polC) de la polymérase II. Un choc thermique de 20 min à 80°C est réalisé comme première étape de purification.

La visualisation des extraits obtenus sur SDS-PAGE (10 %) (cf. figure 5) permet d'obtenir les résultats suivants :
Masse molaire apparente de la sous-unité 1 : polB : 90kDa
Masse molaire apparente de la sous-unité 2 : polC : 140kDa.

Après le choc thermique, l'extrait est déposé sur une colonne de chromatographie : Resource Q (Pharmacia) ; tampon Phosphate de potassium 50 mM pH 6,5, 2 mM β-mercaptoéthanol. Le gradient de NaCl est de 0 à 0,5 M. Une fraction contenant une activité polymérase est éluée aux alentours de 350 mM en NaCl.

### Détermination du pH optimum

Le protocole de mesure de l'activité polymérase en fonction du pH est le suivant. Tampon du milieu réactionnel :

Phosphate de potassium 20 mM, MgC12 10mM pH 6, 6.5, 7, 7,5, 8.

Le volume réactionnel de 20 µl contient 0,2 mM de chacun des quatre dNTPs (dATP, dCTP, dGTP, dTTP), 0,35 µM de [3H]TTP (119 Ci/mmole) et 5 µg/mL de matrice polydA/oligodT (40:1). L'incubation est de 20 min à 70°C. 18 µl de ce mélange réactionnel est déposé sur des filtres GF-C (Whatman) et précipité au TCA 5 %, pyrophosphate de sodium (8,9 g/l). Après 2 lavages à l'éthanol 95 % puis séchage des filtrés, la radioactivité est mesurée au compteur à scintillation (Packard).

Une unité d'ADN polymérase est la quantité d'enzyme qui permet l'incorporation de 1 nmole de dNTP par minute, dans ces conditions expérimentales.

Le pH optimum de l'activité polymérase dans les conditions décrites est de 6,5 (cf. figure 6).

### C) Activation de la polymérase II par ajout de facteurs de réplication (PCNA) dans le milieu réactionnel

Le protocole de mesure de l'activité polymérase est le suivant :

Tampon du milieu réactionnel : Tris-HCl 50 mM pH 8,8, 1 mM dithiothréitol, 10 mM KCl, 0,4 mg/ml BSA, MgC12 2,5 mM. Le volume réactionnel est de 20 µl contenant 0,2 mM de chacun des quatre dNTPs (dATP, dCTP, dGTP, dTTP), 0,35 µM de [3H]TTP (119 Ci/mmole) et 6 µg/mL d'ADN de phage M13 amorcé avec un oligonucléotide du génome du phage ou du substrat poly dA-oligo dT 1:40. L'incubation est de 20 min à 70°C. 18 µl de ce mélange réactionnel est déposé sur des filtres GF-C (Whatman) et précipité au TCA 5 %, pyrophosphate de sodium (8,9 g/l). Après 2 lavages des filtres à l'éthanol 95 %, la radioactivité est mesurée au compteur à scintillation.

**TABLEAU 8 : Activation de l'ADN polymérase II par addition de PCNA**

| | DNA Polymerase II + PCN A (0,2 µg/ml) (U/ml) | DNA Polymerase II (U/ml) |
|---|---|---|
| M13 circulaire | 6,2 | 3,15 |
| PolydA / oligodT (1:40) | 6,95 | 3,75 |

Ainsi, l'ADN polymérase thermostable de type II selon la présente invention a été clonée et exprimée. L'enzyme recombinante comporte deux sous-unités (90 et 140kDa). La grande sous-unité est capable de catalyser la formation de liaisons phosphodiesters dans un processus de polymérisation. Cependant, cette activité est accrue en présence de la petite sous-unité de l'enzyme.

L'activité catalytique de l'ADN polymérase II thermostable de P. *abyssi* est sensible à la présence dans le milieu réactionnel du facteur de réplication : PCNA. En effet, celui-ci stimule l'activité catalytique de l'ADN polymérase II dans nos conditions réactionnelles.

Par conséquent, ladite ADN polymérase de type II pourra être utilisée pour :
- l'amélioration de l'amplification d'ADN par PCR par utilisation de l'ADN polymérase décrite avec ou sans utilisation de facteurs de réplication de type PCNA, RF-C et RPA ; notamment pour :
   - l'augmentation de la vitesse de polymérisation;
   - l'augmentation de processivité ;
   - l'augmentation de la fidélité ; et/ou
   - l'augmentation de la longueur des fragments synthétisés,
ainsi que pour :
- l'amélioration de l'efficacité de n'importe quelle ADN polymérase thermostable ou non, d'eucaryote ou d'archaea utilisé en PCR par mélange des 2 polymérases (dont la polymérase de type II selon l'invention) dans le milieu réactionnel avec ou sans utilisation de facteurs de réplication de type PCNA, rf-C et RPA, notamment pour :
   - l'augmentation de la vitesse de polymérisation ;
   - l'augmentation de processivité ;
   - l'augmentation de la fidélité ; et ou
   - l'augmentation de la longueur des fragments synthétisés.

Ladite ADN polymérase de type II selon la présente invention pourra être également utilisée pour :
- la PCR longue distance (synthèse de fragments supérieurs à 100 kb) ;
- la mise en oeuvre d'un sytème de duplication *in vitro* de chromosome bactérien, d'archaea ou eucaryotes ;
- le séquençage de très longs fragments d'ADN en une réaction ; ou
- la mise en oeuvre d'un système de réplication *in vitro.*

Lesdites utilisations ci-avant décrites pour ladite ADN polymérase de type II font bien entendu partie de la présente invention.

### Exemple 6 : Protéine de réplication A (RPA) de Pyrococcus abyssi (souche Orsay) (EXEMPLE NON-ILLUSTRATIF)

### A) Caractéristiques de la séquence

Protéine apparentée facteur A de réplication (rfA-like)
Référence Génome de Pyrococcus *abyssi* : PAB 2163.
Famille de gènes : [09.1] métabolisme de l'ADN / réplication, recombinaison et réparation de l'ADN.
Taille : 1125 paires de bases ; 43 kDa

Position sur le génome de séquence SEQ ID N° 1 : 292479-291352 (orientation -). Ce fragment ainsi délimité correspond à la séquence de l'ORF 811.

La séquence d'acides aminés de la protéine de réplication A est représentée par la séquence SEQ ID N° 811.

### B) Clonage, expression et caractérisation de la proteine A (facteur de réplication)

### 1. Clonage et expression

- Obtention de l'ADN génomique de P. *abyssi* souche Orsay :
   La souche P. *abyssi* souche Orsay est cultivée en anaérobie à 95°C pendant 16 heures sur un milieu contenant 2 g/l peptone, 0,5 g/l extrait de levure, 10 g/l de soufre, 30 g/l sel de mer. La culture est réalisée en flacon de 100ml contenant 30 ml de milieu. A l'issue de la culture, les cellules sont récupérées par centrifugation, puis resuspendues dans 500 µl de tampon TE (tris 10 mM, EDTA 1 mM, pH 8,3). 500 *µ*l de sarcosyl 10 % et 20 µl de protéinase K (20 mg/ml) sont ajoutés. Le mélange est ensuite incubé 1 h 30 à 50°C. A l'issue de la réaction, une extraction au phénol-chloroforme (v/v), suivie d'une précipitation à l'éthanol est réalisée nous permettant d'obtenir environ 1 mg d'ADN génomique.
- Amplification du gène par PCR et clonage dans un vecteur d'expression :
   Des amorces basées sur la séquence du gène polB ont été synthétisées pour amplifier le gène par PCR (DNA thermal cycler, Stratagène). Les séquences des amorces utilisées sont les suivantes :
      Site initiateur (NdeI) : 5'-ACCAAGTCATATGGTAAATGTTAAAAACCG-3'
      Site terminateur (BamH1) 5'-TAACTTGAGGATCCATCTCACATCACCCCC-3'

Les conditions de la réaction de PCR sont les suivantes :

| | |
|---|---|
| ADN génomique | 20ng |
| amorces | 100 pmol de chaque |
| DNTP | 10 nmol de chaque (dATP, dCTP, dGTP, dTTP) |
| Tampon 10X Pfu polymérase | 5 *µ*l |
| Taq polymérase (Appligene) | 0,3 U |
| H2O | qsp 50 *µ*l*.* |

Le mélange réactionnel est ensuite incubé : 15 min à 94°C, puis 20 fois (30s à 94°C, 30s à 50°C, 30s min à 72°C) et 15 min à 72°C. Une fois la réaction terminée, le produit PCR est cloné dans un vecteur de sequençage pGEMT easy (Promega). Le plasmide recombinant est transformé dans les cellules *E*. *coli* DH5α. La séquence du gène est contrôlée par séquençage complet de l'insert.

Le plasmide est ensuite digéré par les enzymes de restriction Nde1 et BamH1 et ligaturé dans le vecteur d'expression pET-26 b+. Le vecteur recombinant, pRPA, est ensuite transformé dans les cellules d'expression *E. coli* B121 pLys S(DE3). Expression :

Les cellules d'expression *E*. *coli* B121 pLys S (DE3) contenant le vecteur pRPA sont cultivées à 37°C dans 1 litre de milieu 2xYT (NaCI 10g/l, extrait de levure 10 g/l, peptone 16g/1) contenant de la kanamycine à une concentration de 34 µg/ml. Quand la D.O.₆₀₀ atteint 0,6, 0,5 mM de l'inducteur IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCl 50 mM, pH 8,2, 2-mercaptoéthanol 2 mM, glycérol 10 %. Les cellules sont ensuite soniquées et centrifugées, puis incubées 15 minutes à 75°C. Après centrifugation on récupère 10 ml d'une solution contenant le facteur de réplication A.

### 2. Caractérisation

- Visualisation des extraits obtenus sur SDS-PAGE (10 %) (cf. figure 7) :
   L'interprétation du gel de la figure 7 permet d'évaluer la masse molaire apparente de la protéine située entre 40 et 65 kDa.
- Analyse des séquences nucléotidiques et protéiques (cf. figure 8) :
   On notera la présence sur la séquence d'un hypothétique doigt de zinc qui interviendrait dans la reconnaissance de modification de base sur l'ADN et serait à l'origine de la forte affinité du RPA pour l'ADN simple brin.

Il existe également une région flexible en C terminal de la protéine. Cette région est nécessaire pour réaliser sa trimérisation.
Séquence de la région variable : EEIAEKIKELEESGLTTKEAARKLAEDEF.

**TABLEAU 9 : Pourcentage d'identité de la séquence de RPA entre différentes espèces**

| | RPA PAB | RPA PHO | RPA SCE | RPA HUM |
|---|---|---|---|---|
| RPA PAB | 100 | 85 | 13 | 13 |
| RPA PHO | | 100 | 13 | 12 |
| RPA SCE | | | 100 | 31 |
| RPA HUM | | | | 100 |

**TABLEAU 10 : Pourcentage de similarité de la séquence de RPA entre différentes espèces**

| | | | | |
|---|---|---|---|---|
| | RPA PAB | RPA PHO | RPA SCE | RPA HUM |
| RPA PAB | 100 | 92 | 26 | 24 |
| RPA PHO | | 100 | 26 | 23 |
| RPA SCE | | | 100 | 55 |
| RPA HUM | | | | 100 |

La protéine de réplication A selon la présente invention pourra être utilisée en particulier comme :
- facteur permettant d'améliorer la fidélité *in vitro* de toute ADN polymérase thermostable ou non, d'eucaryote ou d'archaea ;
- facteur pour le séquençage de très longs fragments d'ADN en une réaction ; et/ou
- facteur pour la mise en oeuvre d'un système de réplication *in vitro.*
   Les utilisations de la protéine de réplication A de Pyrococcus selon l'invention telles que ci-avant mentionnées font bien entendu partie de la présente invention.

### Références:

Ye Lao et al (2000). Replication proteine A interactions with DNA. III. Molecular basis of recognition of damaged DNA
Biochemistry feb 8 ;39(5) :850-9
Lavrick OI et al (1999).RPA subunit arrangement near the 3' -end of the primer is modulated by the lenth of the template strand and cooperative proteine interaction
Nucleic Acids Res nov 1 ;27(21) :4235-40
Wang Y et al (1999). Roles of replication proteine A and DNA-dependent proteine Kinase in the regulation of DNA replication following DNA damage
J Biol Chem jul 30 ;274(31) ;22060-4
Perdigao j et al (1999). Molecular cloning, developmental expression, and cellular localization of the 70 kDa RPA-1 subunit of Drosophila melanogaster
DNA cell biol dec; 18(12):923-36

### Exemple 7 : ADN polymérase I de Pyroccocus abyssi (souche Orsay) (EXEMPLE NON-ILLUSTRATIF)

Objet de l'étude : clonage, expression et caractérisation d'une ADN polymérase de type I d'origine archaebactérienne hyperthermophile.

Cette étude intervient dans le cadre du séquençage et de la valorisation du génome de Pyroccocus *abyssi* - souche Orsay.

Le présent exemple décrit le clonage, les conditions d'obtention et la caractérisation de l'ADN polymérase I recombinante thermostable exprimée dans *E.coli.*

### A) Méthode de production de l'ADN polymérase I

### 1. Caractéristiques des séquences

**ADN polymérase I**
Séquences de l' ADN POLYMERASE I :
Famille de gènes : [9.1] métabolisme de l'ADN / réplication, recombinaison et réparation de l'ADN
Référence Génome de Pyrococcus *abyssi :* PAB 1128
Nom : pol I
Position sur le génome de séquence SEQ ID N° 1 ; Début : 1695183 ; Fin : 1697495
Longueur du gène : 2313bp, Orientation dans le génome : +
La séquence d'acides aminés de l'ADN polymérase I est représentée par la séquence SEQ ID N° 812.

### 2. Clonage et expression

- Obtention de l'ADN génomique de *P. abyssi* souche Orsay :
   La souche *P. abyssi* souche Orsay est cultivée en anaérobie à 95°C pendant 16 heures sur un milieu contenant 2 g/l peptone, 0,5g/l extrait de levure, 10 g/l de soufre, 30 g/l sel de mer. La culture est réalisée en flacon de 100ml contenant 30 ml de milieu. A l'issue de la culture, les cellules sont récupérées par centrifugation, puis resuspendues dans 500 µl de tampon TE (tris 10 mM, EDTA 1 mM, pH 8,3). 500 µl de sarcosyl 10 % et 20 µl de protéinase K (20 mg/ml) sont ajoutés. Le mélange est ensuite incubé 1 h 30 à 50°C. A l'issue de la réaction, une extraction au phénol-chloroforme (v/v), suivie d'une précipitation à l'éthanol 95 % est réalisée nous permettant d'obtenir environ 1 mg d'ADN génomique.
- Amplification du gène par PCR et clonage dans un vecteur d'expression :
   Des amorces basées en partie sur la séquence du gène pol I ont été synthétisées pour amplifier le gène par PCR (DNA thermal cycler, Stratagène). Les séquences des amorces utilisées sont les suivantes :
      Site initiateur (Nde I) : 5'-CAGATTGGGTGGGG**CATATG**ATAATCGATGC-3'
      Site terminateur (BamH1) : 5'-CCCGA**GGATCC**TAGAACTTAAGCCATGCTCC-3'

Les conditions de la réaction de PCR sont les suivantes :

| | |
|---|---|
| ADN génomique | 20ng |
| amorces | 100 pmol de chaque |
| dNTP | 10 nmol de chaque (dATP, dCTP, dGTP, dTTP) |
| Tampon 10x Pfu polymérase | 5 µl |
| Pfu polymérase (Stratagene) | 1 U |
| H2O | qsp 50 µl. |

Le mélange réactionnel est ensuite incubé : 15 min à 94°C, puis 20 fois (30s à 94°C, 30s à 45/50/55°C, 3 min à 72°C) et 15 min à 72°C. Une fois la réaction terminée, le produit PCR est digéré par les enzymes de restriction Nde1 et BamH1 et ligaturé dans le vecteur d'expression pET26b. Le produit de la ligation est utilisé pour transformer (technique de transformation au CaCl2) les cellules d'expression E.coli BL21(DE3) pLysS. La séquence du gène pol I est controlé par séquençage du plasmide recombinant pET-pol I.
Expression du gène pol I :
Les cellules d'expression *E*. *coli* BL21(DE3) pLysS contenant le vecteur recombinant pET-pol I sont cultivées à 37°C dans 1 litre de milieu 2xYT (NaCl 10 g/l, extrait de levure 10 g/l, peptone 16 g/l) contenant de la kanamycine à une concentration de 34 µg/ml. Quand la D.O.₆₀₀ atteint 0,6, 0,5 mM de l'inducteur IPTG (isopropyl-β-D-thiogalactoside) est ajouté au milieu et l'incubation à 37°C se poursuit 15 heures. La culture est centrifugée et les cellules sont resuspendues dans 10 ml de tampon Tris-HCI 10 mM pH 8,0, NaCl 10 mM, MgC12 2mM, Triton X 1 %. Les cellules sont ensuite soniquées et centrifugées, puis incubées 15 minutes à 75°C. Après centrifugation on récupère 10 ml d'une solution contenant l'ADN polymérase potl.

### B) Caractérisation

1. Visualisation de l'expression de l'ADN polymérase 1 dans E. *coli* BL21(DE3) pLysS par SDS-PAGE
   Tous les extraits déposés ont été dénaturés 15 min à 70°C (cf. figure 9).
   Masse molaire apparente de l'ADN polymérase 1: 85kDa.
2. Purification de l'ADN polymérase I
   Extraction dans Tris-HCl 10 mM pH 8,0, NaCl 10 mM, MgCl2 2 mM, Triton X 1 %.
   Choc thermique : 15 min, 70°C.
   Chromatographie : Ressource Q (Pharmacia) ; Tris-HCl 50 mM pH 8,0, gradient de NaCl de 0 à 0,5 M.

L'ADN polymérase I est éluée aux alentours de 150 mM en NaCl.

### 3. Propriétés de l'ADN polymérase I

### a) Détermination du pH optimum

Le protocole de mesure de l'activité polymérase en fonction du pH est le suivant :
Tampon du milieu réactionel :
   Tris-HCl 50 mM, Glycine-NaOH 50 mM, et CAPS 50 mM pH 7,5 à 11.
   Le volume réactionnel de 20 µl contient 0,2 mM de chacun des quatre dNTPs (dATP, dCTP, dGTP; dTTP), 0.35 µM de [3H]TTP (119 Ci/mmole), 2,5 mM de MgCl2 et 5 µg/mL de matrice polydA/oligodT (40:1). L'incubation est de 20 min à 70°C. 15 µl de ce mélange réactionnel est déposé sur des filtres GF-C (Whatman) et précipité au TCA 5 % , pyrophosphate de sodium (8,9 g/l). Après 2 lavages à l'éthanol 95 % puis séchage des filtres, la radioactivité est mesurée au compteur à scintillation (Packard).
   Une unité d'ADN polymérase est la quantité d'enzyme qui permet l'incorporation de 1 nmole de dNTP par minute, dans ces conditions expérimentales.
   Le pH optimum de l'activité polymérase dans les conditions décrites est compris entre 9 et 9,5 (cf. figure 10).

### b) Détermination de la température optimum

Tampon du milieu réactionel :
Tris-HCl 50 mM pH 8,8, 1 mM dithiothréitol, 10 mM KCl, 0,4 mg/ml BSA, MgCl2 2,5 mM.

Le volume réactionnel de 20 µl contient 0,2 mM de chacun des quatre dNTPs (dATP, dCTP, dGTP, dTTP), 0,35 µM de [3H]TTP (119 Ci/mmole) et 5 µg/mL de matrice polydA/oligodT (40: 1). L'incubation est de 20 min de 37°C à 90°C. 15 µl de ce mélange réactionnel est déposé sur des filtres GF-C (Whatman) et précipité au TCA 5 %, pyrophosphate de sodium (8,9 g/l). Après 2 lavages à l'éthanol 95 % puis séchage des filtres, la radioactivité est mesurée au compteur à scintillation (Packard).

Une unité d'ADN polymérase est la quantité d'enzyme qui permet l'incorporation de 1 µmole de dNTP par minute, dans ces conditions expérimentales.

La température optimale de l'activité polymérase dans les conditions décrites est comprise entre 55 et 65°C.

### c) Détermination de la concentration optimale en MgC12

Tampon du milieu réactionel :
Tris-HCI 50 mM pH 8,8, 1 mM dithiothréitol, 10 mM KCI, 0,4 mg/ml BSA, MgC12 de 0 à 50 mM.

Le volume réactionnel de 20 µl contient 0,2 mM de chacun des quatre dNTPs (dATP, dCTP, dGTP, dTTP), 0,35 µM de [3H]TTP (119 Ci/mmole) et 5 µg/mL de matrice polydA/oligodT. (40:1). L'incubation est de 20 min de 37°C à 90°C. 15 µl de ce mélange réactionnel est déposé sur des filtres GF-C (Whatman) et précipité au TCA 5 %, pyrophosphate de sodium (8,9g/l). Après 2 lavages à l'éthanol 95 % puis séchage des filtres, la radioactivité est mesurée au compteur à scintillation (Packard). Une unité d'ADN polymérase est la quantité d'enzyme qui permet l'incorporation de 1 µmole de dNTP par minute, dans ces conditions expérimentales.

La concentration optimale en MgCl2 de activité polymérase de la poll dans les conditions décrites est de 3mM.

### d) Détermination de la concentration optimale en KCl

Tampon du milieu réactionel :
Tris-HCl 50 mM pH 8,8, 1 mM dithiothréitol, KCl de 0 à 100 mM, 0,4 mg/ml BSA, MgCl2 2,5 mM.

Le volume réactionnel de 20 µl contient 0,2 mM de chacun des quatre dNTPs (dATP, dCTP, dGTP, dTTP), 0.35 µM de [3H]TTP (119 Ci/mmole) et 5 µg/mL de matrice polydA/oligodT (40:1). L'incubation est de 20 min de 37°C à 90°C. 15 µl de ce mélange réactionnel est déposé sur des filtres GF-C (Whatman) et précipité au TCA 5 %, pyrophosphate de sodium (8,9 g/l). Après 2 lavages à l'éthanol 95 % puis séchage des filtres, la radioactivité est mesurée au compteur à scintillation (Packard). Une unité d'ADN polymérase est la quantité d'enzyme qui permet l'incorporation de 1 *µ*mole de dNTP par minute, dans ces conditions expérimentales.

La concentration optimale en KCl de activité polymérase de la polI dans les conditions décrites est comprise entre 50 et 80 mM.

### e) Mise en évidence d'une activité exonucléase 3' ----> 5'

Le protocole de détection d'une activité exonucléase 3' ---> 5' utilisée est le suivant : du plamide pBluescript SK est digéré par l'enzyme de restriction EcoRI. Le radionucléotide [3H]TTP ainsi que les 4 nucléotides non marqués (dATP, dCTP, dGTP, dTTP) sont incorporés à l'aide du fragment de Klenow de l'ADN polymérase de *E*. *coli.* Après une heure de marquage à 37°C, le plamide est purifié sur colonne QUIAGEN puis incubé avec l'ADN polymérase I recombinante. Après précipitation de l'ADN au TCA 10 %, la radioactivité résiduelle est mesurée compteur à scintillation (Packard). L'activité polymérase de l'ADN polymérase I a été mesurée dans les mêmes conditions et comparée à celle de la Taq polymérase et la Pfu polymérase (Stratagène).

Une unité de chacune des ADNs polymérases a été utilisée.

**TABLEAU 11 : Mesure de l'activité de l'ADN polymérase I recombinante comparée à la Taq et Pfu polymérase**

| | % Activité exonucléase 3' ---> 5' |
|---|---|
| Pol I de P.abyssi | 100 |
| Pfu (stratagène) | 94 |
| Taq polymérase (Appligène) | 0 |

Par conséquent, ladite ADN polymérase de type 1 pourra être utilisée pour :
- l'amélioration de l'amplification d'ADN par PCR par utilisation de l'ADN polymérase I décrite avec ou sans utilisation de facteurs de réplication de type PCNA, RF-C et RPA grâce à la présence d'une activité exonucléase 3' ---> 5' ; notamment pour :
   • l'augmentation de la vitesse de polymérisation ;
   • l'augmentation de processivité ;
   • l'augmentation de la fidélité ; et/ou
   • l'augmentation de la longueur des fragments synthétisés ;
   • la robustesse de l'enzyme (permettant d'augmenter le nombre de cycles PCR),
ainsi que pour :
- l'amélioration de l'efficacité de toute ADN polymérase thermostable ou non, d'eucaryote ou d'archaea utilisé en PCR par mélange des 2 polymérases (dont la polymérase de type I selon l'invention) dans le milieu réactionnel avec ou sans utilisation de facteurs de réplication de type PCNA, rf-C et RPA, notamment pour :
   - l'augmentation de la vitesse de polymérisation ;
   - l'augmentation de processivité ;
   - l'augmentation de la fidélité ; et/ou
   - l'augmentation de la longueur des fragments synthétisés.

Ladite ADN polymérase de type I selon la présente invention pourra être également utilisée pour :
- la PCR longue distance (synthèse de fragments supérieurs à 100 kb) ;
- la mise en oeuvre d'un sytème de duplication *in vitro* de chromosome bactérien, d'archaea ou eucaryotes ;
- le séquençage de très longs fragments d'ADN en une réaction ; ou
- la mise en oeuvre d'un système de réplication *in vitro.*

Lesdites applications ci- avant mentionées pour l'ADN polymérase de type 1 selon l'invention, font également partie de la présente invention.

### Références bibliographiques

Altschul, S.F. et al., 1990, J. Mol. Biol., 215 : 403-410.
Barany, F., 1911, Proc. Natl. Acad. Sci. USA, 88 : 189-193.
Buckholz, R.G., 1993, Curr. Op. Biotechnology, 4 : 538-542.
Burg, J.L. et al., 1996, Mol. and Cell. Probes, 10 :257-271.
Chu ,B.C.F. et al., 1986, Nucleic Acids Research, 14:5591-5603.
Duck, P. et al., 1990, Biotechniques, 9 : 142-147.
Edwards, C.P., and Aruffo, A., 1993, Curr. Op. Biotechnology 4 : 558-563.
Erlich, H.A., 1989, In PCR Technology. Principles and Applications for DNA Amplification. New York : Stockton Press.
Guateli, J.C. et al., 1990, Proc. Natl. Acad. Sci. USA, 87: 1874-1878.
Hingorani, M. M., and M, O. D. (2000) Sliding clamps: A (tail)ored fit Cur. Biol. 10: R25-R29.
Houbenweyl, 1974, in Meuthode der Organischen Chemie, E. Wunsch Ed., Volume 15-I et 15-II, Thieme, Stuttgart.
Innis, M.A. et al. 1990, in PCR Protocols. A guide to Methods and Applications. San Diego : Academic Press.
Jonsson, Z. O., Hindges, R., and Hubscher, U. (1998) Regulation of DNA replication and repair proteins through interaction with the front side of proliferating cell nuclear antigen EMBO J. 17:2412-2425.
Kelman, Z. (1997) PCNA: Structure, functions and interactions Oncogene 14: 629-640.
Kelman, Z., and Hurwitz, J. (1998) Protein-PCNA interactions: a DNA-scanning mechanism Trends Biochem. Sci. 23:236-238.
Kelman, Z., and O'Donnel, M. (1995) Structural and functional similarities of prokaryotic and eukaryotic DNA polymerase sliding clamps Nucl. Acid Res. 23:3613-3620.
Kievitis, T. et al., 1991, J. Virol. Methods, 35 : 273-286.
Krishna, T. S. R., Kong, X. P., Gary, S., Burgers, P. M., and Kuriyan, J. (1994) Crystal structure of the eukaryotic DNA polymerase processivity factor PCNA Cell 79:1233-1243.
Kwoh, D.Y. et al., 1989, Proc. Natl. Acad. Sci. USA, 86 : 1173-1177.
Lavrick O.I. et al. (1999). RPA subunit arrangement near the 3'-end of the primer is modulated by the lenth of the template strand and cooperative proteine interaction, Nucleic Acids Res nov 1 ; 27(21) : 4235-40.
Loor, G., Zhang, S. J., Zhang, P., Toomey, N. L., and Lee, M. Y. W. T. (1997) Identification of DNA replication and cell cycle proteins that interact with PCNA Nucl. Acid Res. 25: 5041-5046.
Luckow, V.A., 1993, Curr. Op. Biotechnology, 4 : 564-572.
Merrifield, R.D., 1966, J. Am. Chem. Soc., 88(21) : 5051-5052.
Miele, E.A. et al., 1983, J. Mol. Biol., 171 : 281-295.
Perdigao J. et al. (1999). Molecular cloning, developmental expression, and cellular localization of the 70 kDa RPA-1 subunit of Drosophila melanogaster, DNA cell. biol. dec ; 18(12) : 923-36.
Raulston JE., Mol Microbiol 1995 15:607-616.
Sambrook, J. et al., 1989, In Molecular cloning : A Laboratory Manual. Cold Spring Harbor, NY : Cold Spring Harbor Laboratory Press.
Segev D., 1992, in « Non-radioactive Labeling and Detection of Biomolecules ». Kessler C. Springer Verlag, Berlin, New-York : 197-205.
Tsurimoto, T. (1998) PCNA, a multifunctional ring on DNA Biochim. Biophys. Acta 1443:23-39.
Walker, G.T. et al., 1992, Nucleic Acids Research, 20 : 1691-1696.
Walker, G.T. et al., 1992, Proc. Natl. Acad. Sci. USA, 89 : 392-396.
Wang Y. et al..(1999). Roles of replication proteine A and DNA-dependent proteine Kinase in the regulation of DNA replication following DNA damage, J. Biol. Chem. jul 30 ; 274(31) : 22060-4.
White, B.A. et al., 1997, Methods in Molecular Biology, 67, Humana Press, Towota
Ye Lao et al. (2000). Replication proteine A interactions with DNA. III. Molecular basis of recognition of damaged DNA, Biochemistry feb 8 ; 39(5) : 850-9.

## Revendications

1. Séquence nucléotidique isolée ou purifiée codant pour une protéine de séquence d'acides aminés SEQ ID N° 807.

2. Séquence nucléotidique isolée ou purifiée, **caractérisée en ce qu'**elle comprend une séquence nucléotidique choisie parmi :
a) une séquence nucléotidique selon la revendication 1 ; et
b) une séquence nucléotidique complémentaire à une séquence telle que définie en a).

3. Séquence nucléotidique selon la revendication 1, **caractérisée en ce qu'**il s'agit d'une séquence ADN ou ARN.

4. Polypeptide codé par une séquence nucléotidique selon l'une des revendications 1 et 3.

5. Polypeptide de Pyrococcus *abyssi* de séquence SEQ ID N° 807.

6. Polypeptide **caractérisé en ce qu'**il comprend un polypeptide choisi parmi :
a) un polypeptide selon l'une des revendications 4 et 5 ; et
b) un fragment d'au moins 100 acides aminés d'un polypeptide de séquence SEQ ID N° 807 et accroissant l'activité de formation de liaisons phosphodiesters de la grande sous unité de l'ADN polymérase thermostable de type II.

7. Séquence nucléotidique isolée ou purifiée codant pour une protéine de séquence d'acides aminés SEQ ID N° 809.

8. Séquence nucléotidique isolée ou purifiée, **caractérisée en ce qu'**elle comprend une séquence nucléotidique choisie parmi :
a) une séquence nucléotidique selon la revendication 7 ; et
b) une séquence nucléotidique complémentaire à une séquence telle que définie en a).

9. Séquence nucléotidique selon la revendication 7, **caractérisée en ce qu'**il s'agit d'une séquence ADN ou ARN.

10. Polypeptide codé par une séquence nucléotidique selon l'une des revendications 7 et 9.

11. Polypeptide de Pyrococcus *abyssi* de séquence SEQ ID N° 809.

12. Polypeptide **caractérisé en ce qu'**il comprend un polypeptide choisi parmi :
a) un polypeptide selon l'une des revendications 10 et 11 ; et
b) un fragment biologiquement actif à activité ADN polymérase d'au moins 100 acides aminés d'un polypeptide de séquence SEQ ID N° 809 et comprenant les acides aminés 954 et suivants de séquence SEQ ID N° 809.

13. Séquence nucléotidique codant pour un polypeptide selon la revendication 6 ou 12.

14. Séquence nucléotidique utilisable comme amorce ou sonde, **caractérisée en ce que** ladite séquence est choisie parmi les séquences nucléotidiques selon l'une des revendications 1 à 3, 7 à 9 et 13.

15. Séquence nucléotidique selon la revendication 14, **caractérisée en ce qu'**elle est immobilisée sur un support, de manière covalente ou non-covalente.

16. Vecteur de clonage, et/ou d'expression, **caractérisé en ce qu'**il contient une séquence nucléotidique selon l'une des revendications 1 à 3, 7 à 9 et 13.

17. Cellule hôte, **caractérisée en ce qu'**elle est transformée par un vecteur selon la revendication 16.

18. Animal, excepté l'Homme, comprenant une cellule transformée selon la revendication 17.

19. Procédé de préparation d'un polypeptide selon l'une des revendications 4 à 6 et 10 à 12, **caractérisé en ce qu'**il met en oeuvre un vecteur selon la revendication 16, une cellule selon la revendication 17 ou un animal selon la revendication 18.

20. Polypeptide recombinant selon l'une des revendications 4 à 6 et 10 à 12 susceptible d'être obtenu par un procédé selon la revendication 19.

21. Procédé de préparation d'un polypeptide synthétique, **caractérisé en ce qu'**il utilise une séquence d'acides aminés d'un polypeptide selon l'une des revendications 4 à 6 et 10 à 12.

22. Polypeptide hybride, **caractérisé en ce qu'**il comporte au moins la séquence d'un polypeptide selon l'une des revendications 4 à 6 et 10 à 12.

23. Séquence nucléotidique codant pour un polypeptide hybride selon la revendication 22.

24. Vecteur **caractérisé en ce qu'**il contient une séquence nucléotidique selon la revendication 23.

25. Polypeptide hybride selon la revendication 22, **caractérisé en ce qu'**il s'agit d'un polypeptide recombinant susceptible d'être obtenu par la mise en oeuvre d'un vecteur selon la revendication 24.

26. Procédé de biosynthèse de composé d'intérêt, **caractérisé en ce qu'**il met en oeuvre un polypeptide selon l'une des revendications 4 à 6 et 10 à 12.

27. Utilisation de polypeptide selon l'une des revendications 4 à 6 et 10 à 12, pour la biosynthèse de composé d'intérêt.

28. Procédé pour la polymérisation et/ou l'amplification d'un acide nucléique **caractérisé en ce qu'**il met en oeuvre un polypeptide à activité ADN polymérase selon l'une des revendications 4 à 6, 10 à 12, 20, 22 et 25.

29. Procédé selon la revendication 28, **caractérisé en ce qu'**il comprend l'étape de mise en contact d'un échantillon biologique avec ledit polypeptide, ou un de ses fragments à activité ADN polymérase dans des conditions permettant une réaction de polymérisation et/ou d'amplification entre ledit polypeptide et les acides nucléiques présents dans l'échantillon biologique.

30. Réactif ou nécessaire comprenant un polypeptide à activité ADN polymérase selon l'une des revendications 4 à 6, 10 à 12, 20, 22 et 25.

31. Utilisation d'un réactif ou nécessaire selon la revendication 30, pour la détection et/ou l'identification d'acide nucléique cible dans un échantillon biologique, notamment pour la détection et/ou l'identification d'acide nucléique spécifique de micro-organisme pathogène.

32. Utilisation d'un réactif ou nécessaire selon la revendication 30, pour la détection et/ou l'identification d'acide nucléique cible, notamment de gène d'intérêt ou de mutation dudit gène d'intérêt dans un échantillon biologique.

## Claims

1. Isolated or purified nucleotide sequence encoding a protein of amino acid sequence SEQ ID No. 807.

2. Isolated or purified nucleotide sequence, **characterized in that** it comprises a nucleotide sequence chosen from:
a) a nucleotide sequence according to Claim 1; and
b) a nucleotide sequence complementary to a sequence as defined in a).

3. Nucleotide sequence according to Claim 1, **characterized in that** it is a DNA or RNA sequence.

4. Polypeptide encoded by a nucleotide sequence according to either of Claims 1 and 3.

5. Polypeptide of *Pyrococcus abyssi* of sequence SEQ ID No. 807.

6. Polypeptide **characterized in that** it comprises a polypeptide chosen from:
a) a polypeptide according to either of Claims 4 and 5; and
b) a fragment of at least 100 amino acids of a polypeptide of SEQ ID No. 807 and that increases the activity of formation of phosphodiester bonds of the large subunit of thermostable DNA polymerase type II.

7. Isolated or purified nucleotide sequence encoding a protein of amino acid sequence SEQ ID No. 809.

8. Isolated or purified nucleotide sequence, **characterized in that** it comprises a nucleotide sequence chosen from:
a) a nucleotide sequence according to Claim 7; and
b) a nucleotide sequence complementary to a sequence as defined in a).

9. Nucleotide sequence according to Claim 7, **characterized in that** it is a DNA or RNA sequence.

10. Polypeptide encoded by a nucleotide sequence according to either of Claims 7 and 9.

11. Polypeptide of *Pyrococcus abyssi,* of sequence SEQ ID No. 809.

12. Polypeptide, **characterized in that** it comprises a polypeptide chosen from:
a) a polypeptide according to either of Claims 10 and 11; and
b) a biologically active fragment with DNA polymerase activity of at least 100 amino acids of a polypeptide of sequence SEQ ID No. 809 and comprising amino acids 954 et seq. of sequence SEQ ID No. 809.

13. Nucleotide sequence encoding a polypeptide according to Claim 6 or 12.

14. Nucleotide sequence which can be used as a primer or probe, **characterized in that** said sequence is chosen from the nucleotide sequences according to one of Claims 1 to 3, 7 to 9 and 13.

15. Nucleotide sequence according to Claim 14, **characterized in that** it is immobilized on a support, covalently or noncovalently.

16. Cloning and/or expression vector, **characterized in that** it contains a nucleotide sequence according to one of Claims 1 to 3, 7 to 9 and 13.

17. Host cell, **characterized in that** it is transformed with a vector according to Claim 16.

18. Animal, except a human, comprising a transformed cell according to Claim 17.

19. Method for preparing a polypeptide according to one of Claims 4 to 6 and 10 to 12, **characterized in that** it uses a vector according to Claim 16, a cell according to Claim 17 or an animal according to Claim 18.

20. Recombinant polypeptide according to one of Claims 4 to 6 and 10 to 12 which can be obtained using a method according to Claim 19.

21. Method for preparing a synthetic polypeptide, **characterized in that** it uses an amino acid sequence of a polypeptide according to one of Claims 4 to 6 and 10 to 12.

22. Hybrid polypeptide, **characterized in that** it comprises at least the sequence of a polypeptide according to one of Claims 4 to 6 and 10 to 12.

23. Nucleotide sequence encoding a hybrid polypeptide according to Claim 22.

24. Vector, **characterized in that** it contains a nucleotide sequence according to Claim 23.

25. Hybrid polypeptide according to Claim 22, **characterized in that** it is a recombinant polypeptide which can be obtained using a vector according to Claim 24.

26. Method of biosynthesis of a compound of interest, **characterized in that** it uses a polypeptide according to one of Claims 4 to 6 and 10 to 12.

27. Use of a polypeptide according to one of Claims 4 to 6 and 10 to 12 for the biosynthesis of a compound of interest.

28. Method for polymerizing and/or amplifying a nucleic acid, **characterized in that** it uses a polypeptide with DNA polymerase activity, according to one of Claims 4 to 6, 10 to 12, 20, 22 and 25

29. Method according to Claim 28, **characterized in that** it comprises the step of bringing a biological sample into contact with said polypeptide, or a fragment thereof, with DNA polymerase activity, under conditions which allow a polymerization and/or amplification reaction between said polypeptide and the nucleic acids present in the biological sample.

30. Reagent or pack comprising a polypeptide with DNA polymerase activity, according to one of Claims 4 to 6, 10 to 12, 20, 22 and 25.

31. Use of a reagent or pack according to Claim 30, for detecting and/or identifying a target nucleic acid in a biological sample, in particular for detecting and/or identifying a nucleic acid specific for a pathogenic microorganism.

32. Use of a reagent or pack according to Claim 30, for detecting and/or identifying a target nucleic acid, in particular a gene of interest, or a mutation of said gene of interest, in a biological sample.

## Patentansprüche

1. Isolierte oder gereinigte Nukleotidsequenz, welche ein Protein mit der Aminosäuresequenz SEQ ID Nr. 807 kodiert.

2. Isolierte oder gereinigte Nukleotidsequenz, **dadurch gekennzeichnet, dass** sie eine Nukleotidsequenz umfasst, welche ausgewählt wird aus:
a) einer Nukleotidsequenz nach Anspruch 1; und
b) einer Nukleotidsequenz, welche zu einer Sequenz, wie unter a) definiert, komplementär ist.

3. Nukleotidsequenz nach Anspruch 1, **dadurch gekennzeichnet, dass** es sich um eine DNA- oder RNA-Sequenz handelt.

4. Polypeptid, welches durch eine Nukleotidsequenz nach einem der Ansprüche 1 und 3 kodiert wird.

5. Polypeptid von Pyrococcus *abyssi* mit der Sequenz SEQ ID Nr. 807.

6. Polypeptid, **dadurch gekennzeichnet, dass** es ein Polypeptid umfasst, welches ausgewählt wird unter:
a) einem Polypeptid nach einem der Ansprüche 4 und 5; und
b) einem Fragment von wenigstens 100 Aminosäuren eines Polypeptids mit der Sequenz SEQ ID Nr. 807 und welches die Bildungsaktivität von Phosphodiesterbindungen der großen Untereinheit der thermostabilen DNA-Polymerase vom Typ II erhöht.

7. isolierte oder gereinigte Nukleotidsequenz, welche ein Protein mit der Aminosäuresequenz SEQ ID Nr. 809 kodiert.

8. Isolierte oder gereinigte Nukleotidsequenz, **dadurch gekennzeichnet, dass** sie eine Nukleotidsequenz umfasst, welche ausgewählt wird aus:
a) einer Nukleotidsequenz nach Anspruch 7; und
b) einer Nukleotidsequenz, welche zu einer Sequenz, wie unter a) definiert, komplementär ist.

9. Nukleotidsequenz nach Anspruch 7, **dadurch gekennzeichnet, dass** es sich um eine DNA-oder RNA-Sequenz handelt.

10. Polypeptid, welches durch eine Nukleotidsequenz nach einem der Ansprüche 7 und 9 kodiert wird.

11. Polypeptid von Pyrococcus *abyssi* mit der Sequenz SEQ ID Nr. 809.

12. Polypeptid, **dadurch gekennzeichnet, dass** es ein Polypeptid umfasst, welches ausgewählt wird unter:
a) einem Polypeptid nach einem der Ansprüche 10 und 11; und
b) einem biologisch aktiven Fragment mit DNA-Polymerase-Aktivität von wenigstens 100 Aminosäuren eines Polypeptids mit der Sequenz SEQ ID Nr. 809 und welches die Aminosäuren 954 ff. der Sequenz SEQ ID Nr. 809 umfasst.

13. Nukleotidsequenz, welche ein Polypeptid nach Anspruch 6 oder 12 kodiert.

14. Nukleotidsequenz, welche als Primer oder Sonde einsetzbar ist, **dadurch gekennzeichnet, dass** die Sequenz ausgewählt wird unter den Nukleotidsequenzen nach einem der Ansprüche 1 bis 3, 7 bis 9 und 13.

15. Nukleotidsequenz nach Anspruch 14, **dadurch gekennzeichnet, dass** sie auf einem Träger kovalent oder nicht-kovalent immobilisiert ist.

16. Klonierungs- und/oder Expressionsvektor, **dadurch gekennzeichnet, dass** er eine Nukleotidsequenz nach einem der Ansprüche 1 bis 3, 7 bis 9 und 13 enthält.

17. Wirtszelle, **dadurch gekennzeichnet, dass** sie durch einen Vektor nach Anspruch 16 transformiert ist.

18. Tier mit Ausnahme des Menschen, welches eine transformierte Zelle nach Anspruch 17 umfasst.

19. Verfahren zur Herstellung eines Polypeptids nach einem der Ansprüche 4 bis 6 und 10 bis 12, **dadurch gekennzeichnet, dass** es einen Vektor nach Anspruch 16, eine Zelle nach Anspruch 17 oder ein Tier nach Anspruch 18 einsetzt.

20. Rekombinantes Polypeptid nach einem der Ansprüche 4 bis 6 und 10 bis 12, welches durch ein Verfahren nach Anspruch 19 erhalten werden kann.

21. Verfahren zur Herstellung eines synthetischen Polypeptids, **dadurch gekennzeichnet, dass** es eine Aminosäuresequenz eines Polypeptids nach einem der Ansprüche 4 bis 6 und 10 bis 12 verwendet.

22. Hybrides Polypeptid, **dadurch gekennzeichnet, dass** es wenigstens die Sequenz eines Polypeptids nach einem der Ansprüche 4 bis 6 und 10 bis 12 umfasst.

23. Nukleotidsequenz, welche ein hybrides Polypeptid nach Anspruch 22 kodiert.

24. Vektor, **dadurch gekennzeichnet, dass** er eine Nukleotidsequenz nach Anspruch 23 enthält.

25. Hybrides Polypeptid nach Anspruch 22, **dadurch gekennzeichnet, dass** es sich um ein rekombinantes Polypeptid handelt, welches durch das Einsetzen eines Vektors nach Anspruch 24 erhalten werden kann.

26. Verfahren zur Biosynthese einer Verbindung von Interesse, **dadurch gekennzeichnet, dass** es ein Polypeptid nach einem der Ansprüche 4 bis 6 und 10 bis 12 einsetzt.

27. Verwendung eines Polypeptids nach einem der Ansprüche 4 bis 6 und 10 bis 12 für die Biosynthese einer Verbindung von Interesse.

28. Verfahren für die Polymerisation und/oder die Amplifizierung einer Nukleinsäure, **dadurch gekennzeichnet, dass** es ein Polypeptid mit DNA-Polymerase-Aktivität nach einem der Ansprüche 4 bis 6, 10 bis 12, 20, 22 und 25 einsetzt.

29. Verfahren nach Anspruch 28, **dadurch gekennzeichnet, dass** es den Schritt eines Inkontaktbringens einer biologischen Probe mit dem Polypeptid oder einem von dessen Fragmenten mit DNA-Polymerase-Aktivität unter Bedingungen, welche eine Polymerisations- und/oder Amplifizierungsreaktion zwischen dem Polypeptid und den in der biologischen Probe vorhandenen Nukleinsäuren erlauben, umfasst.

30. Reagens oder Kit, welches bzw. welcher ein Polypeptid mit DNA-Polymerase-Aktivität nach einem der Ansprüche 4 bis 6, 10 bis 12, 20, 22 und 25 umfasst.

31. Verwendung eines Reagens oder Kits nach Anspruch 30 für die Detektion und/oder die Identifizierung einer Ziel-Nukleinsäure in einer biologischen Probe, insbesondere für die Detektion und/oder die Identifizierung von für einen pathogenen Mikroorganismus spezifischer Nukleinsäure.

32. Verwendung eines Reagens oder Kits nach Anspruch 30 für die Detektion und/oder die Identifizierung einer Ziel-Nukleinsäure, insbesondere eines Gens von Interesse oder einer mutierten Version des Gens von Interesse in einer biologischen Probe.
